(19) Europäisches Patentamt
European Patent Office
Office européen des brevets

(11) Publication number: **0 346 791 B1**

(12) **EUROPEAN PATENT SPECIFICATION**

(45) Date of publication of patent specification: **06.04.94**

(51) Int. Cl.[5]: **C07D 295/00**, C07D 211/70, C07D 211/14, C07D 333/20, C07D 307/36, A61K 31/395

(21) Application number: **89110572.8**

(22) Date of filing: **12.06.89**

# (54) 1,2-diarylethylamines for treatment of neurotoxic injury.

(30) Priority: **14.06.88 US 206642**

(43) Date of publication of application:
**20.12.89 Bulletin 89/51**

(45) Publication of the grant of the patent:
**06.04.94 Bulletin 94/14**

(84) Designated Contracting States:
**AT BE CH DE ES FR GB GR IT LI LU NL SE**

(56) References cited:
**DE-A- 2 610 433**
**GB-A- 1 143 263**
**JP-A- 5 239 686**
**US-A- 4 617 409**

**THE JOURNAL OF THE AMERICAN CHEMICAL SOCIETY, vol. 75, no. 13, 1953 R.V.HEINZELMAN et al. "Compounds containing the pyrrolidine ring. Analogs of sympathomimetic amines" pages 3409-3413**

(73) Proprietor: **G.D. Searle & Co.**
**P.O. Box 5110**
**Chicago Illinois 60680(US)**

(72) Inventor: **Gray, Nancy M.**
**16523 West Glen Farms Drive**
**Ellisville Missouri 63011(US)**
Inventor: **Cheng, Brian K.**
**426 Savannah Ridge Drive**
**St. Charles Missouri 63033(US)**

(74) Representative: **Wolff, Hans Joachim, Dr.jur. Dipl.-Chem. et al**
**BEIL, WOLFF & BEIL,**
**Rechtsanwälte,**
**Postfach 80 01 40**
**D-65901 Frankfurt am Main (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

THE JOURNAL OF THE AMERICAN CHEMICAL SOCIETY, vol. 72, January - April 1950 L.H.GOODSON et al. "Diphenyl-ethylamines.I.The preparation of tertiary amines by the Grignard reaction" pages 358-362

CHEMICAL ABSTRACTS, vol. 51, no. 2, January 25, 1957, Columbus, Ohio, USA LOUIS H.GOODSON et al. "1-(Bicyclic carbocyclic aryl)-2(monocyclic carbo- cyclic aryl) ethylamines and salts thereof which are agents for producing mild analgesia" Abstract-no. 1300g

CHEMICAL ABSTRACTS, vol. 100, no. 9, February 27, 1984, Columbus, Ohio, USA GABRIELYAN, S.A. et al. "Synthesis and antiinflamma- tory activity of substituted alpha-aminoacetophenone hydrochlorides" page 568, Abstract-no. 67 937n

CHEMICAL ABSTRACTS, vol. 106, no. 25, June 22, 1987, Columbus, Ohio, USA CI,XIAHONG LEE "Photoinduced eletrontransfer fragmentation of amino alcohols:stereochemical effects and connectivity between one-and two-electron events" page 391, Abstract-no. 213 192h

CHEMICAL ABSTRACTS, vol. 69, no. 17, October 21, 1968, Columbus, Ohio, USA MUNK, MORTON E. et al. "Con- formational equilibria in the 2-amino-1,2-diphenylethanol system. I. Nuclear magnetic resonance studies" page 6224, Abstract-no. 66 765j

CHEMICAL ABSTRACTS, vol. 84, no. 1, January 5, 1976, Columbus, Ohio, USA G.FLAD et al. "New epoxides" page 402, Abstract-no. 4 746e

CHEMICAL ABSTRACTS, vol. 87, no. 7, August 15, 1977, Columbus, Ohio, USA UNO, HITOSHI et al. "1-Sub- stituted-4-(1,2-diphenyl- ethyl)piperazines" page 485, Abstract-no. 53 381c

CHEMICAL ABSTRACTS, vol. 97, no. 22, November 29, 1982, Columbus, Ohio, USA NAKAMURA, H. et al. "Chemical structure and pharmacological activities of diphenylethyl- piperazine derivatives" page 15, Abstract-no. 192 732n

CHEMICAL ABSTRACTS, vol. 99, no. 3, July 18, 1983, Columbus, Ohio, USA C.F.BERNASCONI "Nucleophilic addition to olefins.8. Addition of piperidine, morpholine, and n-butylamine to alpha-cyano-4-nitrostilbene and alpha-cyano-2,4- -dinitrostilbene. Kinetics and equilibria" page 550, Abstract-no. 21 676j

CHEMICAL ABSTRACTS, vol. 108, no. 3, January 18, 1988, Columbus, Ohio, USA K.NATSUKA "Synthesis and structure-activity relation- ships of 1-substituted 4- -(1,2-diphenylethyl)pipera- zine derivatives having narcotic agonist and anta- gonist activity" page 550, Abstract-no. 21 847v

CHEMICAL ABSTRACTS, vol. 109, no. 5, August 1, 1988, Columbus, Ohio, USA S.HAMANN et al. "Synthesis and stereochemistry of the formation of tertiary -fluoro amine by fluorina- tion of amino alcohols with N,N-diethyl-1,1,2-trifluoro 2-chloroethylamine (FAR) and mixture of hydrogen fluoride and pyridine" page 582, Abstract-no. 37 477f

CHEMICAL ABSTRACTS, vol. 68, no. 25, June 17, 1968, Columbus, Ohio, USA RYUICHI KIMURA et al. "Novel arylalkylamines" page 11026, Abstract-no. 114 428e

## Description

### FIELD OF THE INVENTION

This invention is in the field of clinical neurology and relates specifically to compounds, compositions and methods for neuroprotective purposes such as controlling brain damage which occurs during periods of anoxia or ischemia associated with stroke, cardiac arrest or perinatal asphyxia.

### BACKGROUND OF THE INVENTION

Unlike other tissue which can survive extended periods of hypoxia, brain tissue is particularly sensitive to deprivation of oxygen or energy. Permanent damage to neurons can occur during brief periods of hypoxia, anoxia or ischemia. Neurotoxic injury is known to be caused or accelerated by certain excitatory amino acids (EAA) found naturally in the central nervous system (CNS). Glutamate (Glu) is an endogenous amino acid which was early characterized as a fast excitatory transmitter in the mammalian brain. Glutamate is also known as a powerful neurotoxin capable of killing CNS neurons under certain pathological conditions which accompany stroke and cardiac arrest. Normal glutamate concentrations are maintained within brain tissue by energy-consuming transport systems. Under low energy conditions which occur during periods of hypoglycemia, hypoxia or ischemia, cells can release glutamate. Under such low energy conditions the cell is not able to take glutamate back into the cell. Initial glutamate release stimulates further release of glutamate which results in an extracellular glutamate accumulation and a cascade of neurotoxic injury.

It has been shown that the sensitivity of central neurons to hypoxia and ischemia can be reduced by either interfering with synaptic transmission through blockade of the sodium or calcium ion channel or by the specific antagonism of postsynaptic glutamate receptors [see S. M. Rothman and J. W. Olney, "Glutamate and the Pathophysiology of Hypoxia - Ischemic Brain Damage," Annals of Neurology, Vol. 19, No. 2 (1986)]. Glutamate is characterized as a broad spectrum agonist having activity at three neuronal excitatory amino acid receptor sites. These receptor sites are named after the amino acids which selectively excite them, namely: kainate (KA), N-methyl-D-aspartate (NMDA or NMA) and quisqualate (QUIS). Glutamate is believed to be a mixed agonist capable of binding to and exciting all three receptor types.

Neurons which have EAA receptors on their dendritic or somal surfaces undergo acute excitotoxic degeneration when these receptors are excessively activated by glutamate. Thus, agents which selectively block or antagonize the action of glutamate at the EAA synaptic receptors of central neurons can prevent neurotoxic injury associated with anoxia, hypoxia or ischemia caused by stroke, cardiac arrest or perinatal asphyxia.

Phencyclidine (PCP) and the PCP-like compound ketamine have been found to reduce selectively the excitatory effects of NMDA as compared to KA and QUIS [Anis, N.A. et al, "The Dissociative Anaesthetics, Ketamine and Phencyclidine, Selectively Reduce Excitation of Central Mammalian Neurones by N-Methyl-Aspartate", Br. J. Pharmacol., 79, 565 (1983)]. Other compounds having PCP-like properties such as cyclazocine, kynurenate and various barbiturates such as secobarbital, amobarbital and pentobarbital, have been tested as antagonists in blocking NMDA- or KA-induced neurotoxicity [J. W. Olney et al., "The Anti-Excitotoxic Effects of Certain Anesthetics, Analgesics and Sedative-Hypnotics," Neuroscience Letters, 68, 29-34 (1986)].

A correlation has been found between the PCP binding effects of some PCP-derivative stereoisomers and NMDA antagonism. For example, the stereoselective effects of cis-N-(1-phenyl-4-methylcyclohexyl)-piperidine and (+)-1-(1-phenylcyclohexyl)-3-methylpiperidine [(+)-PCMP] over each of their corresponding isomer counterparts in reducing the excitatory action of NMDA have been confirmed in binding and behavioral data [S.D. Berry et al, "Stereoselective Effects of Two Phencyclidine Derivatives on N-Methylaspartate Excitation of Spinal Neurones in the Cat and Rat", Eur. J. Pharm., 96, 261-267 (1983)]. Also, the compound (+)-PCMP has been found to be a potent inhibitor of the specific binding of [$^3$H]PCP to rat cerebral cortical membranes [M. E. Goldman et al, "Differentiation of [$^3$H]Phencyclidine and (+)-[$^3$H]-SKF-10,047 Binding Sites in Rat Cerebral Cortex", FEBS Lett., 170, 333-336 (1985)].

Other neurochemical mechanisms by which PCP alters behavior are known. For example, binding assays of the PCP/sigma site have been used to evaluate arylcycloalkylamines [R. Quirion, "Phencyclidine (Angel Dust)/Sigma 'Opiate' Receptor: Visualization by Tritium-Sensitive Film", Proc. Natl. Acad. Sci. U.S.A., 78, 5881 (1981)]. PCP-like drugs may induce ipsilateral turning in rats by action on the PCP/sigma receptor as indicated by studies with arylcycloalkylamines, sigma-agonist benzomorphans and 1,3-dioxolanes.

These PCP-like classes of compounds have been found to inhibit NMDA-induced acetylcholine (ACh) release and such ACh release has been correlated with their affinity for the PCP receptor and with

behavioral activity [L. D. Snell et al, "Antagonism of N-Methyl-D-Aspartate-Induced Transmitter Release in the Rat Striatum by Phencyclidine-Like Drugs and its Relationship to Turning Behavior", J. Pharmacol.Exp. Ther., 235, No. 1, 50-56 (1985)].

Certain 1,2-diarylethylamines have been described for use as human pharmaceutical therapeutic purposes. For example, U.K. Patent No. 1,143,263 mentions (±)-1,2-diphenyl-2-(1-piperidinyl)ethanone for use as an anti-depressant. Japanese Patent No. 8526/61 mentions (±)-1-(1,2-diphenylethyl)piperidine as having actions on the central nervous system and mentions several possible uses for the compound, namely as an anti-tussive, as an anesthetic or as a monoamine oxidase inhibitor. Japanese Patent No. 15,937 mentions (±)-1-[2-phenyl-1-(2-thienyl)ethyl]piperidine hydrosulfate for use as an anti-spasmodic, analgesic or an anti-tussive. The compound (±)-1-(1,2-diphenylethyl)pyrrolidine has been mentioned for sympathomimetic or bronchodilator uses [R.V. Heinzelmann et al, J. Am. Chem. Soc., 75 3409-3413 (1953)-].

Other 1,2-diarylethylamines have also been described without mention of pharmaceutical utility. For example, the compound (±)-1-(1,2-diphenyl-1-hydroxyethyl)piperidine is mentioned as an intermediate to making certain derivatives of the alkaloid bicuculline which is a convulsant [T.V. Hung et al, Aust. J. Chem., 34, 383-395 (1981)]. The laboratory synthesis of the compounds (±)-1-[1-(4-methylphenyl)-2-phenylethyl]-piperidine and (±)-1-[1-(4-methoxyphenyl)-2-phenylethyl)piperidine has been described without mention of any pharmaceutically-related use [L.A. Goodson et al, J. Am. Chem. Soc., 72, 358-362 (1950)].

The following references disclose substituted 1,2-diarylethylamines and related compounds wich exhibit antidepressant (see (1), (7), analgetic ((3),(12),(13,(16), (17),(5),(15)), bronchodilatory ((13)), or antiinflammatory activity (8):

(1) GB-A 1 143 263, (2) US-A 4 617 409, (3) DE-A 2 610 433, (4) JP-A 52-39 686, (5) Heinzelmann, R., V. et al., J.Am. Chem. (1953), 13, 3409-3413, (6) Goodson, L., H. et al., J. Am.Chem.Soc. (1950), 72, 358-362, (7) Chem. Abs. 51, No.2 (1957), Abs. No: 1300g, (8) Chem.Abs., 100, No. 9, (1984), 568, Abs.No.: 67937n, (9) Chem.Abs. 106, No.25 (1987), 391, Abs.No.: 213192h, (10) Chem.Abs. 69, No. 17 (1968), 6224, Abs.No.: 66765j, (11) Chem.Abs. 84, No.1 (1976), 402, Abs.No.: 4746e, (12) Chem.Abs. 87, No. 7 (1977), 485, Abs.No.: 53381c, (13) Chem.Abs. 97, No. 22, (1982), 15, Abs.No.: 192732n, (14) Chem.Abs. 99, No. 3, (1983), 550, Abs.No.: 21676j, (15) Chem.Abs. 108, No. 3 (1988), 550, Abs.No.: 21847v, (16) Chem.Abs. 109, No. 5 (1988), Abs.No. 37477f, (17) Chem.Abs. 68, No. 25 (1968), Abs.No. 114428.

None of these references however discloses the utility of such compounds to treat neurotoxic injury.

## DESCRIPTION OF THE INVENTION

Control of neuropathological processes and the neurodegenerative consequences thereof in mammals is provided by treating a mammal susceptible to neurotoxic injury with an anti-excitotoxic amount of a compound of a class of 1,2-diarylethylamines represented by formula I:

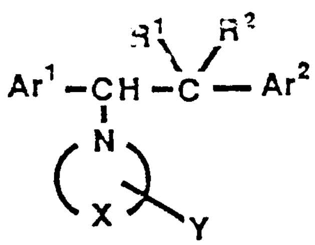

(I)

wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated $C_3$-$C_{10}$ cycloalkyl carbocyclic group having three to eight ring carbons; wherein each of $Ar^1$ and $Ar^2$ is a group independently selected from phenyl, thienyl, furanyl, pyridinyl, thiazolyl and naphthyl groups, and wherein any of the foregoing $Ar^1$ and $Ar^2$ groups may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, $C_2$-$C_{20}$ alkenyl, $C_2$-$C_{20}$ alkynyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein X is selected from CH or $CH_2$ to form a ring having five to seven members provided that such ring is saturated or contains one double bond; and wherein Y is a group selected from hydrido, $C_1$-$C_{20}$ alkyl, halo, hydroxyl, $C_1$-$C_{10}$ alkoxy, amino, cyano, nitro and mercapto; with the proviso that when each of $Ar^1$ and $Ar^2$ is phenyl and each of $R^1$ and $R^2$ is hydrido or $R^1$ is H and $R^2$ is hydroxy, then X cannot be a linear alkylene chain having four or five carbon atoms so as to form a

racemic mixture;
with the further proviso that when $A^1$ is para-methylphenyl or para-methoxyphenyl and each of $R^1$ and $R^2$ is hydrido and $Ar^2$ is phenyl, then X cannot be a linear alkylene chain having five carbon atoms,
except the compounds wherein
$Ar^1$ is phenyl, $R^2$ is H and X is $CH_2$ to form a 6-numbered ring and at the same time
a) $Ar^2$ is phenyl, $R^1$ is F or OH
b) $Ar^2$ is furanyl and $R^1$ is OH
and wherein
$Ar^1$, $Ar^2$ is 2, 3 or 4 $NO_2$-phenyl, $R^2$ is H, $R^1$ OH and X is $CH_2$ to form a 6-numbered ring;
or wherein $R^1$, $R^2$ is H, X is $CH_2$ to form a 6-numbered ring and $Ar_1$ is naphthyl, $Ar_2$ is phenyl or $Ar_1$ is thienyl and $Ar_2$ is phenyl
and wherein
$Ar^1$ is methoxyphenyl, X is $CH_2$ to form a 5-numbered ring and at the same time
$Ar^2$ is methoxyphenyl, $R^1$ = H, $R^2$ = OH or $R^1$,$R^2$ = H.

A preferred class of compounds within Formula

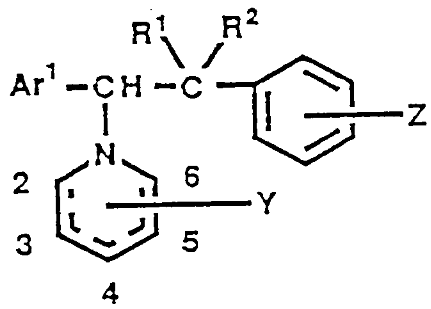

consists of those compounds wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated carbocyclic group having three or seven ring carbons; wherein $Ar^1$ is a group selected from phenyl, thienyl, furanyl, pyridinyl, thiazolyl and naphthyl groups, and wherein any of the foregoing $Ar^1$ groups having a substitutable position may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ alkyl, halo, hydroxyl, alkoxy and amino; and wherein Z is one or more groups selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto.

A more preferred class of compounds within Formula I consists of those compounds wherein each of $R^1$ and $R^2$ is a group independently selected from hydrido, $C_1$-$C_{20}$ linear or branched alkyl, $C_3$-$C_{10}$ cycloalkyl, hydroxyl, halo, $C_1$-$C_{10}$ alkoxy, or wherein $R^1$ and $R^2$ may be taken together to form a saturated or partially unsaturated carbocyclic group having three or seven ring carbons; wherein $Ar^1$ is selected from phenyl, thienyl, pyridinyl and naphthyl groups, and wherein any of the foregoing $Ar^1$ groups having a substitutable position may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalky, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; and wherein Z is one or more groups selected from hydrido, $C_1$-$C_{20}$ linear or branched alkyl, $C_3$-$C_{10}$ cycloalkyl, halo, hydroxyl, $C_1$-$C_{10}$ alkoxy and amino.

It is believed that the compounds defined by Formula I are novel where the Formula I definition is qualified by the following proviso descriptions and the disclaimers as indicated above.

With regard to the foregoing proviso descriptions, it is intended that where specific substituents are recited, such proviso relates to those specific substituents, only, and not to substituents modified by removal or addition of a radical. For example, where $Ar^1$ or $Ar^2$ is phenyl or thiophene, then such proviso does not exclude substituted phenyl or substituted thiophene unless otherwise specified. Also, it is intended that where a racemate is excluded from Formula I by a proviso description, individual isomers of such racemate remain within the scope of Formula I.

A more highly preferred class of compounds consists of those compounds wherein each of $R^1$ and $R^2$ is a group independently selected from hydrido, alkyl, cycloalkyl, hydroxyl, halo and alkoxy, or wherein $R^1$ and $R^2$ may be taken together to form a saturated or partially unsaturated carbocyclic group having three to seven ring carbons; wherein $Ar^1$ is selected from phenyl, thienyl, pyridinyl and naphthyl groups, and wherein any of the foregoing $Ar^1$ groups having a substitutable position may be substituted with one or more radicals selected from hydrido, alkyl, cycloalkyl, halo, haloalkyl, hydroxyl, hydroxyalkyl, alkoxy, alkoxyalkyl, amino, cyano, nitro and mercapto; wherein Y is a group selected from hydrido, alkyl, halo, hydroxyl, alkoxy and amino; and wherein Z is one or more groups selected from hydrido, alkyl, cycloalkyl, halo, hydroxyl, alkoxy and amino.

A most preferred class of compounds of those compounds wherein each of $R^1$ and $R^2$ is a group independently selected from hydrido, alkyl and hydroxyl, or wherein $R^1$ and $R^2$ may be taken together to form a saturated or partially unsaturated carbocyclic group having three to six ring carbons; wherein $Ar^1$ is a group selected from phenyl, thienyl, pyridinyl and naphthyl groups, and wherein any of the foregoing $Ar^1$ groups having one or more substitutable position may be substituted with one or more radicals selected from hydrido, alkyl, halo, haloalkyl, hydroxyl, alkoxy, amino and nitro; wherein Y is a group selected from hydrido, alkyl, halo, hydroxyl, alkoxy and amino; and wherein Z is one or more groups selected from hydrido, alkyl, halo, hydroxyl and alkoxy.

A further preferred class of more particularly preferred compounds within Formula I contains a first sub-class of compounds defined by of Formula III:

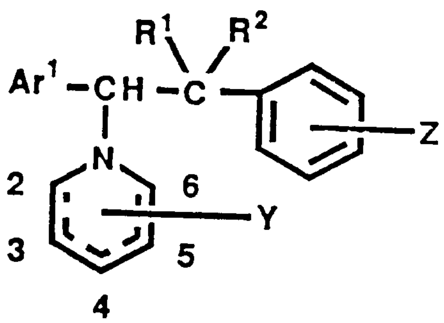

(III)

wherein each of $R^1$ and $R^2$ is a group independently selected from hydrido, alkyl, cycloalkyl, hydroxyl, halo and alkoxy, or wherein $R^1$ and $R^2$ may be taken together to form a saturated or partially unsaturated carbocyclic group having three to seven ring carbons; wherein $Ar^1$ is a group selected from phenyl, thienyl, furanyl, pyridinyl, thiazolyl and naphthyl groups, and wherein any of the foregoing $Ar^1$ groups having a substitutable position is substituted with one or more radicals selected from hydrido, alkyl, cycloalkyl, halo, haloalkyl, hydroxyl, hydroxyalkyl, alkoxy, alkoxyalkyl, amino, cyano, nitro and mercapto; wherein Y is a group selected from hydrido, alkyl, halo, hydroxyl, alkoxy and amino; wherein Z is one or more groups selected from alkyl, cycloalkyl, halo, haloalkyl, hydroxyl, hydroxyalkyl, alkoxy, alkoxyalkyl, amino, cyano, nitro and mercapto; and wherein the broken line within the N-containing ring represents a double bond between any two adjacent carbon atoms involving the 2-, 3-, 4-, 5- and 6-positions of the N-containing ring.

A more highly preferred class of compounds within the second sub-class of compounds of Formula III consists of those compounds wherein each of $R^1$ and $R^2$ is a group independently selected from hydrido, alkyl, cycloalkyl, hydroxyl, halo and alkoxy, or wherein $R^1$ and $R^2$ may be taken together to form a saturated or partially unsaturated carbocyclic group having three to seven ring carbons; wherein $Ar^1$ is a group selected from phenyl, thienyl, furanyl, pyridinyl, thiazolyl and naphthyl groups, and wherein any of the foregoing groups having one or more substitutable positions may be substituted with one or more radicals selected from hydrido, alkyl, cycloalkyl, halo, haloalkyl, hydroxyl, hydroxyalkyl, alkoxy, alkoxyalkyl, amino, cyano, nitro and mercapto; wherein Y is a group selected from hydrido, alkyl, halo, hydroxyl, alkoxy and amino; wherein Z is one or more groups selected from hydrido, alkyl, cycloalkyl, halo, hydroxyl, alkoxy and amino; and wherein the broken line within the N-containing ring represents a double bond between any two adjacent carbon atoms involving the 3-, 4-, 5-positions of the N-containing ring.

An even more highly preferred class of compounds within the second sub-class of compounds of Formula III consists of those compounds wherein each of $R^1$ and $R^2$ is a group independently selected from hydrido, alkyl, cycloalkyl, hydroxyl, halo and alkoxy; wherein $R^1$ and $R^2$ may be taken together to form a saturated or partially unsaturated cycloalkyl having three to seven ring carbons; wherein $Ar^1$ is selected from phenyl, thienyl, pyridinyl and naphthyl groups, and wherein any of the foregoing groups having a

substitutable position is substituted with one or more radicals selected from hydrido, alkyl, cycloalkyl, halo, haloalkyl, hydroxyl, hydroxylalkyl, alkoxy, alkoxyalkyl, amino, cyano, nitro and mercapto; wherein Y is a group selected from hydrido, alkyl, halo, hydroxyl, alkoxy and amino; wherein Z is one or more groups selected from hydrido, alkyl, cycloalkyl, halo, hydroxyl, alkoxy and amino; and wherein the broken line within the N-containing ring represents either a double bond between any two adjacent carbon atoms involving the 3-, 4-, 5-positions of the N-containing ring.

A most preferred class of compounds within the second sub-class of compounds of Formula III consists of those compounds wherein each of $R^1$ and $R^2$ is a group independently selected from hydrido, alkyl and hydroxyl; wherein $R^1$ and $R^2$ may be taken together to form a saturated or partially unsaturated cycloalkyl having three to six ring carbons; wherein $Ar^1$ is selected from phenyl, thienyl, pyridinyl and naphthyl groups, and wherein any of the foregoing groups having one or more substitutable positions is substituted with one or more radicals selected from hydrido, alkyl, halo, haloalkyl, hydroxyl, alkoxy, amino and nitro; wherein Y is a group selected from hydrido, alkyl, halo, hydroxyl, alkoxy and amino; wherein Z is one or more groups selected from hydrido, alkyl, halo, hydroxyl and alkoxy; and wherein the broken line within the N-containing ring represents either a double bond between any two adjacent carbon atoms involving the 3-, 4-, 5-positions of the N-containing ring.

Specific compounds of Formula II which are most highly preferred compounds are the following:
(-)-1-(1,2-diphenylethyl)piperidine;
(+)-1-(1,2-diphenylethyl)piperidine;
(±)-1-[1-phenyl-2-(2-pyridinyl)ethyl]piperidine;
1-[1-phenyl-2-(2-pyridinyl)ethenyl]piperidine;
(±)-1-(1,2-diphenylethyl)-1H-azepine;
(±)-1-(1,2-diphenylbutyl)piperidine;
(±)-1-(1,2-diphenyl-3-methylbutyl)piperidine;
(±)-1-(1,2-diphenylethyl)-4-methylpiperazine;
(±)-1-[1-(1-phenylcyclopentyl)benzyl]piperidine;
(±)-1-[1-(1-phenylcyclohexyl)benzyl]piperidine;
(±)-1-[1-(1-phenylcyclopropyl)benzyl]piperidine;
(±)-1-[2,2-dimethyl-2-phenyl-1-(2-thienyl)ethyl]piperidine;
(±)-1-[1-(3-methoxyphenyl)-2-phenylethyl]piperidine;
(±)-1-[1-(4-fluorophenyl)2-phenylethyl]piperidine;
(±)-1-(2,2-dimethyl-1,2-diphenylethyl)piperidine;
(±)-1-[1-(3-methylphenyl)2-phenylethyl]piperidine;
(±)-1-[1-(2-furyl)-2-phenylethyl]piperidine;
(±)-1-[1-(2-chlorophenyl)-2-phenylethyl]piperidine;
(±)-1-[1-(2-methoxyphenyl)-2-phenylethyl]piperidine;

Specific compounds of Formula III which are most highly preferred compounds are the following: (±)-1-(1,2-diphenylethyl)-1,2,3,6-tetrahydropyridine and (±)-1-[2-phenyl-1-(2-thienyl)ethyl]-1,2,3,6-tetrahydropyridine.

The term "hydrido" denotes a single hydrogen atom (H) which may be attached, for example, to a carbon atom or attached to an oxygen atom to form an hydroxyl group. Where the term "alkyl" is used, either alone or within other terms such as "haloalkyl" and "hydroxyalkyl", the term "alkyl" embraces linear or branched radicals having one to about twenty carbon atoms or, preferably, one to about ten carbon atoms. More preferred alkyl radicals are "lower alkyl" radicals having one to about five carbon atoms. The term "cycloalkyl" embraces cyclic radicals having three to about ten ring carbon atoms, preferably three to about six carbon atoms, such as cyclopropyl and cyclobutyl. The term "haloalkyl" embraces radicals wherein any one or more of the alkyl carbon atoms is substituted with one or more halo groups, preferably selected from bromo, chloro and fluoro. Specifically embraced by the term "haloalkyl" are monohaloalkyl, dihaloalkyl and polyhaloalkyl groups. A monohaloalkyl group, for example, may have either a bromo, a chloro, or a fluoro atom within the group. Dihaloalkyl and polyhaloalkyl groups may be substituted with two or more of the same halo groups, or may have a combination of different halo groups. A dihaloalkyl group, for example, may have two bromo atoms, such as a dibromomethyl group, or two chloro atoms, such as a dichloromethyl group, or one bromo atom and one chloro atom, such as a bromochloromethyl group. Examples of a polyhaloalkyl are trifluoromethyl, 2,2,2-trifluoroethyl, perfluoroethyl and 2,2,3,3-tetrafluoropropyl groups. The terms "alkylol" and "hydroxyalkyl" embrace linear or branched alkyl groups having one to about ten carbon atoms any one of which may be substituted with one or more hydroxyl groups. The term "alkenyl" embraces linear or branched radicals having two to about twenty carbon atoms, preferably three to about ten carbon atoms, and containing at least one carbon-carbon double bond. The

term "alkynyl" embraces linear or branched radicals having two to about twenty carbon atoms, preferably two to about ten carbon atoms, and containing at least one carbon-carbon triple bond. The terms "cycloalkenyl" and "cycloalkynyl" embrace cyclic radicals having three to about ten ring carbon atoms including, respectively, one or more double or triple bonds involving adjacent ring carbons. The terms "alkoxy" and "alkoxyalkyl" embrace linear or branched oxy-containing radicals each having alkyl portions of one to about ten carbon atoms, such as methoxy group. The "alkoxy" or "alkoxyalkyl" radicals may be further substituted with one or more halo atoms, such as fluoro, chloro or bromo, to provide haloalkoxy or haloalkoxyalkyl groups. The term "heteroaryl" embraces aromatic ring systems containing one or two hetero atoms selected from oxygen, nitrogen and sulfur in a ring system having five or six ring members, examples of which are thienyl, furanyl, pyridinyl, thiazolyl, pyrimidyl and isoxazolyl. The term "alkylene chain" describes a chain of two to six methylene ($-CH_2-$) groups which, as shown in Formula I, may form a cyclic structure including the nitrogen atom of Formula I.

Specific examples of alkyl groups are methyl, ethyl, n-propyl, isopropyl, n-butyl, sec-butyl, iso-butyl, tert-butyl, n-pentyl, iso-pentyl, methylbutyl, dimethylbutyl and neopentyl. Typical alkenyl and alkynyl groups may have one unsaturated bond, such as an allyl group, or may have a plurality or unsaturated bonds, with such plurality of bonds either adjacent, such as allene-type structures, or in conjugation, or separated by several saturated carbons.

Included within the family of compounds of Formulas I-III are the tautomeric forms of the described compounds, isomeric forms including diastereoisomers, and the pharmaceutically-acceptable salts thereof. The term "pharmaceutically-acceptable salts" embraces salts commonly used to form alkali metal salts and to form addition salts of free acids or free bases. Since the compounds of Formulas I-III contain basic nitrogen atoms, such salts are typically acid addition salts or quaternary salts. The nature of the salt is not critical, provided that it is pharmaceutically acceptable, and acids which may be employed to form such salts are, of course, well known to those skilled in this art. Examples of acids which may be employed to form pharmaceutically acceptable acid addition salts include such inorganic acids as hydrochloric acid, sulfuric acid and phosphoric acid, and such organic acids as maleic acid, succinic acid and citric acid. Other pharmaceutically acceptable salts include salts with alkali metals or alkaline earth metals, such as sodium, potassium, calcium and magnesium, or with organic bases, such as dicyclohexylamine. All of these salts may be prepared by conventional means by reacting, for example, the appropriate acid or base with the corresponding compound of Formulas I-III.

Compounds of Formulas I-III may be prepared in accordance with the following general procedures:

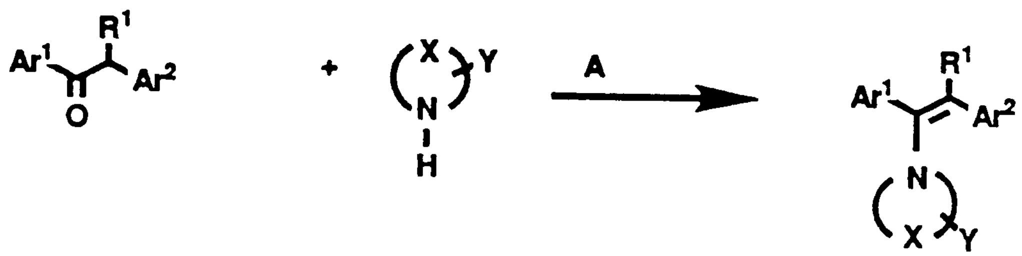


1 2 3

wherein $Ar^1$, $Ar^2$, $R^1$, X and Y are as defined before; wherein A can be a variety of Bronsted or Lewis acids such as p-toluenesulfonic acid, hydrochloric acid, sulfuric acid, titanium tetrachloride, stannous chloride, or zinc chloride.

One of the processes that can be used to synthesize the products of the invention starts with diarylketones of general formula 1 where $R^1$, $Ar^1$ and $Ar^2$ have the values defined previously, and with a cyclic secondary amine of general formula 2 where X and Y have the values defined previously. The ketone is treated with the amine in the presence of a variety of Bronsted or Lewis acids like p-toluenesulfonic acid, hydrochloric acid, sulfuric acid, titanium tetrachloride, stannous chloride, or zinc chloride to generate the imine of general formula 3. The reaction is best achieved by mixing the reagents in a solvent like toluene, ethyl acetate or lower alkane like hexane or heptane, and the reaction temperature can vary from about 0 ° C to reflux of the reaction mixture.

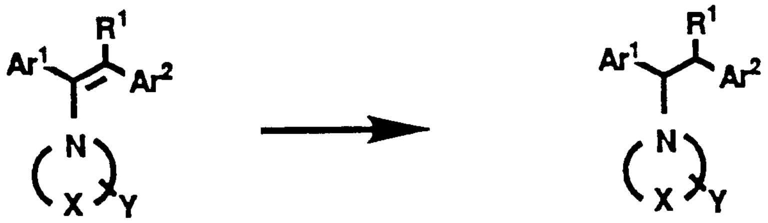

3 4

wherein $Ar^1$, $Ar^2$, $R^1$ X, and Y are as defined before.

In the second step of the process, the imine 3 is transformed into the amine 4 by reduction in a protic or aprotic solvent. Possible reducing agents to perform this transformation are lithium aluminum hydride, sodium borohydride, sodium cyanoborohydride, borane, or any other reducing systems familiar to those skilled in the art.

The reagent ketones of general formula 1 can be prepared in accordance with the following generic procedure:

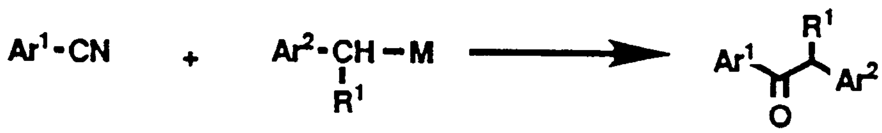

5 6 1

wherein $Ar^1$, $Ar^2$, $R^1$ are as defined before; M = Li, MgBr, MgCl, CuCl, or CuBr.

One of the processes that can be used to generate the ketones of general formula 1 starts with nitriles of general formula 5 where $Ar^1$ is as defined previously. This nitrile is treated with an organometallic reagent of general formula 6 where $Ar^2$ and $R^1$ are as defined previously and M represents a metal or metal complex such as Li, MgCl, MgBr, CuCl, or CuBr. This reaction is best achieved by mixing the reagents in a solvent like toluene, tetrahydrofuran, ether, or dioxane, and the reaction temperature can vary from 0 ° C to reflux of the reaction mixture. The reaction product is isolated under acidic conditions in the presence of water to obtain the ketone product.

Alternately, the ketone can be prepared according to the following generic procedure:

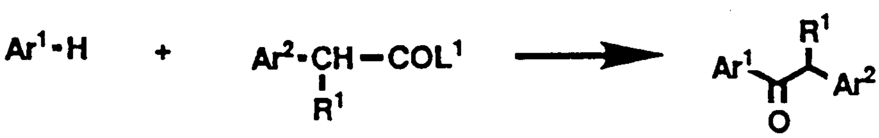

7 8 1

wherein $Ar^1$, $Ar^2$, and $R^1$ are as defined before. $L^1$ is selected from halogen, OH, or OR3; R3 = lower alkyl, aryl or benzyl.

The ketone of general formula 1 can alternately be prepared by mixing the arene of general formula 7 where $Ar^1$ is as previously described with the carboxylic acid derivative of general formula 8 where $Ar^2$ and $R^1$ are as defined before. $L^1$ is a good leaving group, which is , for example, halogen, OH, alkoxy, or aryloxy. The reaction is best achieved by mixing the reagents either neat or in a solvent like ether, tetrahydrofuran, dioxane or lower alkane such as hexane or heptane, in the presence of an appropriate catalyst B. If $L^1$ is OH, B is a dehydrating catalyst such as phosphorus oxychloride, phosphorus pentoxide,

polyphosphoric acid or sulfuric acid. If $L^1$ is alkoxy or aryloxy, a strongly acidic catalyst such as sulfuric acid, phosphoric acid or hydrochloric acid may be used. When $L^1$ is halogen, Lewis acid catalysts such as aluminum chloride, ferric chloride, and zinc chloride are preferred. The reaction temperature can vary from 0 °C to reflux of the reaction mixture.

Generic Procedure II

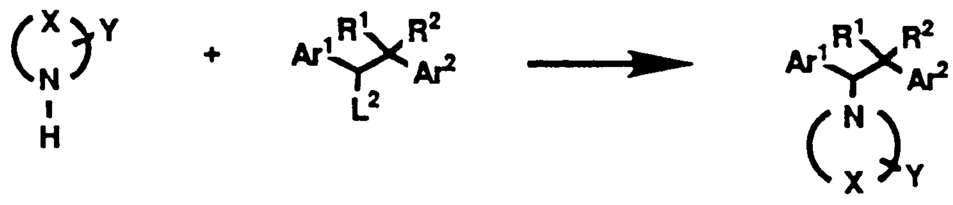

2 9 10

wherein $Ar^1$, $Ar^2$, $R^1$, $R^2$ X, and Y are as defined previously. $L^2$ is halogen, tosylate, mesylate, brosylate, or OH.

The compounds of the invention may be prepared by displacement of the leaving group $L^2$ in general formula 9 by the appropriate secondary amine of general formula 2. Good leaving groups are, for example, halogen, tosylate, mesylate, and brosylate. The conversion can be best achieved by mixing the reagents either neat or in a solvent like acetonitrile, dimethylformamide, dimethylsulfoxide, or tetrahydrofuran, and the reaction temperature can vary from 0 °C to 100 °C.

The reagents of general formula 9 can be prepared in accordance with the following generic procedure:

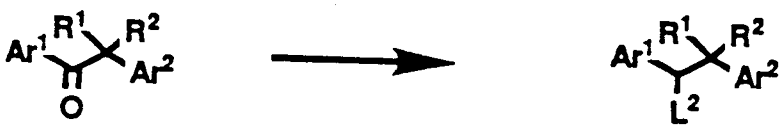

11 9

wherein $Ar^1$, $Ar^2$, $R^1$, $R^2$ and $L^2$ are as previously defined.

One of the processes that can be used to generate the compounds of general formula 9 is conversion of the oxo group in ketones of general formula 11 to a leaving group $L^2$, where $L^2$ is as previously defined. The oxo group can be reduced employing reagents such as lithium aluminum hydride, sodium borohydride, sodium cyanoborohydride, or other reducing agents familiar to those skilled in the art. This reduction can be accomplished in either protic or aprotic solvents, depending on the reducing agent of choice, and at temperatures ranging from 0 °C to reflux of the reaction mixture. The resulting OH is then converted to a good leaving group, $L^2$, through functionalization by reagents such as p-tosyl chloride, brosyl chloride or mesyl chloride or by displacement with a halogen by reagents such as thionyl chloride, thionyl bromide, phosphorus oxychloride, or other reagents familiar to those skilled in the art.

Generic Procedure III

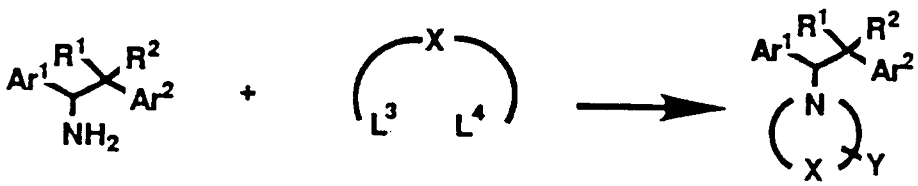

12 13 10

wherein each of $L^3$, $L^4$ is independently halogen, tosylate, brosylate, mesylate, OH; $Ar^1$, $Ar^2$, $R^1$, $R^2$, X, and Y are as previously described.

The products of the invention may be prepared by mixing the primary amine of general formula 12 with reagents of general formula 13 where X and Y have been defined previously and $L^3$ and $L^4$ represent good leaving groups. Good leaving groups are, for example, halogen, tosylate, brosylate, and mesylate. The reaction is best accomplished in a solvent like acetonitrile, dimethylformamide, dimethylsulfoxide, or tetrahydrofuran in the presence of a non-nuleophilic base such as sodium bicarbonate, potassium carbonate or other inorganic bases.

The primary amines of general formula 12 can be prepared according to the following generic procedure:

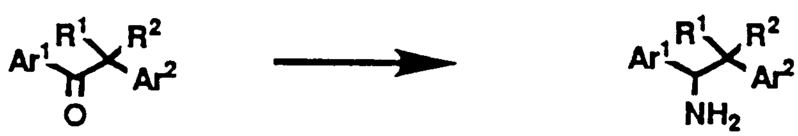

11 12

wherein $Ar^1$, $Ar^2$, $R^1$ and $R^2$ are previously defined.

One procedure for the preparation of the primary amines of general formula 12 is through the conversion of ketones of general formula 11. This conversion can be accomplished by the preparation of an oxime intermediate with hydroxylamine and reduction of the oxime with reagents such as lithium aluminum hydride, sodium borohydride, and other reagents familiar to those skilled in the art. The amine can also be prepared directly from the ketone by employing reductive amination procedures such as catalytic hydrogenation, sodium borohydride, sodium cyanoborohydride or other reducing conditions in the presence of ammonia or ammonium salts. The ketones of general formula 11 are prepared using the same generic procedures described previously for the preparation of ketones of general formula 1.

The amine 12 can also be prepared by the following generic procedure:

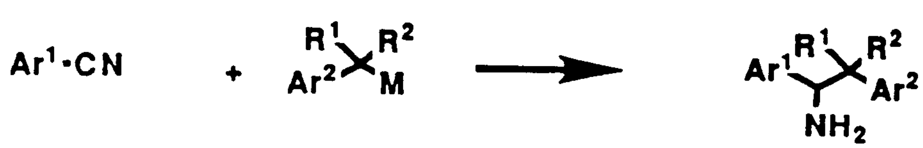

5 14 12

wherein $Ar^1$, $Ar^2$, $R^1$, $R^2$ and M have been previously defined.

Another process that can be used to generate the amines of general formula 12 starts with nitriles of general formula 5 where $Ar^1$ is as defined previously. This nitrile is treated with an organometallic reagent of general formula 14 where $Ar^2$ and $R^1$ are as defined previously and M represents a metal or metal complex such as Li, MgCl, MgBr, CuCl, or CuBr. This reaction is best achieved by mixing the reagents in a solvent like toluene, tetrahydrofuran, ether, or dioxane, and the reaction temperature can vary from 0 °C to reflux of the reaction mixture. The intermediate imine reaction product is isolated under anhydrous conditions and immediately reduced to the primary amine using reagents such as sodium borohydride, sodium cyanoborohydride, lithium aluminum hydride or hydrogen with appropriate catalysts, in a protic or aprotic solvent that is compatible with the reducing agent.

The following Examples I-XX are detailed descriptions of the methods of preparation of compounds of Formula I. These detailed preparations fall within the scope of, and serve to exemplify, the above described Generic Procedures which form part of the invention. These Examples I-XX are presented for illustrative purposes only and are not intended as a restriction on the scope of the invention. All parts are by weight unless otherwise indicated. Most of the commercially available starting materials were obtained from Aldrich Chemical Company, Milwaukee, Wisconsin.

## Example I

2-(4-Methylphenyl)acetophenone

Benzylmagnesium chloride (21.4 ml, 2 M solution in tetrahydrofuran) was cooled to 0 °C under argon. A solution of p-tolunitrile (5 gm) in anhydrous tetrahydrofuran (25 ml) was added dropwise to the Grignard reagent. After the addition, the mixture was heated to reflux and the heating continued for 20 hours. The reaction mixture was cooled in an ice bath and treated dropwise with cold 6 N hydrochloric acid (50 ml). The mixture was heated to reflux and the heating continued for 4 hours. The reaction mixture was allowed to cool to room temperature. The mixture was extracted with ether (3 X 75 ml) and methylene chloride (2 X 50 ml). The organic solutions were combined, dried over magnesium sulfate, and concentrated on a rotary evaporator to yield a yellow solid. The product was purified by distillation on a Kugelrohr apparatus (110-120 °C @ 0.04 mm Hg) to yield a colorless oil which solidified upon standing.

## Example II

1-[1-(4-Methylphenyl)-2-phenylethyl]piperidine

2-(4-Methylphenyl)acetophenone (3 gm) and p-toluenesulfonic acid (2.71 gm) were combined with toluene (50 ml). Piperidine (14.1 ml) was added all at once and the mixture heated to reflux. The heating was continued for 20 hours, with removal of the water that was generated during the condensation through use of a Dean-Stark trap. The reaction solution was allowed to cool to room temperature and diluted with ether (50 ml). The organic solution was washed with water (2 X 25 ml), dried over magnesium sulfate and concentrated on a rotary evaporator. The resulting yellow oil was dissolved in ethanol (75 ml) and treated all at once with glacial acetic acid (5 ml) and acetic anhydride (1 ml). Sodium cyanoborohydride (900 mg) was added to the solution and the mixture heated to reflux. The heating was continued for 20 hours. The solution was allowed to cool to room temperature and treated with water (75 ml), followed by enough ammonium hydroxide to make the solution basic. The resulting solution was extracted with ether (3 X 50 ml) and the combined ether extracts were extracted with cold 3.5 N sulfuric acid (3 X 30 ml). The combined acid solutions were made basic by the addition of 50% sodium hydroxide solution. The aqueous mixture was extracted with ether (3 X 50 ml), and the combined ether extracts dried over magnesium sulfate. The organic solution was concentrated on a rotary evaporator to yield a yellow oil. The oil was distilled on a Kugelrohr apparatus (100-110 °C @ 0.03 mm Hg) to yield a colorless oil. Analytical data are reported in Table I.

## Example III

1-[1-Phenyl-2-(2-pyridyl)ethyl]piperidine

2-(2-pyridyl)acetophenone (4 gm) and p-toluenesulfonic acid (150 mg) were combined with toluene (50 ml). Piperidine (10 ml) was added all at once and the mixture heated to reflux. The heating was continued

for 20 hours, with removal of the water that was generated during the condensation through use of a Dean-Stark trap. The reaction solution was allowed to cool to room temperature and the mixture concentrated on a rotary evaporator. The resulting yellow oil was distilled on a Kugelrohr apparatus (120 °C @ 0.05 mm Hg) to yield a yellow oil. The oil was dissolved in ethanol (25 ml) and sodium borohydride (3 gm) was added to the solution. The solution was heated to reflux and the heating continued for 4 hours. The solution was allowed to cool to room temperature and was treated with water. The resulting solution was extracted with ether and the combined ether solutions washed with water. The ether solution was dried over magnesium sulfate and concentrated on a rotary evaporator. The residue was distilled on a Kugelrohr apparatus (120 °C @ 0.05 mm Hg) to give the product as a yellow oil. Analytical data are reported in Table I.

Example IV

alpha-(1-phenylcyclohexyl)benzylamine

Phenylmagnesium bromide (18 ml, 3 M solution in tetrahydrofuran) was cooled to 0 °C under argon. A solution of 1-phenylcyclopropanecarbonitrile (7 gm) in toluene (50 ml) was added dropwise to the Grignard reagent. After the addition, the mixture was heated to reflux and the heating continued for 4 hours. The reaction mixture was cooled in an ice bath and treated dropwise with acetic acid (5 ml). The solid material was filtered and the filtrated concentrated on a rotary evaporator. The crude oil was dissolved in ethanol (50 ml) and treated all at once with sodium borohydride (3 gm). The solution was heated to reflux and the heating continued for 16 hours. The solution was allowed to cool to room temperature and concentrated on a rotary evaporator. The residue was dissolved in a mixture of ether and water and the layers separated. The ether solution was extracted with 6 N hydrochloric acid and the combined aqueous solutions were chilled in an ice bath and made basic with 10% sodium hydroxide. The aqueous solution was extracted with ether and the combined ether solutions were dried over magnesium sulfate, then concentrated on a rotary evaporator. The residue was distilled on a Kugelrohr apparatus (100 °C @ 0.03 mm Hg) to provide a colorless oil.

Example V

1-[1-(1-phenylcyclopropyl)benzyl]piperidine

Alpha-(1-phenylcyclohexyl)benzylamine (4 gm) was combined with potassium carbonate (2 gm) in acetonitrile (25 ml). 1,5-Dibromopentane was added to the mixture all at once and the mixture was heated to reflux. The heating was continued for 20 hours. The mixture was allowed to cool to room temperature and treated with water (50 ml). The solution was extracted with ether (3 X 75 ml) and the combined ether solutions washed with water (2 X 25 ml) and dried over magnesium sulfate. The ether solution was concentrated on a rotary evaporator and the residue distilled on a Kugelrohr apparatus (100 °C @ 0.03 mm Hg) to give a colorless oil. The oil was dissolved in cyclohexane and purified by preparative centrifugally accelerated radial thin layer chromatography on silica gel using 30% ethyl acetate in cyclohexane as an eluant to give the product as a colorless oil. Analytical data are reported in Table I.

Example VI

1,2-Diphenyl-2-(1-piperidinyl)ethanone

2-Bromo-2-phenylacetophenone (2 gm) was dissolved in acetonitrile (25 ml) and treated all at once with piperidine (5 ml). The solution was heated to reflux and the heating was continued for 2 hours. The mixture was allowed to cool to room temperature and was partitioned between ether (75 ml) and water (75 ml). The ether solution was extracted with 6 N hydrochloric acid (3 X 15 ml) and the combined acid solutions cooled in an ice bath. The cold acid solution was made basic with concentrated ammonium hydroxide and the mixture extracted with ether (3 X 25 ml). The combined ether solutions were dried over magnesium sulfate and concentrated on a rotary evaporator to give the product as a white foam. Analytical data are reported in Table I.

Example VII

1-(1,2-Diphenyl-2-hydroxyethyl)piperidine

1,2-Diphenyl-2-(1-piperidyl)ethanone was dissolved in ethanol (15 ml) and treated all at once with sodium borohydride (0.5 g). The solution was heated to reflux and the heating continued for 16 hours. The reaction solution was allowed to cool to room temperature, treated with water (50 ml) and extracted with ether (3 X 50 ml). The combined ether solutions were dried over magnesium sulfate and concentrated on a rotary evaporator to a colorless oil. Analytical data are reported in Table I.

Example VIII

2,2-Dimethylphenylacetamide

2,2-Dimethylphenylacetic acid (5 gm) was combined with thionyl chloride (25 ml) and the solution heated to reflux. The heating was continued for 2 hours. The solution was allowed to cool to room temperature and the excess thionyl chloride removed by concentration on a rotary evaporator. The residue was suspended in cyclohexane (25 ml) and the solvent removed by evaporation on a rotary evaporator. The residue was added to cold ammonium hydroxide (50 ml) and the mixture stirred for two hours. The precipitate was filtered and dried in a vacuum to give the product as a white powder.

Example IX

2,2-Dimethyl-1,2-diphenylethylamine

2,2-Dimethyl-2-phenylacetamide (5 gm) was combined with phosphorus oxychloride and the solution heated to reflux. Heating was continued for 4 hours and the reaction mixture was allowed to cool to room temperature. The solution was poured onto ice and the resulting mixture extracted with ether. The combined ether extracts were dried over magnesium sulfate and concentrated on a rotary evaporator to yield a yellow oil. The oil was dissolved in toluene (50 ml) and added dropwise to a solution of phenylmagnesium bromide (8 ml, 3 M in tetrahydrofuran). The solution was heated to reflux and the heating continued for 4 hours. The reaction solution was cooled in an ice bath and treated with water (5 ml). The resulting mixture was filtered and the filtrated dried over magnesium sulfate and concentrated on a rotary evaporator. The resulting oil was dissolved in ethanol (50 ml) and treated with sodium borohydride (3 gm). The resulting solution was heated to reflux and the heating continued for 16 hours. The solution was allowed to cool to room temperature, treated with water (50 ml) and extracted with ether (3 X 100 ml). The combined ether extracts were dried over magnesium sulfate and concentrated on a rotary evaporator to yield a yellow oil. The oil was distilled on a Kugelrohr apparatus (100 ° C @ 0.04 mm Hg) to obtain the product as a colorless oil.

Example X

1-(2,2-Dimethyl-1,2-diphenylethyl)piperidine

2,2-Dimethyl-1,2-diphenylethylamine (1 gm) was combined with potassium carbonate (2 gm) in acetonitrile (25 ml). 1,5- Dibromopentane (2 ml) was added to the mixture all at once and the mixture was heated to reflux. The heating was continued for 20 hours. The mixture was allowed to cool to room temperature and treated with water (50 ml). The solution was extracted with ether (3 X 75 ml) and the combined ether solutions washed with water (2 X 25 ml). The ether solution was extracted with 6 N hydrochloric acid (3 X 25 ml) and the combined acid solutions were cooled in an ice bath. The acid solution was made basic with 10% sodium hydroxide solution and the aqueous mixture extracted with ether (3 X 50 ml). The combined ether solutions were dried over magnesium sulfate and concentrated on a rotary evaporator. The residue was distilled on a Kugelrohr apparatus (120 ° C @ 0.01 mm Hg) to give a colorless oil. Analytical data are reported in Table I.

Example XI

2,2-Dimethyl-2-phenyl-1-(2-thienyl)ethanone

2,2-Dimethylphenylacetic acid (5 gm) was combined with phosphorus pentoxide (5 gm) in thiophene (25 ml). The solution was heated to reflux and the heating continued for 3 hours. The solution was allowed to cool to room temperature, treated with water (25 ml) and ether (50 ml) and the two layers were separated. The ether solution was extracted with 10% sodium hydroxide solution (2 X 25 ml). The ether solution was dried over magnesium sulfate and concentrated on a rotary evaporator. The residual oil was distilled on a Kugelrohr apparatus (80 °C @ 0.05 mm Hg) to provide the product as an amber oil.

Example XII

2,2-Dimethyl-2-phenyl-1-(2-thienyl)ethanol

2,2-Dimethyl-2-phenyl-1-(2-thienyl)ethanone (1 gm) was dissolved in ethanol (50 ml) and treated with sodium borohydride (1 gm). The solution was heated to reflux and the heating continued for 17 hours. The solution was allowed to cool to room temperature and was concentrated on a rotary evaporator. The residue was dissolved in a mixture of water (25 ml) and ether (25 ml) and the resulting layers separated. The ether solution was dried over magnesium sulfate and concentrated on a rotary evaporator. The residue was distilled on a Kugelrohr apparatus (80 °C @ 0.05 mm Hg) to give the product as a yellow oil.

Example XIII

1-[2,2-Dimethyl-2-phenyl-1-(2-thienyl)ethyl]piperidine

2,2-Dimethyl-2-phenyl-1-(2-thienyl)ethanol (0.8 gm) was combined with thionyl chloride (1 ml) and triethylamine (1 ml) in methylene chloride (25 ml). The solution was heated to reflux and the heating continued for 20 hours. The reaction mixture was allowed to cool to room temperature and was washed with water (2 X 15 ml). The organic solution was dried over magnesium sulfate and concentrated on a rotary evaporator. The resulting oil was dissolved in acetonitrile (25 ml) and treated with potassium carbonate (1 gm) and piperidine (1 ml). The mixture was heated to reflux and the heating continued for 22 hours. The mixture was allowed to cool to room temperature and was treated with water (25 ml). The solution was extracted with ether (3 X 25 ml) and the combined ether solutions were extracted with 6 N hydrochloric acid (3 X 10 ml). The combined acid solutions were cooled in an ice bath and made basic by the addition of 15% sodium hydroxide solution. The aqueous solution was extracted with ether (3 X 25 ml) and the combined ether solutions were dried over magnesium sulfate and concentrated on a rotary evaporator. The resulting oil was distilled on a Kugelrohr apparatus (120 °C @ 0.03 mm Hg) to give the product as a yellow oil. Analytical data are reported in Table I.

Example XIV

2-Phenyl-1-(2-thienyl)ethanone

Phenylacetic acid (5 gm) was combined with phosphorus pentoxide (5 gm) in thiophene (25 ml) and the mixture heated to reflux. The heating was continued for 2 hours, the reaction mixture was allowed to cool to room temperature. The solution was decanted from the solids into methylene chloride (25 ml). The organic solution was washed with 10% sodium hydroxide solution (2 X 10 ml) and dried over magnesium sulfate. The organic solution was concentrated on a rotary evaporator. The residue was distilled on a Kugelrohr apparatus (80 °C @ 0.04 mm Hg) to give the product as a colorless oil which solidified upon standing.

Example XV

1-[2-Phenyl-1-(2-thienyl)ethyl]-1,2,3,6-tetrahydropyridine

2-Phenyl-1-(2-thienyl)ethanone (1 gm) was combined with 1,2,3,6-tetrahydropyridine (5 ml) in toluene (10 ml) and cooled in an ice bath. A solution of titanium tetrachloride (3 ml) in toluene (10 ml) was added dropwise to the cold solution of the ketone. After the addition, the reaction solution was heated to reflux and

the heating continued for 1 hour. The solution was allowed to cool to room temperature and treated with water (25 ml). The resulting mixture was extracted with ether (3 X 25 ml) and the combined ether solutions were dried over magnesium sulfate and concentrated on a rotary evaporator. The residue was distilled on a Kugelrohr apparatus (100 °C @ 0.04 mm Hg) to obtain a yellow oil. The oil was dissolved in ethanol (25 ml) and treated all at once with acetic acid (5 ml), followed by sodium cyanoborohydride (1 gm). The solution was heated to reflux and the heating continued for 16 hours. The solution was allowed to cool to room temperature and treated with water (50 ml). The resulting solution was extracted with ether (3 X 50 ml) and the combined ether solutions dried over magnesium sulfate and concentrated on a rotary evaporator. The residue was distilled on a Kugelrohr apparatus (110 °C @ 0.03 mm Hg) to give a brown oil. The oil was dissolved in cyclohexane and the product purified by preparative centrifugally accelerated radial thin layer chromatography on silica gel using 30% ethyl acetate in cyclohexane as an eluant. The product was obtained as a yellow oil. Analytical data are reported in Table I.

Example XVI

1,2,-Diphenyl-1-butanone oxime

1,2,-Diphenyl-1-butanone was combined with hydroxylamine hydrochloride (5 gm) and sodium acetate (5 gm) in ethanol (100 ml) and the mixture heated to reflux. The heating was continued for 12 hours. The hot solution was treated with water until the solution became turbid. The solution was allowed to cool to room temperature. The white crystals of product that separated during cooling were filtered and air dried.

Example XVII

1,2-Diphenyl-1-aminobutane

1,2,-Diphenyl-1-butanone oxime (2 gm) was combined with glacial acetic acid (2 ml) and 10% palladium on charcoal (100 mg) in ethanol (10 ml). The mixture was hydrogenated at 50 p.s.i at room temperature for 15 hours. The catalyst was removed by filtration and the filtrate was treated with water (50 ml). The solution was made basic by the addition of 10% sodium hydroxide solution and extracted with ether (3 X 50 ml). The combined ether solutions were extracted with 6 N hydrochloric acid (3 X 25 ml) and the combined acid solutions were made basic by the addition of 10% sodium hydroxide. This aqueous solution was extracted with ether (3 X 50 ml) and the combined ether solutions dried over magnesium sulfate and concentrated on a rotary evaporator to give the product as a yellow oil.

Example XVIII

1-(1,2-Diphenylbutyl)piperidine

1,2-Diphenyl-1-aminobutane (1.2 gm) was combined with potassium carbonate (2 gm) in acetonitrile (25 ml). The mixture was treated all at once with 1,5-dibromopentane (1.2 gm) and heated to reflux. Heating was continued for 20 hours. The mixture was allowed to cool to room temperature and treated with water (50 ml). The solution was extracted with ether (3 X 25 ml). The combined ether solutions were washed with water (2 X 25 ml) and dried over magnesium sulfate. The solution was concentrated on a rotary evaporator and the residue distilled on a Kugelrohr apparatus (100 °C @ 0.05 mm Hg) to obtain the product as a colorless oil. Analytical data are reported in Table I.

Example XIX

1-(1,2-Diphenylethyl)-1,2,3,6-tetrahydropyridine

1,2-Diphenylethanol (3 gm) was combined with triethylamine (2.5 ml) in methylene chloride (50 ml). The solution was treated with thionyl chloride (1.22 ml) and stirred at room temperature for 2 hours. Water (20 ml) was added to the solution and the resulting layers separated. The methylene chloride solution was washed with water (20 ml) and dried over magnesium sulfate. The solution was concentrated on a rotary evaporator to give a colorless oil which solidified upon standing. This solid was combined with potassium carbonate (1 gm) in acetonitrile (25 ml) and treated with 1,2,3,6-tetrahydropyridine (5 ml). The mixture was heated to reflux and the heating continued for 20 hours. The mixture was allowed to cool to room

temperature and a mixture of water (50 ml) and ether (75 ml) were added. The resulting layers were separated and the ether solution washed with water (2 X 25 ml). The ether solution was dried over magnesium sulfate and concentrated on a rotary evaporator. The residual oil was dissolved in ether (25 ml) and extracted with a solution of sulfuric acid (1 ml) in water (10 ml). The aqueous layer was chilled in an ice bath and made basic with solid sodium hydroxide. The resulting mixture was extracted with ether (3 X 20 ml) and the combined ether solutions dried over magnesium sulfate and concentrated on a rotary evaporator. The residue was distilled on a Kugelrohr apparatus (100 °C @ 0.05 mm Hg) to give the product as an amber oil. Analytical data are reported in Table I.

Example XX

(+)- and (-)-1-(1,2-Diphenylethyl)piperidine

(±)-(1,2-Diphenylethyl)piperidine (1.5 gm) was combined with L-tartaric acid (0.84 gm) in water (25 ml) and heated to dissolve the solids. The solution was allowed to cool to room temperature to effect crystallization. The first crop of crystals were collected by filtration and designated (+)-(1,2-diphenylethyl)-piperidine. The filtrate was concentrated by heating to boiling on a hot plate. The solution was allowed to cool to room temperature to effect crystallization. The second crop of crystals were collected by filtration and designated (-)-(1,2-diphenylethyl)piperidine. The specific rotation for the (+)- isomer in methylene chloride is $[\alpha]_D$ = +1.4. The specific rotation for the (-)- isomer in methylene chloride is $[\alpha]_D$ = -1.4. Analytical data are reported in Table I.

Table I is a list of 33 specific compounds of most interest within Formula I. The preparation of 12 of the compounds in Table I is described in detail in Example Procedures I-XX, above. The remaining compounds may likewise be prepared in accordance with the above-described Example Procedures, as noted in Table I.

| Compound Number | Name | Structure | Method of Preparation | Elemental Analysis Theo | Found | MP / BP (phys state) |
|---|---|---|---|---|---|---|
| 1 | (±)-1-(1,2-Diphenylethyl)-piperidine | 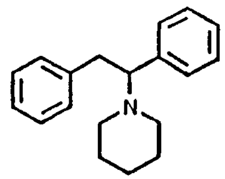 | II | C 85.40<br>H 8.75<br>N 5.24 | 85.26<br>9.03<br>5.31 | 100°C @ 0.05mmHg (Yellow Oil) |
| 2 | (±)-1,2-Diphenyl-2-(1-piperidinyl)ethanone | 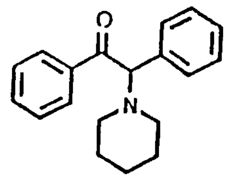 | VI | NA | | 100°C @ 0.05mmHg (Oil) |
| 3 | (-)-1-(1,2-Diphenylethyl)-piperidine | 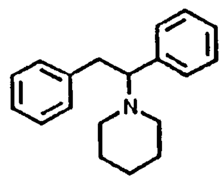 | XX | C 84.27<br>H 8.78<br>N 5.17 | 84.03<br>8.80<br>5.13 | 100°C @ 0.05mmHg (Oil) |
| 4 | (+)-1-(1,2-Diphenylethyl)-piperidine | 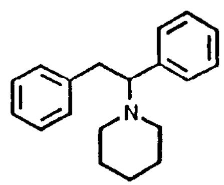 | XX | C 84.27<br>H 8.78<br>N 5.17 | 84.66<br>8.80<br>5.14 | 100°C @ 0.05mmHg (Oil) |

| Compound number | Name | Structure | Method of preparation | Elemental Analysis Theo | Elemental Analysis Found | MP / BP (phys state) |
|---|---|---|---|---|---|---|
| 5 | (±)-1-[2-Phenyl-1-(2-thienyl)-ethyl]piperidine Hydrosulfate | 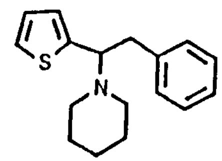 | XIV,XV | C 54.46<br>H 6.34<br>N 3.73 | 54.48<br>6.12<br>3.69 | >300°C (white solid) |
| 6 | (±)-1-(1,2-Diphenylethyl)-1,2,3,6-tetrahydropyridine | 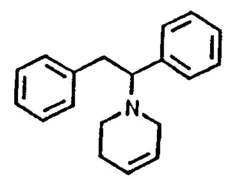 | XIX | C 85.47<br>H 8.07<br>N 5.24 | 85.75<br>7.88<br>5.47 | 80°C @ 0.04mmHg (Yellow Oil) |
| 7 | 1-(1,2-Diphenylethenyl)-piperidine | 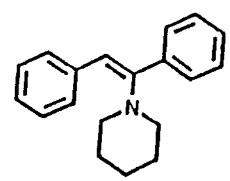 | III | NA | | 80°C @ 0.04mmHg (Yellow Oil) |
| 8 | 1-[Phenyl-2-(2-pyridinyl)-ethenyl]piperidine | 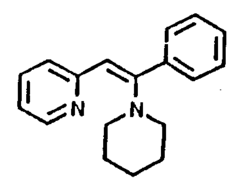 | III | NA | | 80°C @ 0.04mmHg (Yellow Oil) |

| Compound Number | Name | Structure | Method of Preparation | Elemental Analysis Theo | Found | MP / BP (phys state) |
|---|---|---|---|---|---|---|
| 9 | (±)-1-[1-Phenyl-2-(2-pyridinyl) ethyl]piperidine | 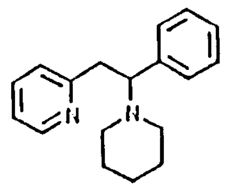 | III | C 81.15<br>H 8.32<br>N 10.51 | 80.73<br>8.33<br>10.38 | 120°C @ 0.05mmHg (Yellow Oil) |
| 10 | (±)-1-(1,2-Diphenylethyl)-1H-azepine | 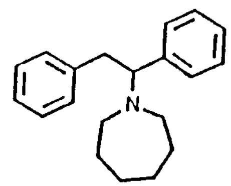 | V | C 84.60<br>H 9.05<br>N 4.93 | 84.50<br>8.88<br>4.86 | 100°C @ 0.05mmHg (Yellow Oil) |
| 11 | (±)-1-(1,2-Diphenylbutyl)-piperidine | 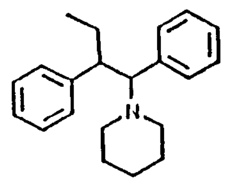 | XI,XII,XIII | NA | | 120°C @ 0.05mmHg (Colorless Oil) |
| 12 | (±)-1-(1,2-Diphenyl-3-methyl-butyl)piperidine | 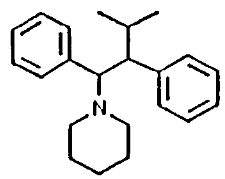 | XI,XII,XIII | NA | | 120°C @ 0.05mmHg (Colorless Oil) |

| Compound Number | Name | Structure | Method of Preparation | Elemental Analysis Theo | Found | MP / BP (phys state) |
|---|---|---|---|---|---|---|
| 13 | (±)-1-(1,2-diphenyl-1-hydroxyethyl)piperidine | 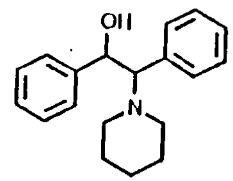 | VI, VII | NA | | 110°C @ 0.05mmHg (Colorless Oil) |
| 14 | (±)-1-(1,2-Diphenylethyl)-4-methylpiperazine | 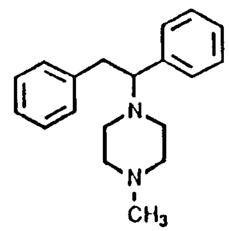 | II | C 81.38<br>H 8.62<br>N 9.98 | 81.41<br>8.74<br>9.72 | 120°C @ 0.05mmHg (Yellow Oil) |
| 15 | (±)-1-[2-Phenyl-1-(2-thienyl)-ethyl]-1,2,3,6-tetrahydropyridine | 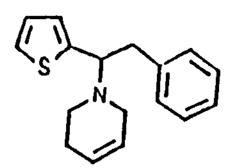 | XIV, XV | C 74.79<br>H 7.16<br>N 5.13 | 74.41<br>6.94<br>5.11 | 110°C @ 0.03mmHg (Yellow Oil) |
| 16 | (±)-1-(1,2-Diphenylethyl)-morpholine | 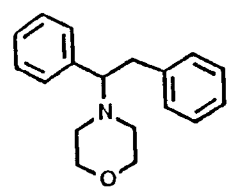 | II | C 81.86<br>H 7.91<br>N 5.23 | 80.80<br>8.07<br>5.28 | 110°C @ 0.03mmHg (Colorless Oil) |

| Compound Number | Name | Structure | Method of Preparation | Elemental Analysis Theo | Elemental Analysis Found | MP / BP (phys state) |
|---|---|---|---|---|---|---|
| 17 | (±)-1-[1-(1-Phenylcyclo-pentyl)benzyl]piperidine | 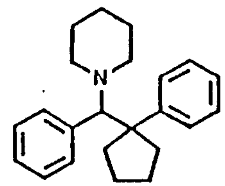 | IV,V | C 85.56<br>H 8.85<br>N 4.15 | 85.60<br>8.68<br>4.19 | 120°C @<br>0.03mmHg<br>(Colorless Oil) |
| 18 | (±)-1-[1,2-Di(4-methoxy-phenyl)ethyl]piperidine | 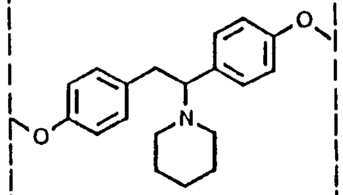 | I, II | C 77.50<br>H 8.36<br>N 4.30 | 77.11<br>8.34<br>4.11 | 150°C @<br>0.06mmHg<br>(Yellow Oil) |
| 19 | (±)-1-[1-(1-Phenylcyclo-hexyl)benzyl]piperidine | 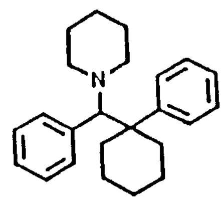 | IV,V | C 86.43<br>H 9.36<br>N 4.19 | 86.42<br>9.54<br>4.20 | 130°C @<br>0.06mmHg<br>(Yellow Oil) |
| 20 | (±)-1-(1,2-Diphenylethyl)-pyrrolidine | 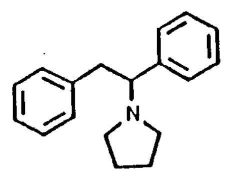 | II | C 86.00<br>H 8.42<br>N 5.57 | 85.56<br>8.43<br>5.47 | 100°C @<br>0.06mmHg<br>(Colorless Oil) |

| Compound Number | Name | Structure | Method of Preparation | Elemental Analysis Theo | Found | MP / BP (phys state) |
|---|---|---|---|---|---|---|
| 21 | (±)-1-[1-(4-Methylphenyl)-2-phenylethyl]piperidine | 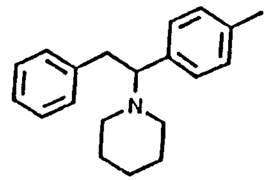 | I, II | C 84.87<br>H 9.04<br>N 4.94 | 84.60<br>9.26<br>5.29 | 100°C @<br>0.03mmHg<br>(Colorless Oil) |
| 22 | (±)-1-[1-(4-Methoxyphenyl)-2-phenylethyl]piperidine | 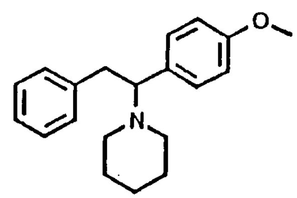 | I, II | C 79.85<br>H 8.57<br>N 4.65 | 79.89<br>8.87<br>4.95 | 100°C @<br>0.03mmHg<br>(Colorless Oil) |
| 23 | (±)-1-[1-(1-Phenylcyclopropyl)benzyl]piperidine | 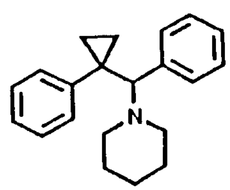 | IV, V | C 86.01<br>H 8.60<br>N 4.77 | 86.05<br>8.52<br>4.70 | 120°C @<br>0.03mmHg<br>(Colorless Oil) |
| 24 | (±)-1-(1,2-Diphenylethyl)-3,5-dimethylpiperidine | 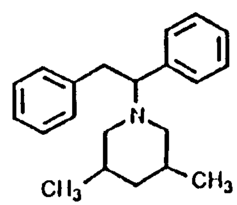 | II | C 85.95<br>H 9.27<br>N 4.77 | 85.68<br>9.35<br>4.83 | 100°C @<br>0.03mmHg<br>(Yellow Oil) |

| Compound Number | Name | Structure | Method of Preparation | Elemental Analysis Theo | Elemental Analysis Found | MP / BP (phys state) |
|---|---|---|---|---|---|---|
| 25 | (±)-1-[2,2-Dimethyl-2-phenyl-1-(2-thienyl)ethyl]piperidine | 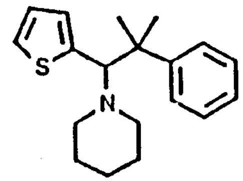 | XI,XII,XIII | C 74.12<br>H 9.14<br>N 5.08 | 74.08<br>9.16<br>5.06 | 120°C @ 0.03mmHg (Yellow Oil) |
| 26 | (±)-1-[1-(3-Methoxyphenyl)-2-phenylethyl]piperidine | 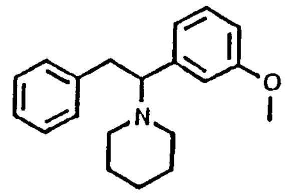 | I,II | C 81.31<br>H 8.53<br>N 4.74 | 81.11<br>8.47<br>4.77 | 120°C @ 0.01mmHg (Colorless Oil) |
| 27 | (±)-1-[1-(4-Fluorophenyl)-2-phenylethyl]piperidine | 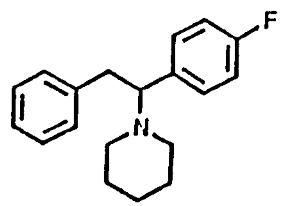 | I,II | C 80.53<br>H 7.83<br>N 4.94 | 80.40<br>7.96<br>4.90 | 110°C @ 0.01mmHg (Colorless Oil) |
| 28 | (±)-1-(2,2-Dimethyl-1,2-diphenylethyl)piperidine | 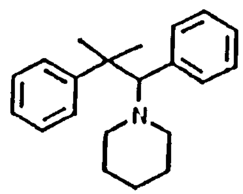 | X,XI,XII | C 85.42<br>H 9.28<br>N 4.74 | 85.25<br>8.87<br>4.80 | 120°C @ 0.01mmHg (Colorless Oil) |

| Compound Number | Name | Structure | Method of Preparation | Elemental Analysis Theo | Elemental Analysis Found | MP / BP (phys state) |
|---|---|---|---|---|---|---|
| 29 | (±)-1-[1-(3-Methylphenyl)-2-phenylethyl]piperidine | 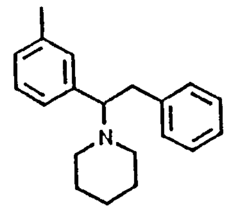 | I, II | C 85.97<br>H 9.02<br>N 5.01 | 85.69<br>9.25<br>5.07 | 100°C @<br>0.05mHg<br>(Colorless Oil) |
| 30 | (±)-1-[1-(2-Furyl)-2-phenylethyl]piperidine | 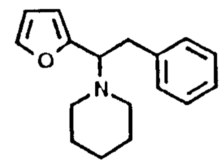 | IV,V | C 78.84<br>H 8.32<br>N 5.40 | 78.82<br>8.29<br>5.15 | 100°C @<br>0.05mmHg<br>(Yellow Oil) |
| 31 | (±)-1-[1-(2-Chlorophenyl)-2-phenylethyl]piperidine | 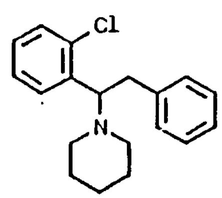 | IV,V | C 80.82<br>H 8.54<br>N 4.70 | 80.85<br>8.59<br>4.55 | 120°C @<br>0.04mmHg<br>(Yellow Oil) |
| 32 | (±)-[1-(2-methoxyphenyl)-2-phenylethyl]piperidine | 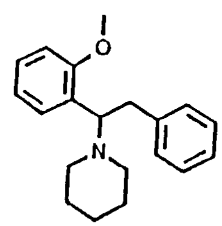 | IV,V | C 76.10<br>H 7.39<br>N 4.67 | 75.91<br>7.46<br>4.60 | 100°C @<br>0.05mmHg<br>(Yellow Oil) |

| Compound Number | Name | Structure | Method of Preparation | Elemental Analysis Theo | Elemental Analysis Found | MP / BP (phys state) |
|---|---|---|---|---|---|---|
| 33 | (±)-1-[1-(1-Naphthyl)-2-phenylethyl]piperidine | 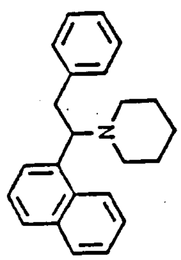 | IV,V | C 81.68<br>H 8.22<br>N 4.11 | 81.51<br>7.87<br>3.64 | 140°C @ 0.05mmHg (Yellow Oil) |

BIOLOGICAL EVALUATION

Prevention of the neurodegenerative consequences associated with conditions of hypoxia or ischemia may be accomplished with administration of a compound of Formula I. In particular, the compounds 1-33 in Table I have been evaluated in biological assays to measure the inhibition of hypoxia- or ischemia-induced neuronal toxicity. Compounds 1-33, as well as some earlier-known PCP-agonist compounds, were evaluated by various in vivo and in vitro assays to determine compound activity as an NMDA antagonist or PCP

agonist. These biological assays, described below, included a radioreceptor assay, a chronic hypoxic insult assay, an acute azide toxicity assay, an NMDA/KA/QUIS antagonist assay, a forebrain ischemia assay, and a behavioral assay.

## Radioreceptor Assay

Compounds 1-33 were compared against PCP and TCP in an assay to determine the relative potency of the compounds interacting with PCP receptors. To determine the effect of the compounds in a PCP receptor assay, crude membrane preparations were prepared by homogenizing whole rat brains in 30 ml of ice-cold 5 mM Tris-HCl, pH 7.4 (Tris buffer), with a Brinkman Polytron (setting 6, 15 sec). The homogenate was centrifuged twice at 20,000 x g for 15 min at 4°C with an intervening resuspension of the pellet in cold Tris buffer. The final pellet was resuspended in Tris buffer to obtain a final concentration of 0.1 g of tissue per ml. Incubation tubes were prepared in triplicate and contained 0.1 ml of tissue suspension, 1 nM of $^3$H-TCP and varying concentrations of displacing ligand (0.1 - 30,000 nM) in a final volume of 0.5 ml. After a 1 hour incubation, the contents of the test tubes were filtered through Schleier & Schuell #32 filters, which had been presoaked for at least 2 hours in 0.05% polyethyleneimine. The test tubes were rinsed twice and the filters once with 4 ml of tris buffer. Radioactivity on the filters was determined by liquid scintillation spectrometry. Specific binding was defined as the total amount of tritiated compound bound minus the amount bound in the presence of 10 $\mu$M of TCP compound. $K_i$ values were determined using the method of Cheng & Prusoff [Biochem. Pharmacol., 22, 3099-3108 (1973)].

Table II

| Test Compound | $K_i$ apparent (nM) (units ± SEM) |
|---|---|
| PCP | 96 ± 15 |
| TCP | 20 ± 6 |
| Compound No. 1 | 39 ± 11 |
| Compound No. 2 | 1800 ± 100 |
| Compound No. 3 | 2900 ± 600 |
| Compound No. 4 | 25 ± 8 |
| Compound No. 5 | 90 ± 20 |
| Compound No. 6 | 54 ± 3 |
| Compound No. 7 | >30000 |
| Compound No. 8 | >30000 |
| Compound No. 9 | 280 ± 40 |
| Compound No. 10 | 180 ± 1 |
| Compound No. 11 | 2300 ± 500 |
| Compound No. 12 | 670 ± 30 |
| Compound No. 13 | 410 ± 10 |
| Compound No. 14 | >30000 |
| Compound No. 15 | 45 ± 14 |
| Compound No. 16 | 4400 ± 100 |
| Compound No. 17 | 4300 ± 200 |
| Compound No. 18 | >30000 |
| Compound No. 19 | 690 ± 70 |

| | |
|---|---|
| Compound No. 20 | 16000 ± 2600 |
| Compound No. 21 | 1800 ± 300 |
| Compound No. 22 | 8100 ± 500 |
| Compound No. 23 | >30000 |
| Compound No. 24 | 850 ± 40 |
| Compound No. 25 | >30000 |
| Compound No. 26 | 100 ± 20 |
| Compound No. 27 | 220 ± 10 |
| Compound No. 28 | 12000 ± 1000 |
| Compound No. 29 | 110 ± 30 |
| Compound No. 30 | 400 ± 10 |
| Compound No. 31 | 0.19 ± 0.11 |
| Compound No. 32 | 170 ± 10 |
| Compound No. 33 | 6000 ± 1700 |

Chronic Hypoxic Insult Assay

Compound Nos. 4 and 5 were tested for their ability to protect hippocampal neurons from hypoxia-induced cell death. Cultures of hippocampal neurons were prepared from embryonic day 17 Sprague-Dawley rats. The hippocampi were dissociated into a single cell suspension by incubation with 0.25% trypsin, 40 mg/ml DNase followed by gentle trituration through a Pasteur pipet. The cells were plated in a polylysine-coated 96-well plate and maintained in a chemically defined medium until use. The cells were grown for 2 to 3 weeks in 5% $CO_2$-in-air humidified environment at 36°C to establish a thick network of neuronal processes with numerous spontaneously active synapses. Exposure to hypoxic/anoxic environment was accomplished by placing the cultures in an anaerobic chamber, and flushing it with a mixture of 95% $N_2$ + 5% $CO_2$ gas to rapidly drop the $O_2$ tension to near zero. The $O_2$ tension was maintained at near zero using a disposable $H_2$ + $CO_2$ generator envelope with palladium catalyst. Compound No. 4 and Compound No. 5 were added to the culture medium prior to incubation in the anaerobic chamber and maintained there for 6 hours. Following 2 hours of exposure to normal $O_2$ tension, the cultures were processed for morphological and quantitive biochemical neuronal cell viability assays.

The neuronal survival assay utilized the compound MTT [(3-(4,5-dimethylthiazol-2-yl)-2,5-diphenyl tetrazolium bromide)], a pale yellow substrate that is cleaved by the mitochondrial enzyme succinate-dehydrogenase to yield a dark blue formazan product. This process requires active mitochondria, present only in live cells. Cultures of hippocampal neurons grown in 96-well plates were incubated with 1 mg/ml MTT at 36°C in a 10% $CO_2$-in-air incubator for 30-60 min. At the end of the incubation, a dark blue precipitate outlined only viable cells. The precipitate was then solubilized using 0.08 N HCl/isopropanol mixture and the absorbance measured with an ELISA plate reader (Dynatech MR600) using a test wavelength of 570 nm and a reference wavelength of 630 nm. The resulting optical density is directly proportional to the number of viable cells.

Maximum protection of neurons from hypoxic insult was obtained with 1 µM of Compound No. 4 and with 10 µM of Compound No. 5:

| Sample | Optical Density (units + SEM) |
|---|---|
| Untreated Control | 0.058 ± 0.01 |
| Hypoxia | 0.020 ± 0.01 |
| Hypoxia + 1 µM Compound No. 4 | 0.046 ± 0.01 |
| Hypoxia + 10 µM Compound No. 5 | 0.047 ± 0.01 |

Acute Azide Toxicity Assay

Compound No. 4 was tested for its ability to protect hippocampal neurons from sodium azide poisoning which selectively kills mature neurons while sparing glial cells. Neuronal cells were prepared and the cell viability assays were performed as described in the chronic hypoxia insult assay, above.

Cultures were exposed to 10 µM sodium azide for 1 hour either in the presence or absence of Compound No. 4 and immediately thereafter processed for qualitative (morphological) and quantitative viability assay. Under this acute and severe toxicity conditions, 100 µM of Compound No. 4 afforded the neurons significant protection from all death.

| Sample | Optical Density (units + SEM) |
|---|---|
| Control | 0.123 ± 0.01 |
| Sodium azide | 0.075 ± 0.01 |
| Sodium azide + 100 µM Compound No. 4 | 0.133 ± 0.01 |

NMDA/KA Antagonist Assay

A 15-day old chick embryo retina, incubated for 30 min. in a balanced salt solution (BSS) containing 1 mM Glu, developed a full lesion characteristic of an immature mouse retina following s.c. administration of Glu. Other excitotoxin agonists also produce acute lesions within 30 min., each agent being effective at a concentration proportional to its known excitatory and toxic potencies. The pattern of cellular degeneration is restricted in each case to the ganglion cell, inner plexiform and inner nuclear layers, but within these areas certain agonists induce different patterns of degeneration, the differences being most pronounced between NMA and KA. Three agonists were employed in the present test, each at a concentration established previously to be the lowest concentration required to consistently cause a fully-developed retinal lesion: KA (25 µM), Quis (50 µM), and NMA (200 µM). Compound No. 1, Compound No. 4, Compound No. 5 and other PCP-like compounds were tested at various concentrations for their ability to prevent KA, Quis or NMA neurotoxicity. Although partial blocking was observed for each antagonist at concentrations below the threshold for complete protection, the criterion used for comparing agents for antagonist potency was the concentration required to completely prevent KA, Quis or NMA from exerting any toxic activity in any specimen (n>6) studied at that concentration. Internal controls in each experiment consisted of at least six specimens being incubated with agonist alone. A typical toxic reaction had to be present in all controls and absent from all experimental specimens in order to qualify as a blocking effect. The method of tissue preparation was as follows: 15-day old chick embryos were decapitated and their eyes removed and cut into quadrants after excising the cornea and removing the lens, vitreous and iris. The retinal quadrants were then gently separated from the pigment epithelium and incubated for 30 min. at 37°C in BSS to which an agonist or agonist plus antagonist was added. The BSS contained 140 mM $Na^+$, 5.0 mM $K^+$, 0.5 mM $Ca^{++}$, 4.5 mM $Mg^{++}$, 150 mM $Cl^-$, 5.6 mM glucose and bicarbonate/phosphate buffer (pH 7.3). After incubation for 30 min., the retinal quadrants were fixed by immersion in phosphate-buffered solution containing 1.5% glutaraldehyde and 1% paraformaldehyde, then additionally fixed in 1% osmium tetroxide, dehydrated in graded ethanols, cleared in toluene and embedded in araldite. Sections were cut 1 micron thick on a Sorval ultratome and stained with methylene blue/Azure 11 for histopathological evaluation by light microscopy.

Table III

POTENCIES OF ANTAGONISTS IN BLOCKING NMV, KA OR QUIS NEUROTOXICITY

Compounds were rated according to the minimal concentration (μM) required to provide total protection against NMA (200 μM), KA (25 μM) or Quis (50 μM). Antagonists were tested over a range of concentrations from 1000 μM downward until a minimal effective concentration was established.

| Potential antagonist | vs NMA | vs KA | vs Quis* |
|---|---|---|---|
| PCP | 1 μM | No Activity | No Activity |
| Metaphit | 50 μM | No Activity | No Activity |
| Compound No. 1 | 0.125 μM | No Activity | No Activity |
| Compound No. 4 | 0.25 μM | No Activity | No Activity |
| Compound No. 5 | 0.25μM | No Activity | No Activity |

*@ up to 500 μM

Behavioral Assay

Nine compounds were tested in comparison with PCP and TCP by an in vivo assay which determined stereotypic behavior in rats treated with the compounds. Male Sprague-Dawley rats weighing 200 to 250 g were used in the behavioral experiments. Each rat was used only once. Rats were anesthetized lightly with ether before a 20-gauge needle was used to make a hole in the rat's skull for i.c.v. injection of drugs at a later date. These rats were allowed to recover for at least 1 day before being used in the behavioral assays. On the day of the experiment, rats were placed individually into plastic rat cages and allowed at least 1 hr to acclimate before testing. Drugs were administered to rats in a random, single-blind fashion. Behavioral ratings were taken at 5-min intervals up to 1 hr after drug administration i.c.v., i.p., or s.c. using the PCP rating scale as described by Sturgeon et al [Sturgeon, R.D., Fessler, R.G. and Meltzer, H.Y., "Behavioral Rating Scales for Accessing Phencyclidine-Induced Locomotor Activity, Stereotyped Behavior And Ataxia In Rats", European J. Pharmacol. 59 169, (1970)].Briefly, the rating scale for stereotyped behavior is: 0, inactive or nonrepetitive activity; 1, sniffing, grooming or rearing; 2, nondirectional movements, and occasional reciprocal forepaw treading; 3, circling or head-weaving behavior or backpeddling; 4, rapid and continuous circling or head-weaving behavior, assuming a praying posture or gagging; and 5, dyskinetic extension and flexion of limbs, head and neck or head-weaving greater than in "4".

Dose-response curves for each treatment were determined at the time of maximal behavioral effect. Peak effects were found 5 min after i.c.v. administration of PCP (25-20000 nmol/rat). Peak effects of PCP (2.0-32 mg/kg) after i.p. administration were observed at 15 min. A rating of 5 in the PCP-rating scale was considered as complete stereotyped behavior, that is, a 100% response. At least 21 rats (at least seven rats/dose) were used to determine each dose-response curve and $ED_{50}$ values. $ED_{50}$ values and dose-response curves were evaluated using a computerized Finney assay [Statistical Methods In Biological Assays, 2nd Edn., Hatner Pub. Co., New York (1964)].

The ability of the tested compounds to induce stereotyped behavior was assessed at 2.5, 5 and 10 minutes and thereafter every 5 min up to 1 hr after i.c.v., i.p., or s.c. administration. Results are summarized in Table IV:

Table IV

| Test Compound | Stereotyped Behavior $ED_{50}$ | | |
|---|---|---|---|
| | i.c.v.[a,b] | s.c.[c] | i.p.[d] |
| PCP | 150(120-170) | NT | NT |
| Compound No. 1 | 220(140-210) | 2.9(2.4-3.6) | 2.0(1.1-3.3) |
| Compound No. 3 | NT[e] | >40 | NT |
| Compound No. 4 | 120(100-140) | 0.78(0.60-1.0) | 2.1(1.2-3.5) |
| Compound No. 5 | NT | 3.5(2.7-4.6) | NT |
| Compound No. 6 | 100(81-120) | NT | NT |
| Compound No. 12 | NT | >50 | NT |
| Compound No. 13 | NT | 11(8.2-14) | NT |
| Compound No. 15 | NT | 1.7(1.3-2.2) | NT |
| Compound No. 20 | NT | 7.8(5.7-11) | NT |

[a] Numbers are in units of nmoles/rat
[b] Values in parenthesis are 95% confidence intervals
[c] Numbers are in units of mg/kg
[d] Numbers are in units of mg/kg
[e] NT = Not tested by this route

Forebrain Ischemia Assay

Male Mongolian gerbils, 50-70 gm, were used as subjects. Compound No. 1 (30 mg/kg) was injected i.p. 30 minutes prior to carotid occlusion into 6 gerbils. In preparation for surgical procedures, the animals were lightly anesthetized with methoxyflurane and placed upside down on a heated pad with their snout within a nosecone. Nitrous oxide (70%): oxygen (30%) plus 0.5% halothane was circulated through the nosecone to provide continuous anesthesia throughout the surgical procedure. A midline incision was made in the neck and the carotid arteries were exposed. A length of suture thread was placed under each carotid. The thread was then tightened around each carotid and pressure applied to the thread to insure flow was occluded. Flow was occluded for 4-5 minutes and then the thread was removed. The carotids were visually inspected to confirm that reflow had occurred. The wound was then closed with autoclips and the gerbils allowed to recover. Following surgery, the gerbils were kept alive for 7 days. They were anesthetized with 100 mg/kg sodium pentobarbital and perfused transcardially with saline (with heparin) followed by buffered formalin. The brain was removed, trimmed and prepared for histological processing. Sections (10 microns) were stained with thionin. At 7 days following the ischemic insult, damaged neurons have been cleared away by glia and the extent of damage can be ascertained within the vulnerable CA1 region of the hippocampus. The cell loss in CA1 was rated as 0 (no loss), 1 (unilateral damage), 2 (bilateral partial cell loss), or 3 (complete bilateral cell loss). The test animals were compared to a group of 69 saline injected gerbils. The groups were compared by the Mann-Whitney U test [*Elementary Applied Statistics* (New York: Wiley and Sons), 1965]. The cell loss was significantly reduced in the gerbils given Compound No. 1 ($p<0.01$).

Also embraced within this invention is a class of pharmaceutical compositions comprising one or more compounds of Formula I in association with one or more non-toxic, pharmaceutically acceptable carriers and/or diluents and/or adjuvants (collectively referred to herein as "carrier" materials) and, if desired, other active ingredients. The compounds of the present invention may be administered by any suitable route, preferably in the form of a pharmaceutical composition adapted to such a route, and in a dose effective for the treatment intended. Therapeutically effective doses of the compounds of the present invention required to prevent or arrest the progress of the medical condition are readily ascertained by one of ordinary skill in the art. The compounds and composition may, for example, be administered intravascularly, intraperitoneally, subcutaneously, intramuscularly or topically.

For oral administration, the pharmaceutical composition may be in the form of, for example, a tablet, capsule, suspension or liquid. The pharmaceutical composition is preferably made in the form of a dosage unit containing a particular amount of the active ingredient. Examples of such dosage units are tablets or capsules. These may with advantage contain an amount of active ingredient from about 1 to 250 mg, preferably from about 25 to 150 mg. A suitable daily dose for a mammal may vary widely depending on the condition of the patient and other factors. However, a dose of from about 0.1 to 3000 mg/kg body weight, particularly from about 1 to 100 mg/kg body weight, may be appropriate.

The active ingredient may also be administered by injection as a composition wherein, for example, saline, dextrose or water may be used as a suitable carrier. A suitable daily dose is from about 0.1 to 100 mg/kg body weight injected per day in multiple doses depending on the disease being treated. A preferred daily dose would be from about 1 to 30 mg/kg body weight. Compounds indicated for prophylactic therapy will preferably be administered in a daily dose generally in a range from about 0.1 mg to about 100 mg per kilogram of body weight per day. A more preferred dosage will be a range from about 1 mg to about 100 mg per kilogram of body weight. Most preferred is a dosage in a range from about 1 to about 50 mg per kilogram of body weight per day. A suitable dose can be administered, in multiple sub-doses per day. These sub-doses may be administered in unit dosage forms. Typically, a dose or sub-dose may contain from about 1 mg to about 100 mg of active compound per unit dosage form. A more preferred dosage will contain from about 2 mg to about 50 mg of active compound per unit dosage form. Most preferred is a dosage form containing from about 3 mg to about 25 mg of active compound per unit dose.

The dosage regimen for treating a disease condition with the compounds and/or compositions of this invention is selected in accordance with a variety of factors, including the type, age, weight, sex and medical condition of the patient, the severity of the disease, the route of administration, and the particular compound employed, and thus may vary widely.

For therapeutic purposes, the compounds of this invention are ordinarily combined with one or more adjuvants appropriate to the indicated route of administration. If administered per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulfuric acids, gelatin, acacia gum, sodium alginate, polyvinylpyrrolidone, and/or polyvinyl alcohol, and then tableted or encapsulated for convenient administration. Such capsules or tablets may contain a controlled-release formulation as may be provided in a dispersion of active compound in hydroxypropylmethyl cellulose. Formulations for parenteral administration may be in the form of aqueous or non-aqueous isotonic sterile injection solutions or suspensions. These solutions and suspensions may be prepared from sterile powders or granules having one or more of the carriers or diluents mentioned for use in the formulations for oral administration. The compounds may be dissolved in water, polyethylene glycol, propylene glycol, ethanol, corn oil, cottonseed oil, peanut oil, sesame oil, benzyl alcohol, sodium chloride, and/or various buffers. Other adjuvants and modes of administration are well and widely known in the pharmaceutical art.

Although this invention has been described with respect to specific embodiments, the details of these embodiments are not to be construed as limitations. Various equivalents, changes and modifications may be made without departing from the spirit and scope of this invention, and it is understood that such equivalent embodiments are part of this invention.

**Claims**

**Claims for the following Contracting States : AT, BE, FR, GB, DE, LU, NL, IT, SE, CH, LI**

**1.** A compound of the formula

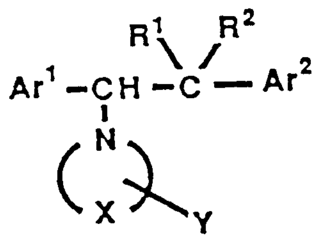

wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together

to form a saturated or partially unsaturated $C_3$-$C_{10}$ cycloalkyl carbocyclic group having three to eight ring carbons;
wherein each of $Ar^1$ and $Ar^2$ is a group independently selected from phenyl, thienyl, furanyl, pyridinyl, thiazolyl and naphthyl groups, and wherein any of the foregoing $Ar^1$ and $Ar^2$ groups may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, $C_2$-$C_{20}$ alkenyl, $C_2$-$C_{20}$ alkynyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein X is selected from CH or $CH_2$ to form a ring having five to seven members provided that such ring is saturated or contains one double bond; and wherein Y is a group selected from hydrido, $C_1$-$C_{20}$ alkyl, halo, hydroxyl, $C_1$-$C_{10}$ alkoxy, amino, cyano, nitro and mercapto;
with the proviso that when each of $Ar^1$ and $Ar^2$ is phenyl and each of $R^1$ and $R^2$ is hydrido or $R^1$ is H and $R^2$ is hydroxy, then X cannot be a linear alkylene chain having four or five carbon atoms so as to form a racemic mixture;
with the further proviso that when $A^1$ is para-methylphenyl or para-methoxyphenyl and each of $R^1$ and $R^2$ is hydrido and $Ar^2$ is phenyl, then X cannot be a linear alkylene chain having five carbon atoms,
except the compounds wherein
$Ar^1$ is phenyl, $R^2$ is H and X is $CH_2$ to form a 6-numbered ring and at the same time
a) $Ar^2$ is phenyl, $R^1$ is F or OH and
b) $Ar^2$ is furanyl and $R^1$ is OH
and wherein
$Ar^1$, $Ar^2$ is 2, 3 or 4 $NO_2$-phenyl, $R^2$ is H, $R^1$ OH and X is $CH_2$ to form a 6-numbered ring;
or wherein $R^1$, $R^2$ is H, X is $CH_2$ to form a 6-numbered ring and $Ar_1$ is naphthyl, $Ar_2$ is phenyl or $Ar_1$ is thienyl and $Ar_2$ is phenyl
and wherein
$Ar^1$ is methoxyphenyl, X is $CH_2$ to form a 5-numbered ring and at the same time
$Ar^2$ is methoxyphenyl, $R^1$ = H, $R^2$ = OH or $R^1$,$R^2$ = H.

**2.** Compound of Claim 1 of the formula

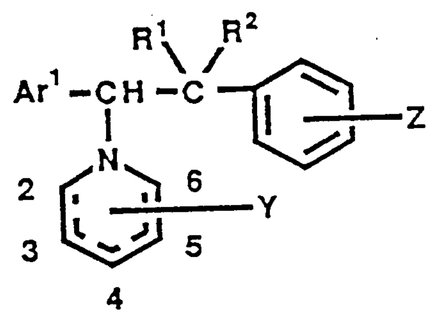

wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated carbocyclic group having three or seven ring carbons; wherein $Ar^1$ is a group selected from phenyl, thienyl, furanyl, pyridinyl, thiazolyl and naphthyl groups, and wherein any of the foregoing $Ar^1$ groups having a substitutable position may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ alkyl, halo, hydroxyl, alkoxy and amino; and wherein Z is one or more groups selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto.

**3.** Compound of Claim 2 wherein each of $R^1$ and $R^2$ is a group independently selected from hydrido, $C_1$-$C_{20}$ linear or branched alkyl, $C_3$-$C_{10}$ cycloalkyl, hydroxyl, halo, $C_1$-$C_{10}$ alkoxy, or wherein $R^1$ and $R^2$ may be taken together to form a saturated or partially unsaturated carbocyclic group having three or seven ring carbons; wherein $Ar^1$ is selected from phenyl, thienyl, pyridinyl and naphthyl groups, and wherein any of the foregoing $Ar^1$ groups having a substitutable position may be substituted with one or

more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; and wherein Z is one or more groups selected from hydrido, $C_1$-$C_{20}$ linear or branched alkyl, $C_3$-$C_{10}$ cycloalkyl, halo, hydroxyl, $C_1$-$C_{10}$ alkoxy and amino.

4. Compound of Claim 3 wherein each of $R^1$ and $R^2$ is a group independently selected from hydrido, $C_1$-$C_{20}$ linear or branched alkyl and hydroxyl, or wherein $R^1$ and $R^2$ may be taken together to form a saturated or partially unsaturated carbocyclic group having three to six ring carbons; wherein $Ar^1$ is a group selected from phenyl, thienyl, pyridinyl and naphthyl groups, and wherein any of the foregoing $Ar^1$ groups having one or more substitutable position may be substituted with one or more radicals selected from hydrido, $C_1$-$C_{20}$ linear or branched alkyl, halo, $C_1$-$C_{20}$ haloalkyl, hydroxyl, $C_1$-$C_{10}$ alkoxy, amino and nitro; wherein Y is a group selected from hydrido, $C_1$-$C_{20}$ linear or branched alkyl, halo, hydroxyl, $C_1$-$C_{10}$ alkoxy and amino; and wherein Z is one or more groups selected from hydrido, $C_1$-$C_{20}$ linear or branched alkyl, halo, hydroxyl and $C_1$-$C_{10}$ alkoxy.

5. Compound of Claim 1 of the formula

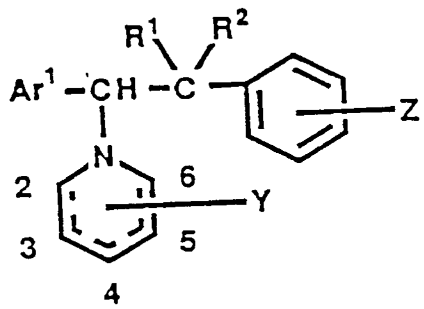

wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated carbocyclic group having three to seven ring carbons; wherein $Ar^1$ is a group selected from phenyl, thienyl, furanyl, pyridinyl, thiazolyl and naphthyl groups, and wherein any of the foregoing $Ar^1$ groups having a substitutable position is substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; wherein Z is one or more groups selected from $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; and wherein the broken line within the N-containing ring represents a double bond between any two adjacent carbon atoms involving the 2-, 3-, 4-, 5- and 6-positions of the N-containing ring.

6. Compound of Claim 5 wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated carbocyclic group having three to seven ring carbons; wherein $Ar^1$ is a group selected from phenyl, thienyl, furanyl, pyridinyl, thiazolyl and naphthyl groups, and wherein any of the foregoing groups having one or more substitutable positions may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; wherein Z is one or more groups selected from hydrido, $C_1$-$C_{20}$ linear or branched alkyl, $C_3$-$C_{10}$ cycloalkyl, halo, hydroxyl, $C_1$-$C_{10}$ alkoxy and amino; and wherein the broken line within the N-containing ring represents a double bond between any two adjacent carbon atoms involving the 3-, 4-, 5- positions of the N-containing ring.

**7.** Compound of Claim 6 wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy; wherein $R^1$ and $R^2$ may be taken together to form a saturated or partially unsaturated cycloalkyl having three to seven ring carbons; wherein $Ar^1$ is selected from phenyl, thienyl, pyridinyl and naphthyl groups, and wherein any of the foregoing groups having a substitutable position is substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; wherein Z is one or more groups selected from hydrido, $C_1$-$C_{20}$ linear or branched alkyl, $C_3$-$C_{10}$ cycloalkyl, halo, hydroxyl, $C_1$-$C_{10}$ alkoxy and amino; and wherein the broken line within the N-containing ring represents either a double bond between any two adjacent carbon atoms involving the 3-, 4-, 5-positions of the N-containing ring.

**8.** Compound of Claim 7 wherein each of $R^1$ and $R^2$ is a group independently selected from hydrido, $C_1$-$C_{20}$ linear or branched alkyl and hydroxyl; wherein $R^1$ and $R^2$ may be taken together to form a saturated or partially unsaturated cycloalkyl having three to six ring carbons; wherein $Ar^1$ is selected from phenyl, thienyl, pyridinyl and naphthyl groups, and wherein any of the foregoing groups having one or more substitutable positions is substituted with one or more radicals selected from hydrido, $C_1$-$C_{20}$ linear or branched alkyl, halo, $C_1$-$C_{20}$ haloalkyl, hydroxyl, $C_1$-$C_{10}$ alkoxy, amino and nitro; wherein Y is a group selected from hydrido, $C_1$-$C_{20}$ linear or branched alkyl, halo, hydroxyl, $C_1$-$C_{10}$ alkoxy and amino; wherein Z is one or more groups selected from hydrido, $C_1$-$C_{20}$ linear or branched alkyl, halo, hydroxyl and $C_1$-$C_{10}$ alkoxy; and wherein the broken line within the N-containing ring represents either a double bond between any two adjacent carbon atoms involving the 3-, 4-, 5-positions of the N-containing ring.

**9.** Compound of Claim 1 selected from the group consisting of
(-)-1-(1,2-diphenylethyl)piperidine;
( + )-1-(1,2-diphenylethyl)piperidine;
(±)-1-[1-phenyl-2-(2-pyridinyl)ethyl]piperidine;
1-[1-phenyl-2-(2-pyridinyl)ethenyl]piperidine;
(±)-1-(1,2-diphenylethyl)-1H-azepine;
(±)-1-(1,2-diphenylbutyl)piperidine;
(±)-1-(1,2-diphenyl-3-methylbutyl)piperidine;
(±)-1-[1-(1-phenylcyclopentyl)benzyl]piperidine;
(±)-1-[1-(1-phenylcyclohexyl)benzyl]piperidine;
(±)-1-[1-(1-phenylcyclopropyl)benzyl]piperidine;
(±)-1-[2,2-dimethyl-2-phenyl-1-(2-thienyl)ethyl]piperidine;
(±)-1-[1-(3-methoxyphenyl)-2-phenylethyl]piperidine;
(±)-1-[1-(4-fluorophenyl)-2-phenylethyl]piperidine;
(±)-1-(2,2-dimethyl-1,2-diphenylethyl)piperidine;
(±)-1-[1-(3-methylphenyl)-2-phenylethyl]piperidine;
(±)-1-[1-(2-furyl)-2-phenylethyl]piperidine;
(±)-1-[1-(2-chlorophenyl)-2-phenylethyl]piperidine;
(±)-1-[1-(2-methoxyphenyl)-2-phenylethyl]piperidine;
(±)-1-(1,2-diphenylethyl)-1,2,3,6-tetrahydropyridine; and
(±)-1-[2-phenyl-1-(2-thienyl)ethyl]-1,2,3,6-tetrahydropyridine.

**10.** Compound of Claim 9 selected from the group consisting of
(-)-1-(1,2-diphenylethyl)piperidine;
( + )-1-(1,2-diphenylethyl)piperidine;
(±)-1-[1-phenyl-2-(2-pyridinyl)ethyl]piperidine;
(±)-1-(1,2-diphenylethyl)-1H-azepine;
(±)-1-(1,2-diphenylbutyl)piperidine;
(±)-1-[1-(1-phenylcyclohexyl)benzyl]piperidine;
(±)-1-[1-(1-phenylcyclopropyl)benzyl)piperidine;
(±)-1-[1-(3-methoxyphenyl)-2-phenylethyl]piperidine;
(±)-1-[1-(4-fluorophenyl)-2-phenylethyl]piperidine,
(±)-1-(2,2-dimethyl-1,2-diphenylethyl)piperidine;

(±)-1-[1-(3-methylphenyl)-2-phenylethyl]piperidine;
(±)-1-[1-(2-chlorophenyl)-2-phenylethyl]piperidine; and
(±)-1-[1-(2-methoxyphenyl)-2-phenylethyl]piperidine.

**11.** Compound of Claim 10 selected from the group consisting of
(±)-1-[1-(3-methoxyphenyl)-2-phenylethyl]piperidine;
(±)-1-[1-(4-fluorophenyl)-2-phenylethyl]piperidine;
(±)-1-[1-(3-methylphenyl)-2-phenylethyl]piperidine;
(±)-1-[1-(2-chlorophenyl)-2-phenylethyl]piperidine; and
(±)-1-[1-(2-methoxyphenyl)-2-phenylethyl]piperidine.

**12.** Compound of Claim 11 which is
(±)-1-[1-(2-chlorphenyl)-2-phenylethyl]piperidine.

**13.** Compound of Caim 9 selected from the group consisting of
(±)-1-(1,2-diphenylethyl)-1,2,3,6-tetrahydropyridine;
and (±)-1-[2-phenyl-1-(2-thienyl)ethyl]-1,2,3,6-tetrahydropyridine.

**14.** Compound of Claim 13 which is
(±)-1-[2-phenyl-1-(2-thienyl)ethyl]-1,2,3,6-tetrahydropyridine.

**15.** A pharmaceutical composition comprising a therapeutically-effective amount of an active compound and a pharmaceutically-acceptable carrier or diluent, said active compound selected from a family of compounds according to Claim 1.

**16.** A pharmaceutical composition comprising a therapeutically-effective amount of an active compound and a pharmaceutically-acceptable carrier or diluent, said active compound being selected from a compound according to Claim 2.

**17.** A pharmaceutical composition comprising a therapeutically-effective amount of an active compound and a pharmaceutically-acceptable carrier or diluent, said active compound being selected from a compound according to Claim 3.

**18.** A pharmaceutical composition comprising a therapeutically-effective amount of an active compound and a pharmaceutically-acceptable carrier or diluent, said active compound being selected from a compound according to Claim 4.

**19.** A pharmaceutical composition comprising a therapeutically-effective amount of an active compound and a pharmaceutically-acceptable carrier or diluent, said active compound being selected from a compound according to Claim 5.

**20.** A pharmaceutical composition comprising a therapeutically-effective amount of an active compound and a pharmaceutically-acceptable carrier or diluent, said active compound being selected from a compound according to Claim 6.

**21.** A pharmaceutical composition comprising a therapeutically-effective amount of an active compound and a pharmaceutically-acceptable carrier or diluent, said active compound being selected from a compound according to Claim 7.

**22.** A pharmaceutical composition comprising a therapeutically-effective amount of an active compound and a pharmaceutically-acceptable carrier or diluent, said active compound being selected from a compound according to Claim 8.

**23.** A pharmaceutical composition comprising a therapeutically-effective amount of an active compound and a pharmaceutically-acceptable carrier or diluent, said active compound being selected from a compound according to Claim 9.

**24.** A pharmaceutical composition comprising a therapeutically-effective amount of an active compound and a pharmaceutically-acceptable carrier or diluent, said active compound being selected from a compound according to Claim 10.

**25.** A pharmaceutical composition comprising a therapeutically-effective amount of an active compound and a pharmaceutically-acceptable carrier or diluent, said active compound being selected from a compound according to Claim 11.

**26.** A pharmaceutical composition comprising a therapeutically-effective amount of an active compound and a pharmaceutically-acceptable carrier or diluent, said active compound being selected from a compound according to Claim 12.

**27.** A pharmaceutical composition comprising a therapeutically-effective amount of an active compound and a pharmaceutically-acceptable carrier or diluent, said active compound being selected from a compound according to Claim 13.

**28.** A pharmaceutical composition comprising a therapeutically-effective amount of an active compound and a pharmaceutically-acceptable carrier or diluent, said active compound being selected from a compound according to Claim 14.

**29.** Use of a compound according to Claim 1 for preparing a medicament to control neuropathogical processses and the neurodegenerative consequences thereof in mammals.

**30.** Use of a compound according to any of Claims 2 to 14 for preparing a medicament for control neuropathological processes and the neurodegenerative consequences thereof in mammals.

**31.** Use according to Claim 29 for preparing a medicament for treating hypoxia, anoxia or ischemia.

**32.** Use according to Claim 31 for preparing a medicament for treating ischemia.

**33.** Use of a compound as defined in claims 29-32, selected from the group consisting of
(±)-1-(1,2-diphenylethyl)piperidine;
(-)-1-(1,2-diphenylethyl)piperidine;
(+)-1-(1,2-diphenylethyl)piperidine;
(±)-1-[2-phenyl-1-(2-thienyl)ethyl]piperidine hydrosulfate;
(±)-1-(1,2-diphenylethyl)-1,2,3,6-tetrahydropyridine;
1-(1,2-diphenylethenyl)piperidine;
1-[1-phenyl-2-(2-pyridinyl)ethenyl]piperidine;
(±)-1-[1-phenyl-2-(2-pyridinyl)ethyl]piperidine;
(±)-1-(1,2-diphenylethyl)-1H-azepine;
(±)-1-(1,2-diphenylbutyl)piperidine;
(±)-1-(1,2-diphenyl-3-methylbutyl)piperidine;
(±)-1-(1,2-diphenyl-1-hydroxyethyl)piperidine;
(±)-1-[2-phenyl-1-(2-thienyl)ethyl]-1,2,3,6-tetrahydropyridine;
(±)-1-[1-(1-phenylcyclopentyl)benzyl]piperidine;
(±)-1-[1-(1-phenylcyclohexyl)benzyl]piperidine;
(±)-1-(1,2-diphenylethyl)pyrrolidine;
(±)-1-[1-(4-methylphenyl)-2-phenylethyl]piperidine;
(±)-1-[1-(4-methoxyphenyl)-2-phenylethyl]piperidine;
(±)-1-[1-(1-phenylcyclopropyl)benzyl]piperidine;
(±)-1-[2,2-dimethyl-2-phenyl-1-(2-thienyl)ethyl]piperidine;
(±)-1-[1-(3-methoxyphenyl)-2-phenylethyl]piperidine;
(±)-1-[1-(4-fluorophenyl)-2-phenylethyl]piperidine;
(±)-1-(2,2-dimethyl-1,2-diphenylethyl)piperidine;
(±)-1-[1-(3-methylphenyl)-2-phenylethyl]piperidine;
(±)-1-[1-(2-furyl)-2-phenylethyl]piperidine;
(±)-1-[1-(2-chlorophenyl)-2-phenylethyl]piperidine;
(±)-1-[1-(2-methoxyphenyl)-2-phenylethyl]piperidine; and

(±)-1-[1-(1-naphthyl)-2-phenylethyl]piperidine.

**Claims for the following Contracting State : ES**

**1.** A process for preparing a compound of the formula

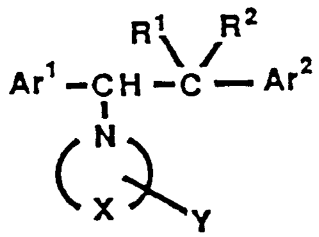

wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated $C_3$-$C_{10}$ cycloalkyl carbocyclic group having three to eight ring carbons;

wherein each of $Ar^1$ and $Ar^2$ is a group independently selected from phenyl, thienyl, furanyl, pyridinyl, thiazolyl and naphthyl groups, and wherein any of the foregoing $Ar^1$ and $Ar^2$ groups may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, $C_2$-$C_{20}$ alkenyl, $C_2$-$C_{20}$ alkynyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein X is selected from CH or $CH_2$ to form a ring having five to seven members provided that such ring is saturated or contains one double bond; and wherein Y is a group selected from hydrido, $C_1$-$C_{20}$ alkyl, halo, hydroxyl, $C_1$-$C_{10}$ alkoxy, amino, cyano, nitro and mercapto;

with the proviso that when each of $Ar^1$ and $Ar^2$ is phenyl and each of $R^1$ and $R^2$ is hydrido or $R^1$ is H and $R^2$ is hydroxy, then X cannot be a linear alkylene chain having four or five carbon atoms so as to form a racemic mixture;

with the further proviso that when $A^1$ is para-methylphenyl or para-methoxyphenyl and each of $R^1$ and $R^2$ is hydrido and $Ar^2$ is phenyl, then X cannot be a linear alkylene chain having five carbon atoms,

except the compounds wherein

$Ar^1$ is phenyl, $R^2$ is H and X is $CH_2$ to form a 6-numbered ring and at the same time

a) $Ar^2$ is phenyl, $R^1$ is F or OH

b) $Ar^2$ is furanyl and $R^1$ is OH

and wherein

$Ar^1$, $Ar^2$ is 2, 3 or 4 $NO_2$-phenyl, $R^2$ is H, $R^1$ OH and X is $CH_2$ to form a 6-numbered ring;

or wherein $R^1$, $R^2$ is H, X is $CH_2$ to form a 6-numbered ring and $Ar_1$ is naphthyl, $Ar_2$ is phenyl or $Ar_1$ is thienyl and $Ar_2$ is phenyl

and wherein

$Ar^1$ is methoxyphenyl, X is $CH_2$ to form a 5-numbered ring and at the same time

$Ar^2$ is methoxyphenyl, $R^1$ = H, $R^2$ = OH or $R^1$,$R^2$ = H,

characterized in that

A. an amine of the formula (2)

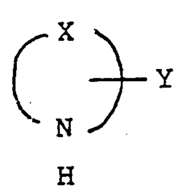

(2)

wherein Y and X are as defined before, is reacted in the presence of a Bronsted or Lewis acid in an appropriate solvent with a ketone of the formula (1)

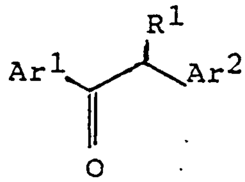

(1)

with $Ar^1$, $Ar^2$, $R^1$ as defined before,
said reaction (A) generating the imine (3) of the formula

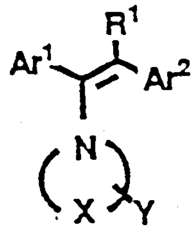

(3)

with all the substituents as defined before, said imine (3) subsequently being transformed by preduction with an appropriate reducing agent in a solvent, yielding the desired compound of the formula

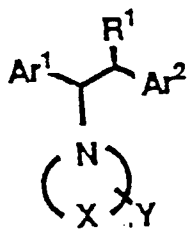

or
B) by reacting an amine of the formula (2)

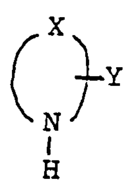

(2)

with X and Y as defined before,
either neat or in a solvent with a compound of the formula (9)

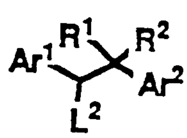

with $Ar^1$, $Ar^2$, $R^1$, $R^2$ as defined before and $L^2$ being an appropriate leaving group,
whereby said compound of the formula (9) being generated from the corresponding ketone,

thus yielding the desired compounds
or
C) by reacting a compound of the formula (12)

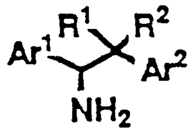

with all the substituents as defined before,
and generated from the corresponding ketone, or from the reaction of the nitrile (1) $AR^2CN$ with a compound of the formula

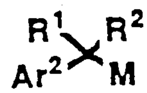

with the substituents as defined,
with a compound of the formula (13)

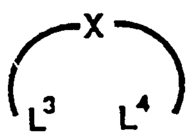

(13)

wherein X is as defined before and $L^3$ and $L^4$ being good leaving groups,
yielding the desired compounds.

**2.** A pocess according to Claim 1 for preparing a compound of the formula

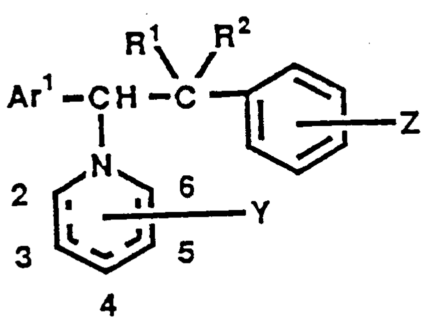

wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated carbocyclic group having three or seven ring carbons; wherein $Ar^1$ is a group selected from phenyl, thienyl, furanyl, pyridinyl, thiazolyl and naphthyl groups, and wherein any of the foregoing $Ar^1$ groups having a substitutable position may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ alkyl, halo, hydroxyl, alkoxy and amino; and wherein Z is one or more groups selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto.

3. A process according to Claim 2 wherein each of $R^1$ and $R^2$ is a group independently selected from hydrido, $C_1$-$C_{20}$ linear or branched alkyl, $C_3$-$C_{10}$ cycloalkyl, hydroxyl, halo, $C_1$-$C_{10}$ alkoxy, or wherein $R^1$ and $R^2$ may be taken together to form a saturated or partially unsaturated carbocyclic group having three or seven ring carbons; wherein $Ar^1$ is selected from phenyl, thienyl, pyridinyl and naphthyl groups, and wherein any of the foregoing $Ar^1$ groups having a substitutable position may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; and wherein Z is one or more groups selected from hydrido, $C_1$-$C_{20}$ linear or branched alkyl, $C_3$-$C_{10}$ cycloalkyl, halo, hydroxyl, $C_1$-$C_{10}$ alkoxy and amino.

4. A process according to Claim 3 wherein each of $R^1$ and $R^2$ is a group independently selected from hydrido, $C_1$-$C_{20}$ linear or branched alkyl and hydroxyl, or wherein $R^1$ and $R^2$ may be taken together to form a saturated or partially unsaturated carbocyclic group having three to six ring carbons; wherein $Ar^1$ is a group selected from phenyl, thienyl, pyridinyl and naphthyl groups, and wherein any of the foregoing $Ar^1$ groups having one or more substitutable position may be substituted with one or more radicals selected from hydrido, $C_1$-$C_{20}$ linear or branched alkyl, halo, $C_1$-$C_{20}$ haloalkyl, hydroxyl, $C_1$-$C_{10}$ alkoxy, amino and nitro; wherein Y is a group selected from hydrido, $C_1$-$C_{20}$ linear or branched alkyl, halo, hydroxyl, $C_1$-$C_{10}$ alkoxy and amino; and wherein Z is one or more groups selected from hydrido, $C_1$-$C_{20}$ linear or branched alkyl, halo, hydroxyl and $C_1$-$C_{10}$ alkoxy.

5. A process according to Claim 1 for preparing a compound of the formula

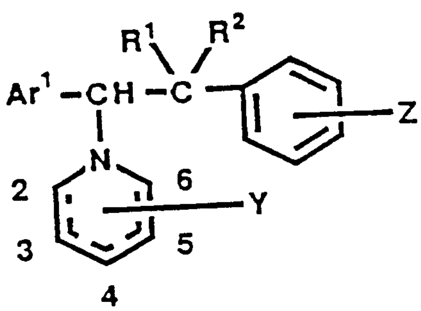

wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated carbocyclic group having three to seven ring carbons; wherein $Ar^1$ is a group selected from phenyl, thienyl, furanyl, pyridinyl, thiazolyl and naphthyl groups, and wherein any of the foregoing $Ar^1$ groups having a substitutable position is substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; wherein Z is one or more groups selected from $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; and wherein the broken line within the N-containing ring represents a double bond between any two adjacent carbon atoms involving the 2-, 3-, 4-, 5- and 6-positions of the N-containing ring.

6. A process according to Claim 5 wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated carbocyclic group having three to seven ring carbons; wherein $Ar^1$ is a group selected from phenyl, thienyl, furanyl, pyridinyl, thiazolyl and naphthyl groups, and wherein any of the foregoing groups having one or more substitutable positions may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; wherein Z is one or

more groups selected from hydrido, $C_1$-$C_{20}$ linear or branched alkyl, $C_3$-$C_{10}$ cycloalkyl, halo, hydroxyl, $C_1$-$C_{10}$ alkoxy and amino; and wherein the broken line within the N-containing ring represents a double bond between any two adjacent carbon atoms involving the 3-, 4-, 5- positions of the N-containing ring.

**7.** A process according to Claim 6 wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy; wherein $R^1$ and $R^2$ may be taken together to form a saturated or partially unsaturated cycloalkyl having three to seven ring carbons; wherein $Ar^1$ is selected from phenyl, thienyl, pyridinyl and naphthyl groups, and wherein any of the foregoing groups having a substitutable position is substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; wherein Z is one or more groups selected from hydrido, $C_1$-$C_{20}$ linear or branched alkyl, $C_3$-$C_{10}$ cycloalkyl, halo, hydroxyl, $C_1$-$C_{10}$ alkoxy and amino; and wherein the broken line within the N-containing ring represents either a double bond between any two adjacent carbon atoms involving the 3-, 4-, 5-positions of the N-containing ring.

**8.** A process according to Claim 7 wherein each of $R^1$ and $R^2$ is a group independently selected from hydrido, $C_1$-$C_{20}$ linear or branched alkyl and hydroxyl; wherein $R^1$ and $R^2$ may be taken together to form a saturated or partially unsaturated cycloalkyl having three to six ring carbons; wherein $Ar^1$ is selected from phenyl, thienyl, pyridinyl and naphthyl groups, and wherein any of the foregoing groups having one or more substitutable positions is substituted with one or more radicals selected from hydrido, $C_1$-$C_{20}$ linear or branched alkyl, halo, $C_1$-$C_{20}$ haloalkyl, hydroxyl, $C_1$-$C_{10}$ alkoxy, amino and nitro; wherein Y is a group selected from hydrido, $C_1$-$C_{20}$ linear or branched alkyl, halo, hydroxyl, $C_1$-$C_{10}$ alkoxy and amino; wherein Z is one or more groups selected from hydrido, $C_1$-$C_{20}$ linear or branched alkyl, halo, hydroxyl and $C_1$-$C_{10}$ alkoxy; and wherein the broken line within the N-containing ring represents either a double bond between any two adjacent carbon atoms involving the 3-, 4-, 5-positions of the N-containing ring.

**9.** A process according to Claim 1 for preparing a compound selected from the group consisting of
(-)-1-(1,2-diphenylethyl)piperidine;
(+)-1-(1,2-diphenylethyl)piperidine;
(±)-1-[1-phenyl-2-(2-pyridinyl)ethyl]piperidine;
1-[1-phenyl-2-(2-pyridinyl)ethenyl]piperidine;
(±)-1-(1,2-diphenylethyl)-1H-azepine;
(±)-1-(1,2-diphenylbutyl)piperidine;
(±)-1-(1,2-diphenyl-3-methylbutyl)piperidine;
(±)-1-[1-(1-phenylcyclopentyl)benzyl]piperidine;
(±)-1-[1-(1-phenylcyclohexyl)benzyl)piperidine;
(±)-1-[1-(1-phenylcyclopropyl)benzyl)piperidine;
(±)-1-[2,2-dimethyl-2-phenyl-1-(2-thienyl)ethyl]piperidine;
(±)-1-[1-(3-methoxyphenyl)-2-phenylethyl]piperidine;
(±)-1-[1-(4-fluorophenyl)-2-phenylethyl]piperidine;
(±)-1-(2,2-dimethyl-1,2-diphenylethyl)piperidine;
(±)-1-[1-(3-methylphenyl)-2-phenylethyl]piperidine;
(±)-1-[1-(2-furyl)-2-phenylethyl]piperidine;
(±)-1-[1-(2-chlorophenyl)-2-phenylethyl]piperidine;
(±)-1-[1(2-methoxyphenyl)-2-phenylethyl]piperidine;
(±)-1-(1,2-diphenylethyl)-1,2,3,6-tetrahydropyridine; and
(±)-1-[2-phenyl-1-(2-thienyl)ethyl]-1,2,3,6-tetrahydropyridine.

**10.** A process according to Claim 9 for preparing a compound selected from the group consisting of
(-)-1-(1,2-diphenylethyl)piperidine;
(+)-1-(1,2-diphenylethyl)piperidine;
(±)-1-[1-phenyl-2-(2-pyridinyl)ethyl]piperidine;
(±)-1-(1,2-diphenylethyl)-1H-azepine;
(±)-1-(1,2-diphenylbutyl)piperidine;
(±)-1-[1-(1-phenylcyclohexyl)benzyl]piperidine;

(±)-1-[1-(1-phenylcyclopropyl)benzyl]piperidine;
(±)-1-[1-(3-methoxyphenyl)-2-phenylethyl]piperidine;
(±)-1-[1-(4-fluorophenyl)-2-phenylethyl]piperidine;
(±)-1-(2,2-dimethyl-1,2-diphenylethyl)piperidine;
(±)-1-[1-(3-methylphenyl)-2-phenylethyl]piperidine;
(±)-1-[1-(2-chlorophenyl)-2-phenylethyl]piperidine; and
(±)-1-[1-(2-methoxyphenyl)-2-phenylethyl]piperidine.

**11.** A process according to Claim 10 for preparing a compound selected from the group consisting of
(±)-1-[1-(3-methoxyphenyl)-2-phenylethyl]piperidine;
(±)-1-[1-(4-fluorophenyl)-2-phenylethyl]piperidine;
(±)-1-[1(3-methylphenyl)-2-phenylethyl]piperidine;
(±)-1-[1-(2-chlorophenyl)-2-phenylethyl]piperidine; and
(±)-1-[1-(2-methoxyphenyl)-2-phenylethyl]piperidine.

**12.** A process according to Claim 11 wherein the compound prepared is (±)-1-[1-(2-chlorphenyl)-2-phenylethyl]piperidine.

**13.** A process according to Caim 9 for preparing a compound selected from the group consisting of
(±)-1-(1,2-diphenylethyl)-1,2,3,6-tetrahydropyridine;
and (±)-1-[2-phenyl-1-(2-thienyl)ethyl]-1,2,3,6-tetrahydropyridine.

**14.** A process according to Claim 13 for preparing (±)-1-[2-phenyl-1-(2-thienyl)ethyl]-1,2,3,6-tetrahydropyridine.

**15.** A process for preparing a pharmaceutical composition by admixing a pharmaceutically-acceptable carrier or diluent and a therapeutically-effective amount of an active compound being selected from a family of compounds as prepared according to Claim 1.

**16.** A process for preparing a pharmaceutical composition by admixing a pharmaceutically-acceptable carrier or diluent and a therapeutically-effective amount of an active compound being selected from a family of compounds as prepared according to Claim 2.

**17.** A process for preparing a pharmaceutical composition by admixing a pharmaceutically-acceptable carrier or diluent and a therapeutically-effective amount of an active compound being selected from a family of compounds as prepared according to Claim 3.

**18.** A process for preparing a pharmaceutical composition by admixing a pharmaceutically-acceptable carrier or diluent and a therapeutically-effective amount of an active compound being selected from a family of compounds as prepared according to Claim 4.

**19.** A process for preparing a pharmaceutical composition by admixing a pharmaceutically-acceptable carrier or diluent and a therapeutically-effective amount of an active compound being selected from a family of compounds as prepared according to Claim 5.

**20.** A process for preparing a pharmaceutical composition by admixing a pharmaceutically-acceptable carrier or diluent and a therapeutically-effective amount of an active compound being selected from a family of compounds as prepared according to Claim 6.

**21.** A process for preparing a pharmaceutical composition by admixing a pharmaceutically-acceptable carrier or diluent and a therapeutically-effective amount of an active compound being selected from a family of compounds as prepared according to Claim 7.

**22.** A process for preparing a pharmaceutical composition by admixing a pharmaceutically-acceptable carrier or diluent and a therapeutically-effective amount of an active compound being selected from a family of compounds as prepared according to Claim 8.

**23.** A process for preparing a pharmaceutical composition by admixing a pharmaceutically-acceptable carrier or diluent and a therapeutically-effective amount of an active compound being selected from a family of compounds as prepared according to Claim 9.

**24.** A process for preparing a pharmaceutical composition by admixing a pharmaceutically-acceptable carrier or diluent and a therapeutically-effective amount of an active compound being selected from a family of compounds as prepared according to Claim 10.

**25.** A process for preparing a pharmaceutical composition by admixing a pharmaceutically-acceptable carrier or diluent and a therapeutically-effective amount of an active compound being selected from a family of compounds as prepared according to Claim 11.

**26.** A process for preparing a pharmaceutical composition by admixing a pharmaceutically-acceptable carrier or diluent and a therapeutically-effective amount of an active compound being selected from a family of compounds as prepared according to Claim 12.

**27.** A process for preparing a pharmaceutical composition by admixing a pharmaceutically-acceptable carrier or diluent and a therapeutically-effective amount of an active compound being selected from a family of compounds as prepared according to Claim 13.

**28.** A process for preparing a pharmaceutical composition by admixing a pharmaceutically-acceptable carrier or diluent and a therapeutically-effective amount of an active compound being selected from a family of compounds as prepared according to Claim 14.

**29.** Use of a compound according to Claim 1 for preparing a medicament to control neuropathogical processses and the neurodegenerative consequences thereof in mammals.

**30.** Use of a compound according to any of Claims 2 to 14 for preparing a medicament for control neuropathological processes and the neurodegenerative consequences thereof in mammals.

**31.** Use according to Claim 29 for preparing a medicament for treating hypoxia, anoxia or ischemia.

**32.** Use according to Claim 31 for preparing a medicament for treating ischemia.

**33.** Use of a compound as defined in claims 29-32, selected from the group consisting of
(±)-1-(1,2-diphenylethyl)piperidine;
(-)-1-(1,2-diphenylethyl)piperidine;
(+)-1-(1,2-diphenylethyl)piperidine;
(±)-1-[2-pheny1-1-(2-thienyl)ethyl]piperidine hydrosulfate;
(±)-1-(1,2-diphenylethyl)-1,2,3,6-tetrahydropyridine;
1-(1,2-diphenylethenyl)piperidine;
1-[1-phenyl-2-(2-pyridinyl)ethenyl]piperidine;
(±)-1-[1-phenyl-2-(2-pyridinyl)ethyl]piperidine;
(±)-1-(1,2-diphenylethyl)-1H-azepine;
(±)-1-(1,2-diphenylbutyl)piperidine;
(±)-1-(1,2-diphenyl-3-methylbutyl)piperidine;
(±)-1-(1,2-diphenyl-1-hydroxyethyl)piperidine;
(±)-1-[2-phenyl-1-(2-thienyl)ethyl]-1,2,3,6-tetrahydropyridine;
(±)-1-[1-(1-phenylcyclopentyl)benzyl]piperidine;
(±)-1-[1-(1-phenylcyclohexyl)benzyl]piperidine;
(±)-1-(1,2-diphenylethyl)pyrrolidine;
(±)-1-[1-(4-methylphenyl)-2-phenylethyl]piperidine;
(±)-1-[1-(4-methoxyphenyl)-2-phenylethyl]piperidine;
(±)-1-[1-(1-phenylcyclopropyl)benzyl]piperidine;
(±)-1-[2,2-dimethyl-2-phenyl-1-(2-thienyl)ethyl]piperidine;
(±)-1-[1-(3-methoxyphenyl)-2-phenylethyl]piperidine;
(±)-1-[1-(4-fluorophenyl)-2-phenylethyl]piperidine;
(±)-1-(2,2-dimethyl-1,2-diphenylethyl)piperidine;

(±)-1-[1-(3-methylphenyl)-2-phenylethyl]piperidine;
(±)-1-[1-(2-furyl)-2-phenylethyl]piperidine;
(±)-1-[1-(2-chlorophenyl)-2-phenylethyl]piperidine;
(±)-1-[1-(2-methoxyphenyl)-2-phenylethyl]piperidine; and
(±)-1-[1-(1-naphthyl)-2-phenylethyl]piperidine.

**Claims for the following Contracting State : GR**

1. A compound of the formula

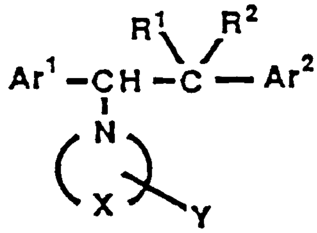

wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated $C_3$-$C_{10}$ cycloalkyl carbocyclic group having three to eight ring carbons;
wherein each of $Ar^1$ and $Ar^2$ is a group independently selected from phenyl, thienyl, furanyl, pyridinyl, thiazolyl and naphthyl groups, and wherein any of the foregoing $Ar^1$ and $Ar^2$ groups may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, $C_2$-$C_{20}$ alkenyl, $C_2$-$C_{20}$ alkynyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein X is selected from CH or $CH_2$ to form a ring having five to seven members provided that such ring is saturated or contains one double bond; and wherein Y is a group selected from hydrido, $C_1$-$C_{20}$ alkyl, halo, hydroxyl, $C_1$-$C_{10}$ alkoxy, amino, cyano, nitro and mercapto;
with the proviso that when each of $Ar^1$ and $Ar^2$ is phenyl and each of $R^1$ and $R^2$ is hydrido or $R^1$ is H and $R^2$ is hydroxy, then X cannot be a linear alkylene chain having four or five carbon atoms so as to form a racemic mixture;
with the further proviso that when $A^1$ is para-methylphenyl or para-methoxyphenyl and each of $R^1$ and $R^2$ is hydrido and $Ar^2$ is phenyl, then X cannot be a linear alkylene chain having five carbon atoms,
except the compounds wherein
$Ar^1$ is phenyl, $R^2$ is H and X is $CH_2$ to form a 6-numbered ring and at the same time
a) $Ar^2$ is phenyl, $R^1$ is F or OH
b) $Ar^2$ is furanyl and $R^1$ is OH
and wherein
$Ar^1$, $Ar^2$ is 2, 3 or 4 $NO_2$-phenyl, $R^2$ is H, $R^1$ OH and X is $CH_2$ to form a 6-numbered ring;
or wherein $R^1$, $R^2$ is H, X is $CH_2$ to form a 6-numbered ring and $Ar_1$ is naphthyl, $Ar_2$ is phenyl or $Ar_1$ is thienyl and $Ar_2$ is phenyl
and wherein
$Ar^1$ is methoxyphenyl, X is $CH_2$ to form a 5-numbered ring and at the same time
$Ar^2$ is methoxyphenyl, $R^1$ = H, $R^2$ = OH or $R^1$,$R^2$ = H

**2.** Compound of Claim 1 of the formula

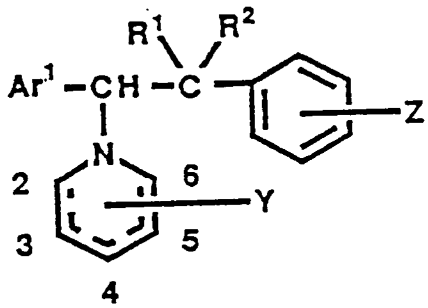

wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated carbocyclic group having three or seven ring carbons; wherein $Ar^1$ is a group selected from phenyl, thienyl, furanyl, pyridinyl, thiazolyl and naphthyl groups, and wherein any of the foregoing $Ar^1$ groups having a substitutable position may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ alkyl, halo, hydroxyl, alkoxy and amino; and wherein Z is one or more groups selected from hydrido, $C_1$-$C_{20}$ linear or branched alkyl, $C_3$-$c_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto.

**3.** Compound of Claim 2 wherein each of $R^1$ and $R^2$ is a group independently selected from hydrido, $C_1$-$C_{20}$ linear or branched alkyl, $C_3$-$C_{10}$ cycloalkyl, hydroxyl, halo, $C_1$-$C_{10}$ alkoxy, or wherein $R^1$ and $R^2$ may be taken together to form a saturated or partially unsaturated carbocyclic group having three or seven ring carbons; wherein $Ar^1$ is selected from phenyl, thienyl, pyridinyl and naphthyl groups, and wherein any of the foregoing $Ar^1$ groups having a substitutable position may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; and wherein Z is one or more groups selected from hydrido, $C_1$-$C_{20}$ linear or branched alkyl, $C_3$-$C_{10}$ cycloalkyl, halo, hydroxyl, $C_1$-$C_{10}$ alkoxy and amino.

**4.** Compound of Claim 3 wherein each of $R^1$ and $R^2$ is a group independently selected from hydrido, $C_1$-$C_{20}$ linear or branched alkyl and hydroxyl, or wherein $R^1$ and $R^2$ may be taken together to form a saturated or partially unsaturated carbocyclic group having three to six ring carbons; wherein $Ar^1$ is a group selected from phenyl, thienyl, pyridinyl and naphthyl groups, and wherein any of the foregoing $Ar^1$ groups having one or more substitutable position may be substituted with one or more radicals selected from hydrido, $C_1$-$C_{20}$ linear or branched alkyl, halo, $C_1$-$C_{20}$ haloalkyl, hydroxyl, $C_1$-$C_{10}$ alkoxy, amino and nitro; wherein Y is a group selected from hydrido, $C_1$-$C_{20}$ linear or branched alkyl, halo, hydroxyl, $C_1$-$C_{10}$ alkoxy and amino; and wherein Z is one or more groups selected from hydrido, $C_1$-$C_{20}$ linear or branched alkyl, halo, hydroxyl and $C_1$-$C_{10}$ alkoxy.

**5.** Compound of Claim 1 of the formula

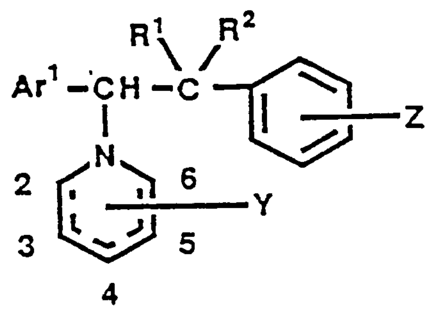

wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated carbocyclic group having three to seven ring carbons; wherein $Ar^1$ is a group selected from phenyl, thienyl, furanyl, pyridinyl, thiazolyl and naphthyl groups, and wherein any of the foregoing $Ar^1$ groups having a substitutable position is substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; wherein Z is one or more groups selected from $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; and wherein the broken line within the N-containing ring represents a double bond between any two adjacent carbon atoms involving the 2-, 3-, 4-, 5- and 6-positions of the N-containing ring.

**6.** Compound of Claim 5 wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy, or wherein $R^1$ and $R^2$ may be taken together to form a saturated or partially unsaturated carbocyclic group having three to seven ring carbons; wherein $Ar^1$ is a group selected from phenyl, thienyl, furanyl, pyridinyl, thiazolyl and naphthyl groups, and wherein any of the foregoing groups having one or more substitutable positions may be substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; wherein Z is one or more groups selected from hydrido, $C_1$-$C_{20}$ linear or branched alkyl, $C_3$-$C_{10}$ cycloalkyl, halo, hydroxyl, $C_1$-$C_{10}$ alkoxy and amino; and wherein the broken line within the N-containing ring represents a double bond between any two adjacent carbon atoms involving the 3-, 4-, 5- positions of the N-containing ring.

**7.** Compound of Claim 6 wherein each of $R^1$ and $R^2$ is a group independently selected from hydrido, $C_1$-$C_{20}$ linear or branched alkyl, $C_3$-$C_{10}$ cycloalkyl, hydroxyl, halo and $C_1$-$C_{10}$ alkoxy; wherein $R^1$ and $R^2$ may be taken together to form a saturated or partially unsaturated cycloalkyl having three to seven ring carbons; wherein $Ar^1$ is selected from phenyl, thienyl, pyridinyl and naphthyl groups, and wherein any of the foregoing groups having a substitutable position is substituted with one or more radicals selected from hydrido, $C_1$-$C_{20}$ linear or branched alkyl, $C_3$-$C_{10}$ cycloalkyl, halo, $C_1$-$C_{20}$ haloalkyl, hydroxyl, $C_1$-$C_{10}$ hydroxyalkyl, $C_1$-$C_{10}$ alkoxy, $C_1$-$C_{20}$ alkoxyalkyl, amino, cyano, nitro and mercapto; wherein Y is a group selected from hydrido, $C_1$-$C_{20}$ linear or branched alkyl, halo, hydroxyl, $C_1$-$C_{10}$ alkoxy and amino; wherein Z is one or more groups selected from hydrido, $C_1$-$C_{20}$ linear or branched alkyl, $C_3$-$C_{10}$ cycloalkyl, halo, hydroxyl, $C_1$-$C_{10}$ alkoxy and amino; and wherein the broken line within the N-containing ring represents either a double bond between any two adjacent carbon atoms involving the 3-, 4-, 5-positions of the N-containing ring.

**8.** Compound of Claim 7 wherein each of $R^1$ and $R^2$ is a group independently selected from hydrido, $C_1$-$C_{20}$ linear or branched alkyl and hydroxyl; wherein $R^1$ and $R^2$ may be taken together to form a saturated or partially unsaturated cycloalkyl having three to six ring carbons; wherein $Ar^1$ is selected

from phenyl, thienyl, pyridinyl and naphthyl groups, and wherein any of the foregoing groups having one or more substitutable positions is substituted with one or more radicals selected from hydrido, $C_1$-$C_{20}$ linear or branched alkyl, halo, $C_1$-$C_{20}$ haloalkyl, hydroxyl, $C_1$-$C_{10}$ alkoxy, amino and nitro; wherein Y is a group selected from hydrido, $C_1$-$C_{20}$ linear or branched alkyl, halo, hydroxyl, $C_1$-$C_{10}$ alkoxy and amino; wherein Z is one or more groups selected from hydrido, $C_1$-$C_{20}$ linear or branched alkyl, halo, hydroxyl and $C_1$-$C_{10}$ alkoxy; and wherein the broken line within the N-containing ring represents either a double bond between any two adjacent carbon atoms involving the 3-, 4-, 5-positions of the N-containing ring.

**9.** Compound of Claim 1 selected from the group consisting of
(-)-1-(1,2-diphenylethyl)piperidine;
(+)-1-(1,2-diphenylethyl)piperidine;
(±)-1-[1-phenyl-2-(2-pyridinyl)ethyl]piperidine;
1-[1-phenyl-2-(2-pyridinyl)ethenyl]piperidine;
(±)-1-(1,2-diphenylethyl)-1H-azepine;
(±)-1-(1,2-diphenylbutyl)piperidine;
(±)-1-(1,2-diphenyl-3-methylbutyl)piperidine;
(±)-1-[1-(1-phenylcyclopentyl)benzyl]piperidine;
(±)-1-[1-(1-phenylcyclohexyl)benzyl]piperidine;
(±)-1-[1-(1-phenylcyclopropyl)benzyl]piperidine;
(±)-1-[2,2-dlmethyl-2-phenyl-1-(2-thienyl)ethyl]piperidine;
(±)-1-[1-(3-methoxyphenyl)-2-phenylethyl]piperidine;
(±)-1-[1-(4-fluorophenyl)-2-phenylethyl]piperidine;
(±)-1-(2,2-dimethyl-1,2-diphenylethyl)piperidine;
(±)-1-[1-(3-methylphenyl)-2-phenylethyl]piperidine;
(±)-1-[1-(2-furyl)-2-phenylethyl]piperidine;
(±)-1-[1-(2-chlorophenyl)-2-phenylethyl]piperidine;
(±)-1-[1-(2-methoxyphenyl)-2-phenylethyl]piperidine;
(±)-1-(1,2-diphenylethyl)-1,2,3,6-tetrahydropyridine; and
(±)-1-[2-phenyl-1-(2-thienyl)ethyl]-1,2,3,6-tetrahydropyridine.

**10.** Compound of Claim 9 selected from the group consisting of
(-)-1-(1,2-diphenylethyl)piperidine;
(+)-1-(1,2-diphenylethyl)piperidine;
(±)-1-[1-phenyl-2-(2-pyridinyl)ethyl]piperidine;
(±)-1-(1,2-diphenylethyl)-1H-azepine;
(±)-1-(1,2-diphenylbutyl)piperidine;
(±)-1-[1-(1-phenylcyclohexyl)benzyl]piperidine;
(±)-1-[1-(1-phenylcyclopropyl)benzyl]piperidine;
(±)-1-[1-(3-methoxyphenyl)-2-phenylethyl]piperidine;
(±)-1-[1-(4-fluorophenyl)-2-phenylethyl]piperidine;
(±)-1-(2,2-dimethyl-1,2-diphenylethyl)piperidine;
(±)-1-[1-(3-methylphenyl)-2-phenylethyl]piperidine;
(±)-1-[1-(2-chlorophenyl)-2-phenylethyl]piperidine; and
(±)-1-[1-(2-methoxyphenyl)-2-phenylethyl]piperidine.

**11.** Compound of Claim 10 selected from the group consisting of
(±)-1-[1-(3-methoxyphenyl)-2-phenylethyl]piperidine;
(±)-1-[1-(4-fluorophenyl)-2-phenylethyl]piperidine;
(±)-1-[1-(3-methylphenyl)-2-phenylethyl]piperidine;
(±)-1-[1-(2-chlorophenyl)-2-phenylethyl]piperidine; and
(±)-1-[1-(2-methoxyphenyl)-2-phenylethyl]piperidine.

**12.** Compound of Claim 11 which is
(±)-1-[1-(2-chlorphenyl)-2-phenylethyl]piperidine.

**13.** Compound of Caim 9 selected from the group consisting of
(±)-1-(1,2-diphenylethyl)-1,2,3,6-tetrahydropyridine;

and (±)-1-[2-phenyl-1-(2-thienyl)ethyl]-1,2,3,6-tetrahydropyridine.

**14.** Compound of Claim 13 which is (±)-1-[2-phenyl-1-(2-thienyl)ethyl]-1,2,3,6-tetrahydropyridine.

**15.** A process for preparing a pharmaceutical composition by admixing a pharmaceutically-acceptable carrier or diluent and a therapeutically-effective amount of an active compound being selected from a family of compounds according to Claim 1.

**16.** A process for preparing a pharmaceutical composition by admixing a pharmaceutically-acceptable carrier or diluent and a therapeutically-effective amount of an active compound being selected from a family of compounds according to Claim 2.

**17.** A process for preparing a pharmaceutical composition by admixing a pharmaceutically-acceptable carrier or diluent and a therapeutically-effective amount of an active compound being selected from a family of compounds according to Claim 3.

**18.** A process for preparing a pharmaceutical composition by admixing a pharmaceutically-acceptable carrier or diluent and a therapeutically-effective amount of an active compound being selected from a family of compounds according to Claim 4.

**19.** A process for preparing a pharmaceutical composition by admixing a pharmaceutically-acceptable carrier or diluent and a therapeutically-effective amount of an active compound being selected from a family of compounds according to Claim 5.

**20.** A process for preparing a pharmaceutical composition by admixing a pharmaceutically-acceptable carrier or diluent and a therapeutically-effective amount of an active compound being selected from a family of compounds according to Claim 6.

**21.** A process for preparing a pharmaceutical composition by admixing a pharmaceutically-acceptable carrier or diluent and a therapeutically-effective amount of an active compound being selected from a family of compounds according to Claim 7.

**22.** A process for preparing a pharmaceutical composition by admixing a pharmaceutically-acceptable carrier or diluent and a therapeutically-effective amount of an active compound being selected from a family of compounds according to Claim 8.

**23.** A process for preparing a pharmaceutical composition by admixing a pharmaceutically-acceptable carrier or diluent and a therapeutically-effective amount of an active compound being selected from a family of compounds according to Claim 9.

**24.** A process for preparing a pharmaceutical composition by admixing a pharmaceutically-acceptable carrier or diluent and a therapeutically-effective amount of an active compound being selected from a family of compounds according to Claim 10.

**25.** A process for preparing a pharmaceutical composition by admixing a pharmaceutically-acceptable carrier or diluent and a therapeutically-effective amount of an active compound being selected from a family of compounds according to Claim 11.

**26.** A process for preparing a pharmaceutical composition by admixing a pharmaceutically-acceptable carrier or diluent and a therapeutically-effective amount of an active compound being selected from a family of compounds according to Claim 12.

**27.** A process for preparing a pharmaceutical composition by admixing a pharmaceutically-acceptable carrier or diluent and a therapeutically-effective amount of an active compound being selected from a family of compounds according to Claim 13.

**28.** A process for preparing a pharmaceutical composition by admixing a pharmaceutically-acceptable carrier or diluent and a therapeutically-effective amount of an active compound being selected from a family of compounds according to Claim 14.

**29.** Use of a compound according to Claim 1 for preparing a medicament to control neuropathogical processses and the neurodegenerative consequences thereof in mammals.

**30.** Use of a compound according to any of Claims 2 to 14 for preparing a medicament for control neuropathological processes and the neurodegenerative consequences thereof in mammals.

**31.** Use according to Claim 29 for preparing a medicament for treating hypoxia, anoxia or ischemia.

**32.** Use according to Claim 31 for preparing a medicament for treating ischemia.

**33.** Use of a compound as defined in claims 29-32, selected from the group consisting of
(±)-1-(1,2-diphenylethyl)piperidine;
(-)-1-(1,2-diphenylethyl)piperidine;
(+)-1-(1,2-diphenylethyl)piperidine;
(±)-1-[2-phenyl-1-(2-thienyl)ethyl]piperidine hydrosulfate;
(±)-1-(1,2-diphenylethyl)-1,2,3,6-tetrahydropyridine;
1-(1,2-diphenylethenyl)piperidine;
1-[1-phenyl-2-(2-pyridinyl)ethenyl]piperidine;
(±)-1-[1-phenyl-2-(2-pyridinyl)ethyl]piperidine;
(±)-1-(1,2-diphenylethyl)-1H-azepine;
(±)-1-(1,2-diphenylbutyl)piperidine;
(±)-1-(1,2-diphenyl-3-methylbutyl)piperidine;
(±)-1-(1,2-diphenyl-1-hydroxyethyl)piperidine;
(±)-1-[2-phenyl-1-(2-thienyl)ethyl]-1,2,3,6-tetrahydropyridine;
(±)-1-[1-(1-phenylcyclopentyl)benzyl]piperidine;
(±)-1-[1-(1-phenylcyclohexyl)benzyl]piperidine;
(±)-1-(1,2-diphenylethyl)piperidine;
(±)-1-[1-(4-methylphenyl)-2-phenylethyl]piperidine;
(±)-1-[1-(4-methoxyphenyl)-2-phenylethyl]piperidine;
(±)-1-[1-(1-phenylcyclopropyl)benzyl]piperidine;
(±)-1-[2,2-dimethyl-2-phenyl-1-(2-thienyl)ethyl]piperidine;
(±)-1-[1-(3-methoxyphenyl)-2-phenylethyl]piperidine;
(±)-1-[1-(4-fluorophenyl)-2-phenylethyl]piperidine;
(±)-1-(2,2-dimethyl-1,2-diphenylethyl)piperidine;
(±)-1-[1-(3-methylphenyl)-2-phenylethyl]piperidine;
(±)-1-[1-(2-furyl)-2-phenylethyl]piperidine;
(±)-1-[1-(2-chlorophenyl)-2-phenylethyl]piperidine;
(±)-1-[1-(2-methoxyphenyl)-2-phenylethyl]piperidine; and
(±)-1-[1-(1-naphthyl)-2-phenylethyl]piperidine.

## Patentansprüche

**Patentansprüche für folgende Vertragsstaaten : AT, BE, FR, GB, DE, LU, NL, IT, SE, CH, LI**

**1.** Verbindung der Formel

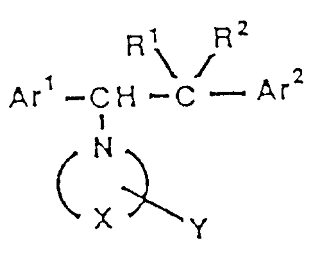

worin jedes von $R^1$ und $R^2$ eine Gruppe ist, die unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählt ist, oder worin $R^1$ und $R^2$ zusammengefaßt werden können, um eine gesättigte oder teilweise ungesättigte $C_3$-$C_{10}$-Cycloalkyl-carbocyclische Gruppe mit drei bis acht Ringkohlenstoffatomen zu bilden;
worin jedes von $Ar^1$ und $Ar^2$ eine Gruppe ist, die unabhängig aus Phenyl-, Thienyl-, Furanyl-, Pyridinyl-, Thiazolyl- und Naphthylgruppen ausgewählt ist und worin jede der genannten $Ar^1$- und $Ar^2$-Gruppen mit einem oder mehreren Resten substituiert sein kann, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, $C_2$-$C_{20}$-Alkenyl, $C_2$-$C_{20}$-Alkinyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin X aus CH oder $CH_2$ ausgewählt ist, um einen fünf- bis siebengliedrigen Ring zu bilden, vorausgesetzt, daß der Ring gesättigt ist oder eine Doppelbindung enthält; und worin Y eine aus Hydrido, $C_1$-$C_{20}$-Alkyl, Halogen, Hydroxyl, $C_1$-$C_{10}$-Alkoxy, Amino, Cyano, Nitro und Mercapto ausgewählte Gruppe ist;
mit der Maßgabe, daß, wenn $Ar^1$ und $Ar^2$ beide Phenyl sind und $R^1$ und $R^2$ Hydrido sind oder $R^1$ H und $R^2$ Hydroxy ist, X nicht eine lineare Alkylenkette mit vier oder fünf Kohlenstoffatomen sein kann, um ein racemisches Gemisch zu bilden;
mit der weiteren Maßgabe, daß, wenn $Ar^1$ para-Methylphenyl oder para-Methoxyphenyl ist und $R^1$ und $R^2$ Hydrido sind und $Ar^2$ Phenyl ist, X nicht eine lineare Alkylenkette mit fünf Kohlenstoffatomen sein kann,
mit Ausnahme der Verbindungen, worin $Ar^1$ Phenyl ist, $R^2$ H ist und X $CH_2$ ist, um einen 6-gliedrigen Ring zu bilden, und gleichzeitig a) $Ar^2$ Phenyl, $R^1$ F oder OH und b) $Ar^2$ Furanyl und $R^1$ OH ist,
und worin $Ar^1$, $Ar^2$ 2, 3 oder 4 $NO_2$-Phenyl ist, $R^2$ H, $R^1$ OH ist und X $CH_2$ ist, um einen 6-gliedrigen Ring zu bilden;
oder worin $R^1$, $R^2$ H sind, X $CH_2$ ist, um einen 6-gliedrigen Ring zu bilden, und $Ar^1$ Naphthyl ist, $Ar^2$ Phenyl ist, oder $Ar^1$ Thienyl und $Ar^2$ Phenyl ist,
und worin $Ar^1$ Methoxyphenyl ist, X $CH_2$ ist, um einen 5-gliedrigen Ring zu bilden, und gleichzeitig $Ar^2$ Methoxyphenyl ist, $R^1$ = H, $R^2$ = OH oder $R^1$, $R^2$ = H.

2. Verbindung nach Anspruch 1 mit der Formel

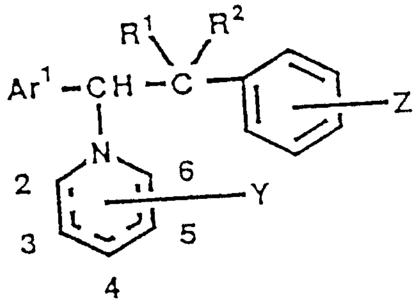

worin jedes von $R^1$ und $R^2$ eine Gruppe ist, die unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählt ist, oder worin $R^1$ und $R^2$ zusammengefaßt werden können, um eine gesättigte oder teilweise ungesättigte carbocyclische Gruppe mit drei oder sieben Ringkohlenstoffatomen zu bilden; worin $Ar^1$ eine aus Phenyl-, Thienyl-, Furanyl-, Pyridinyl-, Thiazolyl- und Naphthylgruppen ausgewählte Gruppe ist und jede der genannten $Ar^1$-Gruppen, die eine substituierbare Position aufweist, mit einem oder mehreren Resten substituiert sein kann, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$-Alkyl, Halogen, Hydroxyl, Alkoxy und Amino ausgewählte Gruppe ist; und worin Z eine oder mehrere Gruppen bedeutet, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind.

3. Verbindung nach Anspruch 2, worin jedes von $R^1$ und $R^2$ eine unabhängig aus Hydrido, $C_1$-$C_{20}$ linearem oder verzweigtem Alkyl, $C_3$-$C_{10}$-Cycloalkyl, Hydroxyl, Halogen, $C_1$-$C_{10}$-Alkoxy ausgewählte Gruppe ist, oder worin $R^1$ und $R^2$ zusammengefaßt werden können, um eine gesättigte oder teilweise

ungesättigte carbocyclische Gruppe mit drei oder sieben Ringkohlenstoffatomen zu bilden; worin $Ar^1$ aus Phenyl-, Thienyl-, Pyridinyl- und Naphthylgruppen ausgewählt ist und worin jede der genannten $Ar^1$-Gruppen, die eine substituierbare Position aufweist, mit einem oder mehreren Resten substituiert sein kann, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; und worin Z eine oder mehrere Gruppen bedeutet, die aus Hydrido, $C_1$-$C_{20}$ linearem oder verzweigtem Alkyl, $C_3$-$C_{10}$-Cycloalkyl, Halogen, Hydroxyl, $C_1$-$C_{10}$-Alkoxy und Amino ausgewählt sind.

**4.** Verbindung nach Anspruch 3, worin jedes von $R^1$ und $R^2$ eine Gruppe ist, die unabhängig aus Hydrido, $C_1$-$C_{20}$ linearem oder verzweigtem Alkyl und Hydroxyl ausgewählt ist, oder worin $R^1$ und $R^2$ zusammengefaßt werden können, um eine gesättigte oder teilweise ungesättigte carbocyclische Gruppe mit drei bis sechs Ringkohlenstoffatomen zu bilden; worin $Ar^1$ eine aus Phenyl-, Thienyl-, Pyridinyl- und Naphthylgruppen ausgewählte Gruppe ist und worin jede der genannten $Ar^1$-Gruppen, die eine oder mehrere substituierbare Positionen aufweist, mit einem oder mehreren Resten substituiert sein kann, die aus Hydrido, $C_1$-$C_{20}$ linearem oder verzweigtem Alkyl, Halogen, $C_1$-$C_{20}$-Halogenalkyl, Hydroxyl, $C_1$-$C_{10}$-Alkoxy, Amino und Nitro ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; und worin Z eine oder mehrere Gruppen bedeutet, die aus Hydrido, $C_1$-$C_{20}$ linearem oder verzweigtem Alkyl, Halogen, Hydroxyl und $C_1$-$C_{10}$-Alkoxy ausgewählt sind.

**5.** Verbindung nach Anspruch 1 mit der Formel

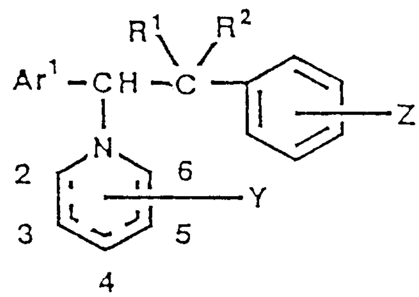

worin jedes von $R^1$ und $R^2$ eine Gruppe darstellt, die unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählt ist, oder worin $R^1$ und $R^2$ zusammengefaßt werden können, um eine gesättigte oder teilweise ungesättigte carbocyclische Gruppe mit drei bis sieben Ringkohlenstoffatomen zu bilden; worin $Ar^1$ eine aus Phenyl-, Thienyl-, Furanyl-, Pyridinyl-, Thiazolyl- und Naphthylgruppen ausgewählte Gruppe ist und worin jede der genannten $Ar^1$-Gruppen, die eine substituierbare Position aufweist, mit einem oder mehreren Resten substituiert ist, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere Gruppen bedeutet, die aus $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; und worin die strichlierte Linie im N-haltigen Ring eine Doppelbindung zwischen irgendwelchen zwei benachbarten Kohlenstoffatomen darstellt, wobei die 2-, 3-, 4-, 5- und 6-Position des N-haltigen Rings involviert ist.

**6.** Verbindung nach Anspruch 5, worin jedes von $R^1$ und $R^2$ eine unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählte Gruppe darstellt, oder worin $R^1$ und $R^2$ zusammengefaßt werden können, um eine gesättigte oder teilweise ungesättigte carbocyclische Gruppe mit drei bis sieben Ringkohlenstoffatomen zu bilden; worin $Ar^1$ eine aus Phenyl-, Thienyl-, Furanyl-, Pyridinyl-, Thiazolyl- und Naphthylgruppen ausgewählte Gruppe ist und worin jede der genannten Gruppen, die eine oder mehrere substituierbare Positionen aufweist, mit einem oder mehreren Resten substituiert sein kann, die aus Hydrido, $C_1$-$C_{20}$ linearem

oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere Gruppen bedeutet, die aus Hydrido, $C_1$-$C_{20}$ linearem oder verzweigtem Alkyl, $C_3$-$C_{10}$-Cycloalkyl, Halogen, Hydroxyl, $C_1$-$C_{10}$-Alkoxy und Amino ausgewählt sind; und worin die strichlierte Linie innerhalb des N-haltigen Rings eine Doppelbindung zwischen irgendwelchen zwei benachbarten Kohlenstoffatomen bedeutet, wobei die 3-, 4- und 5-Position des N-haltigen Rings involviert ist.

**7.** Verbindung nach Anspruch 6, worin jedes von $R^1$ und $R^2$ eine unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählte Gruppe ist; worin $R^1$ und $R^2$ zusammengefaßt werden können, um ein gesättigtes oder teilweise ungesättigtes Cycloalkyl mit drei bis sieben Ringkohlenstoffatomen zu bilden; worin $Ar^1$ aus Phenyl-, Thienyl-, Pyridinyl- und Naphthylgruppen ausgewählt ist und worin jede der genannten Gruppen, die eine substituierbare Position aufweist, mit einem oder mehreren Resten substituiert ist, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere aus Hydrido, $C_1$-$C_{20}$ linearem oder verzweigtem Alkyl, $C_3$-$C_{10}$-Cycloalkyl, Halogen, Hydroxyl, $C_1$-$C_{10}$-Alkoxy und Amino ausgewählte Gruppen bedeutet; und worin die strichlierte Linie innerhalb des N-haltigen Rings jeweils eine Doppelbindung zwischen irgendwelchen zwei benachbarten Kohlenstoffatomen bedeutet, wobei die 3-, 4- und 5-Position des N-haltigen Rings involviert ist.

**8.** Verbindung nach Anspruch 7, worin jedes von $R^1$ und $R^2$ eine unabhängig aus Hydrido, $C_1$-$C_{20}$ linearem oder verzweigtem Alkyl und Hydroxyl ausgewählte Gruppe ist; worin $R^1$ und $R^2$ zusammengefaßt werden können, um ein gesättigtes oder teilweise ungesättigtes Cycloalkyl mit drei bis sechs Ringkohlenstoffatomen zu bilden; worin $Ar^1$ aus Phenyl-, Thienyl-, Pyridinyl- und Naphthylgruppen ausgewählt ist und worin jede der genannten Gruppen, die eine oder mehrere substituierbare Positionen aufweist, mit einem oder mehreren Resten substituiert ist, die aus Hydrido, $C_1$-$C_{20}$ linearem oder verzweigtem Alkyl, Halogen, $C_1$-$C_{20}$-Halogenalkyl, Hydroxyl, $C_1$-$C_{10}$-Alkoxy, Amino und Nitro ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere Gruppen bedeutet, die aus Hydrido, $C_1$-$C_{20}$ linearem oder verzweigtem Alkyl, Halogen, Hydroxyl und $C_1$-$C_{10}$-Alkoxy ausgewählt sind; und worin die strichlierte Linie innerhalb des N-haltigen Rings jeweils eine Doppelbindung zwischen irgendwelchen zwei benachbarten Kohlenstoffatomen darstellt, wobei die 3-, 4- und 5-Position des N-haltigen Rings involviert ist.

**9.** Verbindung nach Anspruch 1, ausgewählt aus der Gruppe bestehend aus
(-)-1-(1,2-Diphenylethyl)piperidin;
( + )-1-(1,2-Diphenylethyl)piperidin;
(±)-1-[1-Phenyl-2-(2-pyridinyl)ethyl]piperidin;
1-[1-Phenyl-2-(2-pyridinyl)ethenyl]piperidin;
(±)-1-(1,2-Diphenylethyl)-1H-azepin;
(±)-1-(1,2-Diphenylbutyl)piperidin;
(±)-1-(1,2-Diphenyl-3-methylbutyl)piperidin;
(±)-1-[1-(1-Phenylcyclopentyl)benzyl]piperidin;
(±)-1-[1-(1-Phenylcyclohexyl)benzyl]piperidin;
(±)-1-[1-(1-Phenylcyclopropyl)benzyl]piperidin;
(±)-1-[2,2-Dimethyl-2-phenyl-1-(2-thienyl)ethyl]piperidin;
(±)-1-[1-(3-Methoxyphenyl)-2-phenylethyl]piperidin;
(±)-1-[1-(4-Fluorphenyl)-2-phenylethyl]piperidin;
(±)-1-(2,2-Dimethyl-1,2-diphenylethyl)piperidin;
(±)-1-[1-(3-Methylphenyl)-2-phenylethyl]piperidin;
(±)-1-[1-(2-Furyl)-2-phenylethyl]piperidin;
(±)-1-[1-(2-Chlorphenyl)-2-phenylethyl]piperidin;
(±)-1-[1-(2-Methoxyphenyl)-2-phenylethyl]piperidin;
(±)-1-(1,2-Diphenylethyl)-1,2,3,6-tetrahydropyridin und

(±)-1-[2-Phenyl-1-(2-thienyl)ethyl]-1,2,3,6-tetrahydropyridin.

**10.** Verbindung nach Anspruch 9, ausgewählt aus der Gruppe bestehend aus
(-)-1-(1,2-Diphenylethyl)piperidin;
( + )-1-(1,2-Diphenylethyl)piperidin;
(±)-1-[1-Phenyl-2-(2-pyridinyl)ethyl]piperidin;
(±)-1-(1,2-Diphenylethyl)-1H-azepin;
(±)-1-(1,2-Diphenylbutyl)piperidin;
(±)-1-[1-(1-Phenylcyclohexyl)benzyl]piperidin;
(±)-1-[1-(1-Phenylcyclopropyl)benzyl]piperidin;
(±)-1-[1-(3-Methoxyphenyl)-2-phenylethyl]piperidin;
(±)-1-[1-(4-Fluorphenyl)-2-phenylethyl]piperidin;
(±)-1-(2,2-Dimethyl-1,2-diphenylethyl)piperidin;
(±)-1-[1-(3-Methylphenyl)-2-phenylethyl]piperidin;
(±)-1-[1-(2-Chlorphenyl)-2-phenylethyl]piperidin und
(±)-1-[1-(2-Methoxyphenyl)-2-phenylethyl]piperidin.

**11.** Verbindung nach Anspruch 10, ausgewählt aus der Gruppe bestehend aus
(±)-1-[1-(3-Methoxyphenyl)-2-phenylethyl]piperidin;
(±)-1-[1-(4-Fluorphenyl)-2-phenylethyl]piperidin;
(±)-1-[1-(3-Methylphenyl)-2-phenylethyl]piperidin;
(±)-1-[1-(2-Chlorphenyl)-2-phenylethyl]piperidin und
(±)-1-[1-(2-Methoxyphenyl)-2-phenylethyl]piperidin.

**12.** Verbindung nach Anspruch 11, die (±)-1-[1-(2-Chlorphenyl)-2-phenylethyl]piperidin ist.

**13.** Verbindung nach Anspruch 9, ausgewählt aus der Gruppe bestehend aus (±)-1-(1,2-Diphenylethyl)-1,2,3,6-tetrahydropyridin und (±)-1-[2-Phenyl-1-(2-thienyl)ethyl]-1,2,3,6-tetrahydropyridin.

**14.** Verbindung nach Anspruch 13, die (±)-1-[2-Phenyl-1-(2-thienyl)ethyl]-1,2,3,6-tetrahydropyridin ist.

**15.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Familie von Verbindungen nach Anspruch 1 ausgewählt ist.

**16.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 2 ausgewählt ist.

**17.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 3 ausgewählt ist.

**18.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 4 ausgewählt ist.

**19.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 5 ausgewählt ist.

**20.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 6 ausgewählt ist.

**21.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 7 ausgewählt ist.

**22.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 8 ausgewählt ist.

**23.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 9 ausgewählt ist.

**24.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 10 ausgewählt ist.

**25.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 11 ausgewählt ist.

**26.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 12 ausgewählt ist.

**27.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 13 ausgewählt ist.

**28.** Pharmazeutische Zusammensetzung, die eine therapeutisch wirksame Menge eines Wirkstoffes und einen pharmazeutisch unbedenklichen Träger oder Verdünnungsmittel enthält, wobei der Wirkstoff aus einer Verbindung nach Anspruch 14 ausgewählt ist.

**29.** Verwendung einer Verbindung nach Anspruch 1 zur Herstellung eines Arzneimittels zur Beherrschung neuropathologischer Prozesse und deren neurodegenerativer Folgen bei Säugern.

**30.** Verwendung einer Verbindung nach irgendeinem der Ansprüche 2 bis 14 zur Herstellung eines Arzneimittels zur Beherrschung neuropathologischer Prozesse und deren neurodegenerativer Folgen bei Säugern.

**31.** Verwendung nach Anspruch 29 zur Herstellung eines Arzneimittels zur Behandlung von Hypoxie, Anoxie oder Ischämie.

**32.** Verwendung nach Anspruch 31 zur Herstellung eines Arzneimittels zur Behandlung von Ischämie.

**33.** Verwendung einer Verbindung, wie in den Ansprüchen 29-32 definiert, ausgewählt aus der Gruppe bestehend aus
(±)-1-(1,2-Diphenylethyl)piperidin;
(-)-1-(1,2-Diphenylethyl)piperidin;
(+)-1-(1,2-Diphenylethyl)piperidin;
(±)-1-[2-Phenyl-1-(2-thienyl)ethyl]piperidin-hydrosulfat;
(±)-1-(1,2-Diphenylethyl)-1,2,3,6-tetrahydropyridin;
1-(1,2-Diphenylethenyl)piperidin;
1-[1-Phenyl-2-(2-pyridinyl)ethenyl]piperidin;
(±)-1-[1-Phenyl-2-(2-pyridinyl)ethyl]piperidin;
(±)-1-(1,2-Diphenylethyl)-1H-azepin;
(±)-1-(1,2-Diphenylbutyl)piperidin;
(±)-1-(1,2-Diphenyl-3-methylbutyl)piperidin;
(±)-1-(1,2-Diphenyl-1-hydroxyethyl)piperidin;
(±)-1-[2-Phenyl-1-(2-thienyl)ethyl]-1,2,3,6-tetrahydropyridin;
(±)-1-[1-(1-Phenylcyclopentyl)benzyl]piperidin;
(±)-1-[1-(1-Phenylcyclohexyl)benzyl]piperidin;
(±)-1-(1,2-Diphenylethyl)pyrrolidin;

(±)-1-[1-(4-Methylphenyl)-2-phenylethyl]piperidin;
(±)-1-[1-(4-Methoxyphenyl)-2-phenylethyl]piperidin;
(±)-1-[1-(1-Phenylcyclopropyl)benzyl]piperidin;
(±)-1-[2,2-Dimethyl-2-phenyl-1-(2-thienyl)ethyl]piperidin;
(±)-1-[1-(3-Methoxyphenyl)-2-phenylethyl]piperidin;
(±)-1-[1-(4-Fluorphenyl)-2-phenylethyl]piperidin;
(±)-1-(2,2-Dimethyl-1,2-diphenylethyl)piperidin;
(±)-1-[1-(3-Methylphenyl)-2-phenylethyl]piperidin;
(±)-1-[1-(2-Furyl)-2-phenylethyl]piperidin;
(±)-1-[1-(2-Chlorphenyl)-2-phenylethyl]piperidin;
(±)-1-[1-(2-Methoxyphenyl)-2-phenylethyl]piperidin und
(±)-1-[1-(1-Naphthyl)-2-phenylethyl]piperidin.

**Patentansprüche für folgenden Vertragsstaat : ES**

**1.** Verfahren zur Herstellung einer Verbindung der Formel

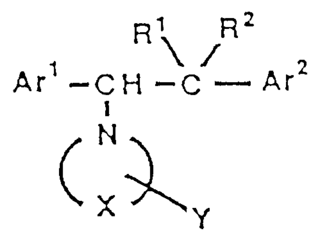

worin jedes von $R^1$ und $R^2$ eine Gruppe ist, die unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählt ist, oder worin $R^1$ und $R^2$ zusammengefaßt werden können, um eine gesättigte oder teilweise ungesättigte $C_3$-$C_{10}$-Cycloalkyl-carbocyclische Gruppe mit drei bis acht Ringkohlenstoffatomen zu bilden;
worin jedes von $Ar^1$ und $Ar^2$ eine Gruppe ist, die unabhängig aus Phenyl-, Thienyl-, Furanyl-, Pyridinyl-, Thiazolyl- und Naphthylgruppen ausgewählt ist und worin jede der genannten $Ar^1$- und $Ar^2$-Gruppen mit einem oder mehreren Resten substituiert sein kann, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, $C_2$-$C_{20}$-Alkenyl, $C_2$-$C_{20}$-Alkinyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin X aus CH oder $CH_2$ ausgewählt ist, um einen fünf- bis siebengliedrigen Ring zu bilden, vorausgesetzt, daß der Ring gesättigt ist oder eine Doppelbindung enthält; und worin Y eine aus Hydrido, $C_1$-$C_{20}$-Alkyl, Halogen, Hydroxyl, $C_1$-$C_{10}$-Alkoxy, Amino, Cyano, Nitro und Mercapto ausgewählte Gruppe ist;
mit der Maßgabe, daß, wenn $Ar^1$ und $Ar^2$ beide Phenyl sind und $R^1$ und $R^2$ Hydrido sind oder $R^1$ H und $R^2$ Hydroxy ist, X nicht eine lineare Alkylenkette mit vier oder fünf Kohlenstoffatomen sein kann, um ein racemisches Gemisch zu bilden;
mit der weiteren Maßgabe, daß, wenn $Ar^1$ para-Methylphenyl oder para-Methoxyphenyl ist und $R^1$ und $R^2$ Hydrido sind und $Ar^2$ Phenyl ist, X nicht eine lineare Alkylenkette mit fünf Kohlenstoffatomen sein kann,
mit Ausnahme der Verbindungen, worin $Ar^1$ Phenyl ist, $R^2$ H ist und X $CH_2$ ist, um einen 6-gliedrigen Ring zu bilden, und gleichzeitig a) $Ar^2$ Phenyl, $R^1$ F oder OH ist, b) $Ar^2$ Furanyl und $R^1$ OH ist,
und worin $Ar^1$, $Ar^2$ 2, 3 oder 4 $NO_2$-Phenyl ist, $R^2$ H, $R^1$ OH ist und X $CH_2$ ist, um einen 6-gliedrigen Ring zu bilden;
oder worin $R^1$, $R^2$ H sind, X $CH_2$ ist, um einen 6-gliedrigen Ring zu bilden, und $Ar^1$ Naphthyl ist, $Ar^2$ Phenyl ist, oder $Ar^1$ Thienyl und $Ar^2$ Phenyl ist,
und worin $Ar^1$ Methoxyphenyl ist, X $CH_2$ ist, um einen 5-gliedrigen Ring zu bilden, und gleichzeitig $Ar^2$ Methoxyphenyl ist $R^1$ = H, $R^2$ = OH oder $R^1$, $R^2$ = H, dadurch gekennzeichnet, daß

A) ein Amin der Formel (2)

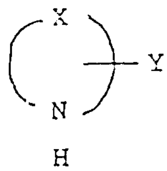

(2)

worin Y und X wie zuvor definiert sind, in Gegenwart einer Bronsted- oder Lewis-Säure in einem geeigneten Lösungsmittel mit einem Keton der Formel (1) umgesetzt wird,

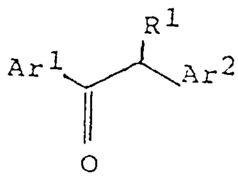

(1)

in der $Ar^1$, $Ar^2$, $R^1$ wie zuvor definiert sind, wobei diese Reaktion (A) das Imin (3) der Formel

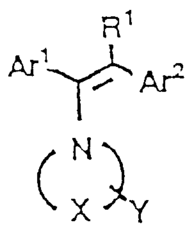

(3)

bildet, in der alle Substituenten wie zuvor definiert sind, dieses Imin (3) anschließend durch Reduktion mit einem geeigneten Reduktionsmittel in einem Lösungsmittel transformiert wird, wodurch man die gewünschte Verbindung der Formel

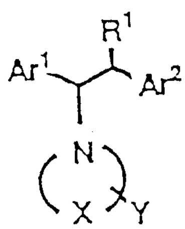

erhält, oder

B) durch Umsetzung eines Amins der Formel (2)

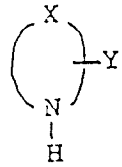

(2)

in der X und Y wie zuvor definiert sind, unverdünnt oder in einem Lösungsmittel, mit einer

Verbindung der Formel (9)

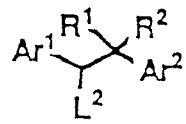

in der $Ar^1$, $Ar^2$, $R^1$, $R^2$ wie zuvor definiert sind und $L^2$ eine geeignete Abgangsgruppe ist, wobei diese Verbindung der Formel (9) aus dem entsprechenden Keton gebildet wird, wodurch die gewünschten Verbindungen erhalten werden, oder

C) durch Umsetzung einer Verbindung der Formel (12)

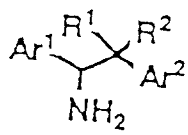

in der alle Substituenten wie zuvor definiert sind und die aus dem entsprechenden Keton gebildet ist oder aus der Reaktion des Nitrils (1) $AR^2CN$ mit einer Verbindung der Formel

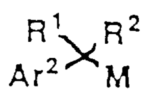

in der die Substituenten wie zuvor definiert sind, mit einer Verbindung der Formel (13)

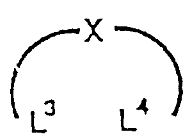

(13)

in der X wie zuvor definiert ist und $L^3$ und $L^4$ gute Abgangsgruppen sind, wodurch die gewünschten Verbindungen erhalten werden.

**2.** Verfahren nach Anspruch 1 zur Herstellung einer Verbindung mit der Formel

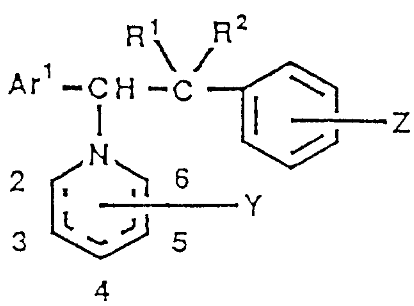

worin jedes von $R^1$ und $R^2$ eine Gruppe ist, die unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählt ist, oder worin $R^1$ und $R^2$ zusammengefaßt werden können, um eine gesättigte oder teilweise ungesättigte carbocyclische Gruppe mit drei oder sieben Ringkohlenstoffatomen zu bilden; worin $Ar^1$ eine aus Phenyl-, Thienyl-, Furanyl-, Pyridinyl-, Thiazolyl- und Naphthylgruppen ausgewählte Gruppe ist und jede der genannten $Ar^1$-Gruppen, die eine substituierbare Position aufweist, mit einem oder mehreren Resten

substituiert sein kann, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$-Alkyl, Halogen, Hydroxyl, Alkoxy und Amino ausgewählte Gruppe ist; und worin Z eine oder mehrere Gruppen bedeutet, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind.

**3.** Verfahren nach Anspruch 2, worin jedes von $R^1$ und $R^2$ eine unabhängig aus Hydrido, $C_1$-$C_{20}$ linearem oder verzweigtem Alkyl, $C_3$-$C_{10}$-Cycloalkyl, Hydroxyl, Halogen, $C_1$-$C_{10}$-Alkoxy ausgewählte Gruppe ist, oder worin $R^1$ und $R^2$ zusammengefaßt werden können, um eine gesättigte oder teilweise ungesättigte carbocyclische Gruppe mit drei oder sieben Ringkohlenstoffatomen zu bilden; worin $Ar^1$ aus Phenyl-, Thienyl-, Pyridinyl- und Naphthylgruppen ausgewählt ist und worin jede der genannten $Ar^1$-Gruppen, die eine substituierbare Position aufweist, mit einem oder mehreren Resten substituiert sein kann, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; und worin Z eine oder mehrere Gruppen bedeutet, die aus Hydrido, $C_1$-$C_{20}$ linearem oder verzweigtem Alkyl, $C_3$-$C_{10}$-Cycloalkyl, Halogen, Hydroxyl, $C_1$-$C_{10}$-Alkoxy und Amino ausgewählt sind.

**4.** Verfahren nach Anspruch 3, worin jedes von $R^1$ und $R^2$ eine Gruppe ist, die unabhängig aus Hydrido, $C_1$-$C_{20}$ linearem oder verzweigtem Alkyl und Hydroxyl ausgewählt ist, oder worin $R^1$ und $R^2$ zusammengefaßt werden können, um eine gesättigte oder teilweise ungesättigte carbocyclische Gruppe mit drei bis sechs Ringkohlenstoffatomen zu bilden; worin $Ar^1$ eine aus Phenyl-, Thienyl-, Pyridinyl- und Naphthylgruppen ausgewählte Gruppe ist und worin jede der genannten $Ar^1$-Gruppen, die eine oder mehrere substituierbare Positionen aufweist, mit einem oder mehreren Resten substituiert sein kann, die aus Hydrido, $C_1$-$C_{20}$ linearem oder verzweigtem Alkyl, Halogen, $C_1$-$C_{20}$-Halogenalkyl, Hydroxyl, $C_1$-$C_{10}$-Alkoxy, Amino und Nitro ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; und worin Z eine oder mehrere Gruppen bedeutet, die aus Hydrido, $C_1$-$C_{20}$ linearem oder verzweigtem Alkyl, Halogen, Hydroxyl und $C_1$-$C_{10}$-Alkoxy ausgewählt sind.

**5.** Verfahren nach Anspruch 1 zur Herstellung einer Verbindung mit der Formel

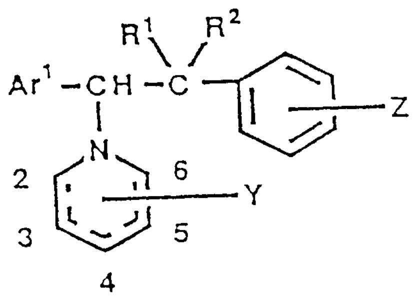

worin jedes von $R^1$ und $R^2$ eine Gruppe darstellt, die unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählt ist, oder worin $R^1$ und $R^2$ zusammengefaßt werden können, um eine gesättigte oder teilweise ungesättigte carbocyclische Gruppe mit drei bis sieben Ringkohlenstoffatomen zu bilden; worin $Ar^1$ eine aus Phenyl-, Thienyl-, Furanyl-, Pyridinyl-, Thiazolyl- und Naphthylgruppen ausgewählte Gruppe ist und worin jede der genannten $Ar^1$-Gruppen, die eine substituierbare Position aufweist, mit einem oder mehreren Resten substituiert ist, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere Gruppen bedeutet, die aus $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano,

Nitro und Mercapto ausgewählt sind; und worin die strichlierte Linie im N-haltigen Ring eine Doppelbindung zwischen irgendwelchen zwei benachbarten Kohlenstoffatomen darstellt, wobei die 2-, 3-, 4-, 5- und 6-Position des N-haltigen Rings involviert ist.

**6.** Verfahren nach Anspruch 5, worin jedes von $R^1$ und $R^2$ eine unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählte Gruppe darstellt, oder worin $R^1$ und $R^2$ zusammengefaßt werden können, um eine gesättigte oder teilweise ungesättigte carbocyclische Gruppe mit drei bis sieben Ringkohlenstoffatomen zu bilden; worin $Ar^1$ eine aus Phenyl-, Thienyl-, Furanyl-, Pyridinyl-, Thiazolyl- und Naphthylgruppen ausgewählte Gruppe ist und worin jede der genannten Gruppen, die eine oder mehrere substituierbare Positionen aufweist, mit einem oder mehreren Resten substituiert sein kann, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere Gruppen bedeutet, die aus Hydrido, $C_1$-$C_{20}$ linearem oder verzweigtem Alkyl, $C_3$-$C_{10}$-Cycloalkyl, Halogen, Hydroxyl, $C_1$-$C_{10}$-Alkoxy und Amino ausgewählt sind; und worin die strichlierte Linie innerhalb des N-haltigen Rings eine Doppelbindung zwischen irgendwelchen zwei benachbarten Kohlenstoffatomen bedeutet, wobei die 3-, 4- und 5-Position des N-haltigen Rings involviert ist.

**7.** Verfahren nach Anspruch 6, worin jedes von $R^1$ und $R^2$ eine unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählte Gruppe ist; worin $R^1$ und $R^2$ zusammengefaßt werden können, um ein gesättigtes oder teilweise ungesättigtes Cycloalkyl mit drei bis sieben Ringkohlenstoffatomen zu bilden; worin $Ar^1$ aus Phenyl-, Thienyl-, Pyridinyl- und Naphthylgruppen ausgewählt ist und worin jede der genannten Gruppen, die eine substituierbare Position aufweist, mit einem oder mehreren Resten substituiert ist, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere aus Hydrido, $C_1$-$C_{20}$ linearem oder verzweigtem Alkyl, $C_3$-$C_{10}$-Cycloalkyl, Halogen, Hydroxyl, $C_1$-$C_{10}$-Alkoxy und Amino ausgewählte Gruppen bedeutet; und worin die strichlierte Linie innerhalb des N-haltigen Rings jeweils eine Doppelbindung zwischen irgendwelchen zwei benachbarten Kohlenstoffatomen bedeutet, wobei die 3-, 4- und 5-Position des N-haltigen Rings involviert ist.

**8.** Verfahren nach Anspruch 7, worin jedes von $R^1$ und $R^2$ eine unabhängig aus Hydrido, $C_1$-$C_{20}$ linearem oder verzweigtem Alkyl und Hydroxyl ausgewählte Gruppe ist; worin $R^1$ und $R^2$ zusammengefaßt werden können, um ein gesättigtes oder teilweise ungesättigtes Cycloalkyl mit drei bis sechs Ringkohlenstoffatomen zu bilden; worin $Ar^1$ aus Phenyl-, Thienyl-, Pyridinyl- und Naphthylgruppen ausgewählt ist und worin jede der genannten Gruppen, die eine oder mehrere substituierbare Positionen aufweist, mit einem oder mehreren Resten substituiert ist, die aus Hydrido, $C_1$-$C_{20}$ linearem oder verzweigtem Alkyl, Halogen, $C_1$-$C_{20}$-Halogenalkyl, Hydroxyl, $C_1$-$C_{10}$-Alkoxy, Amino und Nitro ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere Gruppen bedeutet, die aus Hydrido, $C_1$-$C_{20}$ linearem oder verzweigtem Alkyl, Halogen, Hydroxyl und $C_1$-$C_{10}$-Alkoxy ausgewählt sind; und worin die strichlierte Linie innerhalb des N-haltigen Rings jeweils eine Doppelbindung zwischen irgendwelchen zwei benachbarten Kohlenstoffatomen darstellt, wobei die 3-, 4- und 5-Position des N-haltigen Rings involviert ist.

**9.** Verfahren nach Anspruch 1 zur Herstellung einer Verbindung, die ausgewählt ist aus der Gruppe bestehend aus
(-)-1-(1,2-Diphenylethyl)piperidin;
(+)-1-(1,2-Diphenylethyl)piperidin;
(±)-1-[1-Phenyl-2-(2-pyridinyl)ethyl]piperidin;
1-[1-Phenyl-2-(2-pyridinyl)ethenyl]piperidin;
(±)-1-(1,2-Diphenylethyl)-1H-azepin;
(±)-1-(1,2-Diphenylbutyl)piperidin;
(±)-1-(1,2-Diphenyl-3-methylbutyl)piperidin;

(±)-1-[1-(1-Phenylcyclopentyl)benzyl]piperidin;
(±)-1-[1-(1-Phenylcyclohexyl)benzyl]piperidin;
(±)-1-[1-(1-Phenylcyclopropyl)benzyl]piperidin;
(±)-1-[2,2-Dimethyl-2-phenyl-1-(2-thienyl)ethyl]piperidin;
(±)-1-[1-(3-Methoxyphenyl)-2-phenylethyl]piperidin;
(±)-1-[1-(4-Fluorphenyl)-2-phenylethyl)piperidin;
(±)-1-(2,2-Dimethyl-1,2-diphenylethyl)piperidin;
(±)-1-[1-(3-Methylphenyl)-2-phenylethyl]piperidin;
(±)-1-[1-(2-Furyl)-2-phenylethyl]piperidin;
(±)-1-[1-(2-Chlorphenyl)-2-phenylethyl]piperidin;
(±)-1-[1-(2-Methoxyphenyl)-2-phenylethyl]piperidin;
(±)-1-(1,2-Diphenylethyl)-1,2,3,6-tetrahydropyridin und
(±)-1-[2-Phenyl-1-(2-thienyl)ethyl]-1,2,3,6-tetrahydropyridin.

**10.** Verfahren nach Anspruch 9 zur Herstellung einer Verbindung, die ausgewählt ist aus der Gruppe bestehend aus
(-)-1-(1,2-Diphenylethyl)piperidin;
(+)-1-(1,2-Diphenylethyl)piperidin;
(±)-1-[1-Phenyl-2-(2-pyridinyl)ethyl]piperidin;
(±)-1-(1,2-Diphenylethyl)-1H-azepin;
(±)-1-(1,2-Diphenylbutyl)piperidin;
(±)-1-[1-(1-Phenylcyclohexyl)benzyl]piperidin;
(±)-1-[1-(1-Phenylcyclopropyl)benzyl]piperidin;
(±)-1-[1-(3-Methoxyphenyl)-2-phenylethyl]piperidin;
(±)-1-[1-(4-Fluorphenyl)-2-phenylethyl]piperidin;
(±)-1-(2,2-Dimethyl-1,2-diphenylethyl)piperidin;
(±)-1-[1-(3-Methylphenyl)-2-phenylethyl]piperidin;
(±)-1-[1-(2-Chlorphenyl)-2-phenylethyl]piperidin und
(±)-1-[1-(2-Methoxyphenyl)-2-phenylethyl]piperidin.

**11.** Verfahren nach Anspruch 10 zur Herstellung einer Verbindung, die ausgewählt ist aus der Gruppe bestehend aus
(±)-1-[1-(3-Methoxyphenyl)-2-phenylethyl]piperidin;
(±)-1-[1-(4-Fluorphenyl)-2-phenylethyl]piperidin;
(±)-1-[1-(3-Methylphenyl)-2-phenylethyl]piperidin;
(±)-1-[1-(2-Chlorphenyl)-2-phenylethyl]piperidin und
(±)-1-[1-(2-Methoxyphenyl)-2-phenylethyl]piperidin.

**12.** Verfahren nach Anspruch 11, worin die hergestellte Verbindung (±)-1-[1-(2-Chlorphenyl)-2-phenylethyl]-piperidin ist.

**13.** Verfahren nach Anspruch 9 zur Herstellung einer Verbindung, ausgewählt aus der Gruppe bestehend aus (±)-1-(1,2-Diphenylethyl)-1,2,3,6-tetrahydropyridin und (±)-1-[2-Phenyl-1-(2-thienyl)ethyl]-1,2,3,6-tetrahydropyridin.

**14.** Verfahren nach Anspruch 13 zur Herstellung von (±)-1-[2-Phenyl-1-(2-thienyl)ethyl]-1,2,3,6-tetrahydropyridin.

**15.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 1 hergestellt werden.

**16.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 2 hergestellt werden.

**17.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 3 hergestellt werden.

**18.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 4 hergestellt werden.

**19.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 5 hergestellt werden.

**20.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 6 hergestellt werden.

**21.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 7 hergestellt werden.

**22.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 8 hergestellt werden.

**23.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 9 hergestellt werden.

**24.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 10 hergestellt werden.

**25.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 11 hergestellt werden.

**26.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 12 hergestellt werden.

**27.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 13 hergestellt werden.

**28.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen ausgewählt ist, die nach Anspruch 14

hergestellt werden.

**29.** Verwendung einer Verbindung nach Anspruch 1 zur Herstellung eines Arzneimittels zur Beherrschung neuropathologischer Prozesse und deren neurodegenerativer Folgen bei Säugern.

**30.** Verwendung einer Verbindung nach Anspruch 2 bis 14 zur Herstellung eines Arzneimittels zur Beherrschung neuropathologischer Prozesse und deren neurodegenerativer Folgen bei Säugern.

**31.** Verwendung nach Anspruch 29 zur Herstellung eines Arzneimittels zur Behandlung von Hypoxie, Anoxie oder Ischämie.

**32.** Verwendung nach Anspruch 31 zur Herstellung eines Arzneimittels zur Behandlung von Ischämie.

**33.** Verwendung einer Verbindung, wie in den Ansprüchen 29-32 definiert, ausgewählt aus der Gruppe bestehend aus
(±)-1-(1,2-Diphenylethyl)piperidin;
(-)-1-(1,2-Diphenylethyl)piperidin;
(+)-1-(1,2-Diphenylethyl)piperidin;
(±)-1-[2-Phenyl-1-(2-thienyl)ethyl]piperidin-hydrosulfat;
(±)-1-(1,2-Diphenylethyl)-1,2,3,6-tetrahydropyridin;
1-(1,2-Diphenylethenyl)piperidin;
1-[1-Phenyl-2-(2-pyridinyl)ethenyl]piperidin;
(±)-1-[1-Phenyl-2-(2-pyridinyl)ethyl]piperidin;
(±)-1-(1,2-Diphenylethyl)-1H-azepin;
(±)-1-(1,2-Diphenylbutyl)piperidin;
(±)-1-(1,2-Diphenyl-3-methylbutyl)piperidin;
(±)-1-(1,2-Diphenyl-1-hydroxyethyl)piperidin;
(±)-1-[2-Phenyl-1-(2-thienyl)ethyl]-1,2,3,6-tetrahydropyridin;
(±)-1-[1-(1-Phenylcyclopentyl)benzyl]piperidin;
(±)-1-[1-(1-Phenylcyclohexyl)benzyl]piperidin;
(±)-1-(1,2-Diphenylethyl)pyrrolidin;
(±)-1-[1-(4-Methylphenyl)-2-phenylethyl]piperidin;
(±)-1-[1-(4-Methoxyphenyl)-2-phenylethyl]piperidin;
(±)-1-[1-(1-Phenylcyclopropyl)benzyl]piperidin;
(±)-1-[2,2-Dimethyl-2-phenyl-1-(2-thienyl)ethyl]piperidin;
(±)-1-[1-(3-Methoxyphenyl)-2-phenylethyl]piperidin;
(±)-1-[1-(4-Fluorphenyl)-2-phenylethyl]piperidin;
(±)-1-(2,2-Dimethyl-1,2-diphenylethyl)piperidin;
(±)-1-[1-(3-Methylphenyl)-2-phenylethyl]piperidin;
(±)-1-[1-(2-Furyl)-2-phenylethyl]piperidin;
(±)-1-[1-(2-Chlorphenyl)-2-phenylethyl]piperidin;
(±)-1-[1-(2-Methoxyphenyl)-2-phenylethyl]piperidin und
(±)-1-[1-(1-Naphthyl)-2-phenylethyl]piperidin.

**Patentansprüche für folgenden Vertragsstaat : GR**

**1.** Verbindung der Formel

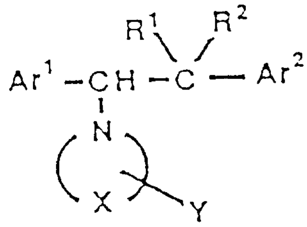

worin jedes von $R^1$ und $R^2$ eine Gruppe ist, die unabhängig aus Hydrido, $C_1$-$C_{20}$ linearem oder

verzweigtem Alkyl, $C_3$-$C_{10}$-Cycloalkyl, Hydroxyl, Halogen und $C_1$-$C_{10}$-Alkoxy ausgewählt ist, oder worin $R^1$ und $R^2$ zusammengefaßt werden können, um eine gesättigte oder teilweise ungesättigte $C_3$-$C_{10}$-Cycloalkyl-carbocyclische Gruppe mit drei bis acht Ringkohlenstoffatomen zu bilden;
worin jedes von $Ar^1$ und $Ar^2$ eine Gruppe ist, die unabhängig aus Phenyl-, Thienyl-, Furanyl-, Pyridinyl-, Thiazolyl- und Naphthylgruppen ausgewählt ist und worin jede der genannten $Ar^1$- und $Ar^2$-Gruppen mit einem oder mehreren Resten substituiert sein kann, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, $C_2$-$C_{20}$-Alkenyl, $C_2$-$C_{20}$-Alkinyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin X aus CH oder $CH_2$ ausgewählt ist, um einen fünf- bis siebengliedrigen Ring zu bilden, vorausgesetzt, daß der Ring gesättigt ist oder eine Doppelbindung enthält; und worin Y eine aus Hydrido, $C_1$-$C_{20}$-Alkyl, Halogen, Hydroxyl, $C_1$-$C_{10}$-Alkoxy, Amino, Cyano, Nitro und Mercapto ausgewählte Gruppe ist;
mit der Maßgabe, daß, wenn $Ar^1$ und $Ar^2$ beide Phenyl sind und $R^1$ und $R^2$ Hydrido sind oder $R^1$ H und $R^2$ Hydroxy ist, X nicht eine lineare Alkylenkette mit vier oder fünf Kohlenstoffatomen sein kann, um ein racemisches Gemisch zu bilden;
mit der weiteren Maßgabe, daß, wenn $Ar^1$ para-Methylphenyl oder para-Methoxyphenyl ist und $R^1$ und $R^2$ Hydrido sind und $Ar^2$ Phenyl ist, X nicht eine lineare Alkylenkette mit fünf Kohlenstoffatomen sein kann,
mit Ausnahme der Verbindungen, worin $Ar^1$ Phenyl ist, $R^2$ H ist und X $CH_2$ ist, um einen 6-gliedrigen Ring zu bilden, und gleichzeitig a) $Ar^2$ Phenyl, $R^1$ F oder OH, b) $Ar^2$ Furanyl und $R^1$ OH ist,
und worin $Ar^1$, $Ar^2$ 2, 3 oder 4 $NO_2$-Phenyl ist, $R^2$ H, $R^1$ OH ist und X $CH_2$ ist, um einen 6-gliedrigen Ring zu bilden;
oder worin $R^1$, $R^2$ H sind, X $CH_2$ ist, um einen 6-gliedrigen Ring zu bilden, und $Ar^1$ Naphthyl ist, $Ar^2$ Phenyl ist, oder $Ar^1$ Thienyl und $Ar^2$ Phenyl ist,
und worin $Ar^1$ Methoxyphenyl ist, X $CH_2$ ist, um einen 5-gliedrigen Ring zu bilden, und gleichzeitig $Ar^2$ Methoxyphenyl ist, $R^1$ = H, $R^2$ = OH oder $R^1$, $R^2$ = H.

2. Verbindung nach Anspruch 1 mit der Formel

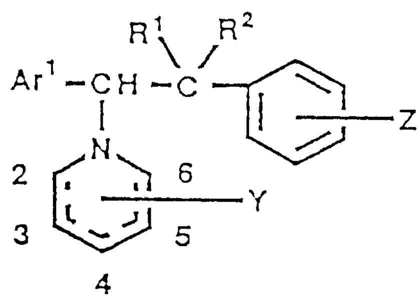

worin jedes von $R^1$ und $R^2$ eine Gruppe ist, die unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählt ist, oder worin $R^1$ und $R^2$ zusammengefaßt werden können, um eine gesättigte oder teilweise ungesättigte carbocyclische Gruppe mit drei oder sieben Ringkohlenstoffatomen zu bilden; worin $Ar^1$ eine aus Phenyl-, Thienyl-, Furanyl-, Pyridinyl-, Thiazolyl- und Naphthylgruppen ausgewählte Gruppe ist und jede der genannten $Ar^1$-Gruppen, die eine substituierbare Position aufweist, mit einem oder mehreren Resten substituiert sein kann, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$-Alkyl, Halogen, Hydroxyl, Alkoxy und Amino ausgewählte Gruppe ist; und worin Z eine oder mehrere Gruppen bedeutet, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind.

3. Verbindung nach Anspruch 2, worin jedes von $R^1$ und $R^2$ eine unabhängig aus Hydrido, $C_1$-$C_{20}$ linearem oder verzweigtem Alkyl, $C_3$-$C_{10}$-Cycloalkyl, Hydroxyl, Halogen, $C_1$-$C_{10}$-Alkoxy ausgewählte Gruppe ist, oder worin $R^1$ und $R^2$ zusammengefaßt werden können, um eine gesättigte oder teilweise ungesättigte carbocyclische Gruppe mit drei oder sieben Ringkohlenstoffatomen zu bilden; worin $Ar^1$

aus Phenyl-, Thienyl-, Pyridinyl- und Naphthylgruppen ausgewählt ist und worin jede der genannten $Ar^1$-Gruppen, die eine substituierbare Position aufweist, mit einem oder mehreren Resten substituiert sein kann, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; und worin Z eine oder mehrere Gruppen bedeutet, die aus Hydrido, $C_1$-$C_{20}$ linearem oder verzweigtem Alkyl, $C_3$-$C_{10}$-Cycloalkyl, Halogen, Hydroxyl, $C_1$-$C_{10}$-Alkoxy und Amino ausgewählt sind.

**4.** Verbindung nach Anspruch 3, worin jedes von $R^1$ und $R^2$ eine Gruppe ist, die unabhängig aus Hydrido, $C_1$-$C_{20}$ linearem oder verzweigtem Alkyl und Hydroxyl ausgewählt ist, oder worin $R^1$ und $R^2$ zusammengefaßt werden können, um eine gesättigte oder teilweise ungesättigte carbocyclische Gruppe mit drei bis sechs Ringkohlenstoffatomen zu bilden; worin $Ar^1$ eine aus Phenyl-, Thienyl-, Pyridinyl- und Naphthylgruppen ausgewählte Gruppe ist, und worin jede der genannten $Ar^1$-Gruppen, die eine oder mehrere substituierbare Positionen aufweist, mit einem oder mehreren Resten substituiert sein kann, die aus Hydrido, $C_1$-$C_{20}$ linearem oder verzweigtem Alkyl, Halogen, $C_1$-$C_{20}$-Halogenalkyl, Hydroxyl, $C_1$-$C_{10}$-Alkoxy, Amino und Nitro ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; und worin Z eine oder mehrere Gruppen bedeutet, die aus Hydrido, $C_1$-$C_{20}$ linearem oder verzweigtem Alkyl, Halogen, Hydroxyl und $C_1$-$C_{10}$-Alkoxy ausgewählt sind.

**5.** Verbindung nach Anspruch 1 mit der Formel

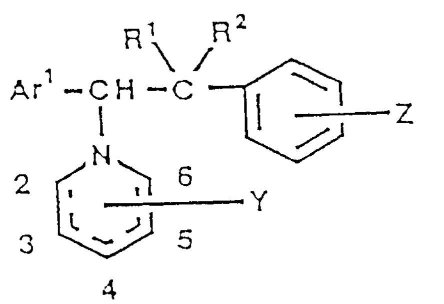

worin jedes von $R^1$ und $R^2$ eine Gruppe darstellt, die unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählt ist, oder worin $R^1$ und $R^2$ zusammengefaßt werden können, um eine gesättigte oder teilweise ungesättigte carbocyclische Gruppe mit drei bis sieben Ringkohlenstoffatomen zu bilden; worin $Ar^1$ eine aus Phenyl-, Thienyl-, Furanyl-, Pyridinyl-, Thiazolyl- und Naphthylgruppen ausgewählte Gruppe ist und worin jede der genannten $Ar^1$-Gruppen, die eine substituierbare Position aufweist, mit einem oder mehreren Resten substituiert ist, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere Gruppen bedeutet, die aus $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; und worin die strichlierte Linie im N-haltigen Ring eine Doppelbindung zwischen irgendwelchen zwei benachbarten Kohlenstoffatomen darstellt, wobei die 2-, 3-, 4-, 5- und 6-Position des N-haltigen Rings involviert ist.

**6.** Verbindung nach Anspruch 5, worin jedes von $R^1$ und $R^2$ eine unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählte Gruppe darstellt, oder worin $R^1$ und $R^2$ zusammengefaßt werden können, um eine gesättigte oder teilweise ungesättigte carbocyclische Gruppe mit drei bis sieben Ringkohlenstoffatomen zu bilden; worin $Ar^1$ eine aus Phenyl-, Thienyl-, Furanyl-, Pyridinyl-, Thiazolyl- und Naphthylgruppen ausgewählte Gruppe ist und worin jede der genannten Gruppen, die eine oder mehrere substituierbare Positionen aufweist, mit einem oder mehreren Resten substituiert sein kann, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxy-

alkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere Gruppen bedeutet, die aus Hydrido, $C_1$-$C_{20}$ linearem oder verzweigtem Alkyl, $C_3$-$C_{10}$-Cycloalkyl, Halogen, Hydroxyl, $C_1$-$C_{10}$-Alkoxy und Amino ausgewählt sind; und worin die strichlierte Linie innerhalb des N-haltigen Rings eine Doppelbindung zwischen irgendwelchen zwei benachbarten Kohlenstoffatomen bedeutet, wobei die 3-, 4- und 5-Position des N-haltigen Rings involviert ist.

**7.** Verbindung nach Anspruch 6, worin jedes von $R^1$ und $R^2$ eine unabhängig aus Hydrido, $C_1$-$C_{20}$ linearem oder verzweigtem Alkyl, $C_3$-$C_{10}$-Cycloalkyl, Hydroxyl, Halogen und $C_1$-$C_{10}$-Alkoxy ausgewählte Gruppe ist; worin $R^1$ und $R^2$ zusammengefaßt werden können, um ein gesättigtes oder teilweise ungesättigtes Cycloalkyl mit drei bis sieben Ringkohlenstoffatomen zu bilden; worin $Ar^1$ aus Phenyl-, Thienyl-, Pyridinyl- und Naphthylgruppen ausgewählt ist und worin jede der genannten Gruppen, die eine substituierbare Position aufweist, mit einem oder mehreren Resten substituiert ist, die aus Hydrido, $C_1$-$C_{20}$ linearem oder verzweigtem Alkyl, $C_3$-$C_{10}$-Cycloalkyl, Halogen, $C_1$-$C_{20}$-Halogenalkyl, Hydroxyl, $C_1$-$C_{10}$-Hydroxyalkyl, $C_1$-$C_{10}$-Alkoxy, $C_1$-$C_{20}$-Alkoxyalkyl, Amino, Cyano, Nitro und Mercapto ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere aus Hydrido, $C_1$-$C_{20}$ linearem oder verzweigtem Alkyl, $C_3$-$C_{10}$-Cycloalkyl, Halogen, Hydroxyl, $C_1$-$C_{10}$-Alkoxy und Amino ausgewählte Gruppen bedeutet; und worin die strichlierte Linie innerhalb des N-haltigen Rings jeweils eine Doppelbindung zwischen irgendwelchen zwei benachbarten Kohlenstoffatomen bedeutet, wobei die 3-, 4- und 5-Position des N-haltigen Rings involviert ist.

**8.** Verbindung nach Anspruch 7, worin jedes von $R^1$ und $R^2$ eine unabhängig aus Hydrido, $C_1$-$C_{20}$ linearem oder verzweigtem Alkyl und Hydroxyl ausgewählte Gruppe ist; worin $R^1$ und $R^2$ zusammengefaßt werden können, um ein gesättigtes oder teilweise ungesättigtes Cycloalkyl mit drei bis sechs Ringkohlenstoffatomen zu bilden; worin $Ar^1$ aus Phenyl-, Thienyl-, Pyridinyl- und Naphthylgruppen ausgewählt ist und worin jede der genannten Gruppen, die eine oder mehrere substituierbare Positionen aufweist, mit einem oder mehreren Resten substituiert ist, die aus Hydrido, $C_1$-$C_{20}$ linearem oder verzweigtem Alkyl, Halogen, $C_1$-$C_{20}$-Halogenalkyl, Hydroxyl, $C_1$-$C_{10}$-Alkoxy, Amino und Nitro ausgewählt sind; worin Y eine aus Hydrido, $C_1$-$C_{20}$ linearem oder verzweigtem Alkyl, Halogen, Hydroxyl, $C_1$-$C_{10}$-Alkoxy und Amino ausgewählte Gruppe ist; worin Z eine oder mehrere Gruppen bedeutet, die aus Hydrido, $C_1$-$C_{20}$ linearem oder verzweigtem Alkyl, Halogen, Hydroxyl und $C_1$-$C_{10}$-Alkoxy ausgewählt sind; und worin die strichlierte Linie innerhalb des N-haltigen Rings jeweils eine Doppelbindung zwischen irgendwelchen zwei benachbarten Kohlenstoffatomen darstellt, wobei die 3-, 4- und 5-Position des N-haltigen Rings involviert ist.

**9.** Verbindung nach Anspruch 1, ausgewählt aus der Gruppe bestehend aus
(-)-1-(1,2-Diphenylethyl)piperidin;
(+)-1-(1,2-Diphenylethyl)piperidin;
(±)-1-[1-Phenyl-2-(2-pyridinyl)ethyl]piperidin;
1-[1-Phenyl-2-(2-pyridinyl)ethenyl]piperidin;
(±)-1-(1,2-Diphenylethyl)-1H-azepin;
(±)-1-(1,2-Diphenylbutyl)piperidin;
(±)-1-(1,2-Diphenyl-3-methylbutyl)piperidin;
(±)-1-[1-(1-Phenylcyclopentyl)benzyl]piperidin;
(±)-1-[1-(1-Phenylcyclohexyl)benzyl]piperidin;
(±)-1-[1-(1-Phenylcyclopropyl)benzyl]piperidin;
(±)-1-[2,2-Dimethyl-2-phenyl-1-(2-thienyl)ethyl]piperidin;
(±)-1-[1-(3-Methoxyphenyl)-2-phenylethyl]piperidin;
(±)-1-[1-(4-Fluorphenyl)-2-phenylethyl]piperidin;
(±)-1-(2,2-Dimethyl-1,2-diphenylethyl)piperidin;
(±)-1-[1-(3-Methylphenyl)-2-phenylethyl]piperidin;
(±)-1-[1-(2-Furyl)-2-phenylethyl]piperidin;
(±)-1-[1-(2-Chlorphenyl)-2-phenylethyl]piperidin;
(±)-1-[1-(2-Methoxyphenyl)-2-phenylethyl]piperidin;
(±)-1-(1,2-Diphenylethyl)-1,2,3,6-tetrahydropyridin und
(±)-1-[2-Phenyl-1-(2-thienyl)ethyl]-1,2,3,6-tetrahydropyridin.

**10.** Verbindung nach Anspruch 9, ausgewählt aus der Gruppe bestehend aus
(-)-1-(1,2-Diphenylethyl)piperidin;
( + )-1-(1,2-Diphenylethyl)piperidin;
(±)-1-[1-Phenyl-2-(2-pyridinyl)ethyl]piperidin;
(±)-1-(1,2-Diphenylethyl)-1H-azepin;
(±)-1-(1,2-Diphenylbutyl)piperidin;
(±)-1-[1-(1-Phenylcyclohexyl)benzyl]piperidin;
(±)-1-[1-(1-Phenylcyclopropyl)benzyl]piperidin;
(±)-1-[1-(3-Methoxyphenyl)-2-phenylethyl]piperidin;
(±)-1-[1-(4-Fluorphenyl)-2-phenylethyl]piperidin;
(±)-1-(2,2-Dimethyl-1,2-diphenylethyl)piperidin;
(±)-1-[1-(3-Methylphenyl)-2-phenylethyl]piperidin;
(±)-1-[1-(2-Chlorphenyl)-2-phenylethyl]piperidin und
(±)-1-[1-(2-Methoxyphenyl)-2-phenylethyl]piperidin.

**11.** Verbindung nach Anspruch 10, ausgewählt aus der Gruppe bestehend aus
(±)-1-[1-(3-Methoxyphenyl)-2-phenylethyl]piperidin;
(±)-1-[1-(4-Fluorphenyl)-2-phenylethyl]piperidin;
(±)-1-[1-(3-Methylphenyl)-2-phenylethyl]piperidin;
(±)-1-[1-(2-Chlorphenyl)-2-phenylethyl]piperidin und
(±)-1-[1-(2-Methoxyphenyl)-2-phenylethyl]piperidin.

**12.** Verbindung nach Anspruch 11, die (±)-1-[1-(2-Chlorphenyl)-2-phenylethyl]piperidin ist.

**13.** Verbindung nach Anspruch 9, ausgewählt aus der Gruppe bestehend aus (±)-1-(1,2-Diphenylethyl)-1,2,3,6-tetrahydropyridin und (±)-1-[2-Phenyl-1-(2-thienyl)ethyl]-1,2,3,6-tetrahydropyridin.

**14.** Verbindung nach Anspruch 13, die (±)-1-[2-Phenyl-1-(2-thienyl)ethyl]-1,2,3,6-tetrahydropyridin ist.

**15.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 1 ausgewählt ist.

**16.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 2 ausgewählt ist.

**17.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 3 ausgewählt ist.

**18.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 4 ausgewählt ist.

**19.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 5 ausgewählt ist.

**20.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 6 ausgewählt ist.

**21.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 7 ausgewählt ist.

**22.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 8 ausgewählt ist.

**23.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 9 ausgewählt ist.

**24.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 10 ausgewählt ist.

**25.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 11 ausgewählt ist.

**26.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 12 ausgewählt ist.

**27.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 13 ausgewählt ist.

**28.** Verfahren zur Herstellung einer pharmazeutischen Zusammensetzung durch Mischen eines pharmazeutisch unbedenklichen Trägers oder Verdünnungsmittels und einer therapeutisch wirksamen Menge eines Wirkstoffes, der aus einer Familie von Verbindungen nach Anspruch 14 ausgewählt ist.

**29.** Verwendung einer Verbindung nach Anspruch 1 zur Herstellung eines Arzneimittels zur Beherrschung neuropathologischer Prozesse und deren neurodegenerativer Folgen bei Säugern.

**30.** Verwendung einer Verbindung nach Anspruch 2 bis 14 zur Herstellung eines Arzneimittels zur Beherrschung neuropathologischer Prozesse und deren neurodegenerativer Folgen bei Säugern.

**31.** Verwendung nach Anspruch 29 zur Herstellung eines Arzneimittels zur Behandlung von Hypoxie, Anoxie oder Ischämie.

**32.** Verwendung nach Anspruch 31 zur Herstellung eines Arzneimittels zur Behandlung von Ischämie.

**33.** Verwendung einer Verbindung, wie in den Ansprüchen 29-32 definiert, ausgewählt aus der Gruppe bestehend aus
(±)-1-(1,2-Diphenylethyl)piperidin;
(-)-1-(1,2-Diphenylethyl)piperidin;
(+)-1-(1,2-Diphenylethyl)piperidin;
(±)-1-[2-Phenyl-1-(2-thienyl)ethyl]piperidin-hydrosulfat;
(±)-1-(1,2-Diphenylethyl)-1,2,3,6-tetrahydropyridin;
1-(1,2-Diphenylethenyl)piperidin;
1-[1-Phenyl-2-(2-pyridinyl)ethenyl]piperidin;
(±)-1-[1-Phenyl-2-(2-pyridinyl)ethyl]piperidin;
(±)-1-(1,2-Diphenylethyl)-1H-azepin;
(±)-1-(1,2-Diphenylbutyl)piperidin;
(±)-1-(1,2-Diphenyl-3-methylbutyl)piperidin;
(±)-1-(1,2-Diphenyl-1-hydroxyethyl)piperidin;
(±)-1-[2-Phenyl-1-(2-thienyl)ethyl]-1,2,3,6-tetrahydropyridin;
(±)-1-[1-(1-Phenylcyclopentyl)benzyl]piperidin;
(±)-1-[1-(1-Phenylcyclohexyl)benzyl]piperidin;
(±)-1-(1,2-Diphenylethyl)pyrrolidin;
(±)-1-[1-(4-Methylphenyl)-2-phenylethyl]piperidin;

(±)-1-[1-(4-Methoxyphenyl)-2-phenylethyl]piperidin;
(±)-1-[1-(1-Phenylcyclopropyl)benzyl]piperidin;
(±)-1-[2,2-Dimethyl-2-phenyl-1-(2-thienyl)ethyl]piperidin;
(±)-1-[1-(3-Methoxyphenyl)-2-phenylethyl]piperidin;
(±)-1-[1-(4-Fluorphenyl)-2-phenylethyl]piperidin;
(±)-1-(2,2-Dimethyl-1,2-diphenylethyl)piperidin;
(±)-1-[1-(3-Methylphenyl)-2-phenylethyl]piperidin;
(±)-1-[1-(2-Furyl)-2-phenylethyl]piperidin;
(±)-1-[1-(2-Chlorphenyl)-2-phenylethyl]piperidin;
(±)-1-[1-(2-Methoxyphenyl)-2-phenylethyl]piperidin und
(±)-1-[1-(1-Naphthyl)-2-phenylethyl]piperidin.

**Revendications**

**Revendications pour les Etats contractants suivants : AT, BE, FR, GB, DE, LU, NL, IT, SE, CH, LI**

**1.** Un composé de formule

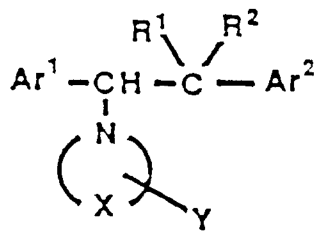

dans laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy ou bien dans laquelle $R^1$ et $R^2$ peuvent être pris ensemble pour former un groupe $C_3$-$C_{10}$ cycloalkyle carbocyclique saturé ou partiellement insaturé ayant 3 à 8 atomes de carbone dans le noyau ;
dans laquelle chacun des $Ar^1$ et $Ar^2$ est un groupe choisi indépendamment parmi les groupes phényle, thiényle, furannyle, pyridinyle, thiazolyle et naphtyle, et dans laquelle chacun des groupes $Ar^1$ et $Ar^2$ précédents peut être substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ haloalkyle, $C_2$-$C_{20}$ alcényle, $C_2$-$C_{20}$ alcynyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle X est choisi parmi CH ou $CH_2$ pour former un noyau ayant 5 à 7 chaînons pourvu que ce noyau soit saturé ou contienne une double liaison ; et dans laquelle Y est un groupe choisi parmi les groupes hydrido, $C_1$-$C_{20}$ alkyle, halo, hydroxyle, $C_1$-$C_{10}$ alcoxy, amino, cyano, nitro et mercapto;
à la condition que lorsque chacun des $Ar^1$ et $Ar^2$ est un groupe phényle et chacun des $R^1$ et $R^2$ est un groupe hydrido, ou bien $R^1$ est H et $R^2$ est hydroxy, alors X ne peut pas être une chaîne alkylène linéaire ayant 4 ou 5 atomes de carbone de façon à former un mélange racémique ; avec en outre la condition que lorsque $A^1$ est un groupe paraméthylphényle ou paraméthoxyphényle et chacun des $R^1$ et $R^2$ est un groupe hydrido et $AR^2$ est un groupe phényle, alors X ne peut pas être une chaîne alkylène linéaire ayant 5 atomes de carbone,
à l'exception des composés dans lesquels :
$Ar^1$ est un groupe phényle, $R^2$ est H et X est $CH_2$ pour former un noyau à 6 chaînons et en même temps
a) $Ar^2$ est un groupe phényle, $R^1$ est F ou OH et
b) $Ar^2$ est un groupe furannyle et $R^1$ est OH et dans laquelle
$Ar^1$, $Ar^2$ est 2, 3 ou 4 $NO_2$-phényle, $R^2$ est H, $R^1$ OH et X est $CH_2$ pour former un noyau à 6 chaînons ; ou bien dans laquelle $R^1$, $R^2$ est H, X est $CH_2$ pour former un noyau à 6 chaînons et $Ar^1$ est un groupe naphtyle, $Ar^2$ est un groupe phényle ou $Ar^1$ est un groupe thiényle et $Ar^2$ est un groupe phényle et dans laquelle
$Ar^1$ est un groupe méthoxyphényle, X est $CH_2$ pour former un noyau à 5 chaînons et en même temps $Ar^2$ est un groupe méthoxyphényle, $R^1$ = H, $R^2$ = OH ou bien $R^1$, $R^2$ = H.

**2.** Composé selon la revendication 1 de formule :

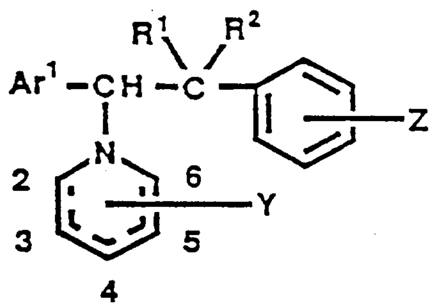

dans laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe carbocyclique saturé ou partiellement insaturé ayant 3 ou 7 atomes de carbone dans le noyau ;
dans laquelle $Ar^1$ est un groupe choisi parmi les groupes phényle, thiényle, furannyle, pyridinyle, thiazolyle et naphtyle, et dans laquelle chacun des groupes $Ar^1$ précédents ayant une position substituable peut être substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ haloalkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle Y est un groupe choisi parmi les groupes hydrido, $C_1$-$C_{20}$ alkyle, halo, hydroxyle, alcoxy et amino ; et dans laquelle Z est un ou plusieurs groupes choisis parmi les groupes hydrido, $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxyalkyle, amino, cyano, nitro et mercapto.

**3.** Composé selon la revendication 2, selon laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo, $C_1$-$C_{10}$ alcoxy, ou dans laquelle $R^1$ et $R^2$ peuvent être pris ensemble pour former un groupe carbocyclique saturé ou partiellement insaturé ayant 3 ou 7 atomes de carbone dans le noyau ; dans laquelle $Ar^1$ est choisi parmi les groupes phényle, thiényle, pyridinyle, et naphtyle, et dans laquelle chacun des groupes $Ar^1$ précédents ayant une position substituable peut être substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ haloalkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un plusieurs groupes choisis parmi les groupes hydrido, $C_1$-$C_{20}$ alkyle linéaire ou ramifié, $C_3$-$C_{10}$ cycloalkyle, halo, hydroxyle, $C_1$-$C_{10}$ alcoxy et amino.

**4.** Composé selon la revendication 3, selon laquelle chacun des $R^1$ et $R^2$ est un groupe choisi indépendamment parmi les groupes hydrido, $C_1$-$C_{20}$ alkyle linéaire ou ramifié et hydroxyle ou dans laquelle $R^1$ et $R^2$ peuvent être pris ensemble pour former un groupe carbocyclique saturé ou partiellement insaturé ayant 3 ou 6 atomes de carbone dans le noyau ;
dans laquelle $Ar^1$ est un groupe choisi parmi les groupes phényle, thiényle, pyridinyle et naphtyle et dans laquelle chacun des groupes $Ar^1$ précédents ayant une position ou plusieurs positions substituables peut être substitué par un ou plusieurs radicaux choisis parmi les groupes hydrido, $C_1$-$C_{20}$ alkyle linéaire ou ramifié, halo, $C_1$-$C_{20}$ halo alkyle, hydroxyle, $C_1$-$C_{10}$ alcoxy, $C_1$-$C_{20}$ alcoxy amino et nitro ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes hydrido, $C_1$-$C_{20}$ alkyle linéaire ou ramifié, halo, hydroxyle et $C_1$-$C_{10}$ alcoxy.

**5.** Composé selon la revendication 1 de formule:

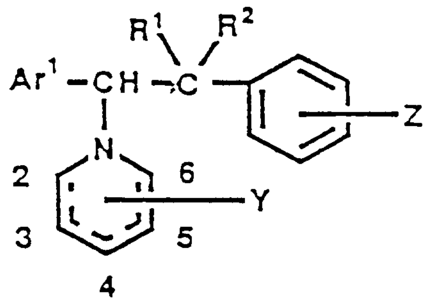

dans laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe carbocyclique saturé ou partiellement insaturé ayant 3 ou 7 atomes de carbone dans le noyau ;
dans laquelle $Ar^1$ est un groupe choisi parmi les groupes phényle, thiényle, furannyle, pyridinyle, thiazolyle et naphtyle ; et dans laquelle chacun des groupes $Ar^1$ précités ayant une position substituable est substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, alcoxy alkyle, amino, cyano, nitro et mercapto ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; et dans laquelle le trait discontinu dans le noyau contenant N représente une double liaison entre deux atomes de carbone adjacents quelconques mettant en oeuvre les positions 2-, 3-, 4-, 5- et 6- du noyau contenant N.

**6.** Composé selon la revendication 5 selon laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe carbocyclique saturé ou partiellement insaturé ayant 3 ou 7 atomes de carbone dans le noyau ;
dans laquelle $Ar^1$ est un groupe choisi parmi les groupes phényle, thiényle, furannyle, pyridinyle, thiazolyle et naphtyle, et dans laquelle chacun des groupes précédents ayant une ou plusieurs positions substituables peut être substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes hydrido, $C_1$-$C_{20}$ alkyle linéaire ou ramifié, $C_3$-$C_{10}$ cycloalkyle, halo, hydroxyle, $C_1$-$C_{10}$ alcoxy et amino ; et dans laquelle le trait discontinu dans le noyau contenant N représente une double liaison entre 2 atomes de carbone adjacents quelconques mettant en oeuvre les positions 3-, 4-, 5- du noyau contenant N.

**7.** Composé selon la revendication 6, selon laquelle chacun des $R^1$ et $R^2$ est un groupe choisi indépendamment parmi les groupes hydrido, $C_1$-$C_{20}$ alkyle linéaire ou substitué, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe cycloalkyle saturé ou partiellement insaturé ayant 3 ou 7 atomes de carbone dans le noyau ;
dans laquelle $Ar^1$ est choisi parmi les groupes phényle, thiényle, pyridinyle et naphtyle, et dans laquelle chacun des groupes précédents ayant une position substituable substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes hydrido, $C_1$-$C_{20}$ alkyle linéaire ou ramifié, $C_3$-$C_{10}$ cycloalkyle, halo, hydroxyle, $C_1$-$C_{10}$ alcoxy et amino ; et dans laquelle le trait discontinu dans le noyau contenant N représente une double liaison entre 2 atomes de carbone adjacents quelconques mettant en oeuvre les positions

3-, 4-, 5- du noyau contenant N.

**8.** Composé selon la revendication 7, selon laquelle chacun des $R^1$ et $R^2$ est un groupe choisi indépendamment parmi les groupes hydrido, $C_1$-$C_{20}$ alkyle linéaire ou ramifié et hydroxyle ; dans laquelle $R^1$ et $R^2$ peuvent être pris ensemble pour former un groupe cycloalkyle saturé ou partiellement insaturé ayant 3 ou 6 atomes de carbone dans le noyau ;
dans laquelle $Ar^1$ est choisi parmi les groupes phényle, thiényle, pyridinyle et naphtyle, et dans laquelle chacun des groupes précédents ayant une ou plusieurs positions substituables est substitué par un ou plusieurs radicaux choisis parmi les groupes hydrido, $C_1$-$C_{20}$ alkyle linéaire ou ramifié, halo, $C_1$-$C_{20}$ halo alkyle, hydroxyle, $C_1$-$C_{10}$ alcoxy, amino et nitro ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes hydrido, $C_1$-$C_{20}$ alkyle linéaire ou ramifié, halo, hydroxyle et $C_1$-$C_{10}$ alcoxy ; et dans laquelle le trait discontinu à l'intérieur des noyaux contenant N représente une double liaison entre 2 atomes de carbone adjacents quelconques mettant en oeuvre les positions 3-, 4-, 5- du noyau contenant N.

**9.** Composé selon la revendication 1 choisi dans le groupe constitué de
(-)-1-(1,2-diphényléthyl)pipéridine ;
(+)-1-(1,2-diphényléthyl)pipéridine ;
(±)-1-[1-phényl-2-(2-pyridinyl)éthyl]pipéridine ;
1-[1-phényl-2-(2-pyridinyl)éthényl]pipéridine ;
(±)-1-(1,2-diphényléthyl)-1H-azépine ;
(±)-1-(1,2-diphénylbutyl)pipéridine ;
(±)-1-(1,2-diphényl-3-méthylbutyl)pipéridine ;
(±)-1-[1-(1-phénylcyclopentyl)benzyl]pipéridine ;
(±)-1-[1-(1-phénylcyclohexyl)benzyl]pipéridine;
(±)-1-[1-(1-phénylcyclopropyl)benzyl]pipéridine ;
(±)-1-[2,2-diméthyl-2-phényl-1-(2-thiényl)éthyl]pipéridine ;
(±)-1-[1-(3-méthoxyphényl)-2-phényléthyl]pipéridine ;
(±)-1-[1-(4-florophényl)-2-phényléthyl]pipéridine ;
(±)-1-(2,2-diméthyl-1,2-diphényléthyl)pipéridine ;
(±)-1-[1-(3-méthylphényl)-2-phényléthyl]pipéridine ;
(±)-1-[1-(2-furyl)-2-phényléthyl]pipéridine ;
(±)-1-[1-(2-chlorophényl)-2-phényléthyl]pipéridine ;
(±)-1-[1-(2-méthoxyphényl)-2-phényléthyl]pipéridine ;
(±)-1-(1,2-diphényléthyl)-1,2,3,6-tétrahydropyridine ; et
(±)-1-[2-phényl-1-(2-thiényl)éthyl]-1,2,3,6-tétrahydropyridine.

**10.** Composé selon la revendication 9, choisi dans le groupe constitué de :
(-)-1-(1,2-diphényléthyl)pipéridine ;
(+)-1-(1,2-diphényléthyl)pipéridine ;
(±)-1-[1-phényl-2-(2-pyridinyl)éthyl]pipéridine ;
(±)-1-(1,2-diphényléthyl)-1H-azépine ;
(±)-1-(1,2-diphénylbutyl)pipéridine ;
(±)-1-[1-(1-phénylcyclohexyl)benzyl]pipéridine ;
(±)-1-[1-(1-phénylcyclopropyl)benzyl]pipéridine ;
(±)-1-[1-(3-méthoxyphényl)-2-phényléthyl]pipéridine ;
(±)-1-[1-(4-fluorophényl)-2-phényléthyl]pipéridine ;
(±)-1-(2,2-diméthyl-1,2-diphényléthyl)pipéridine ;
(±)-1-(1-(3-méthylphényl)-2-phényléthyl)pipéridine ;
(±)-1-[1-(2-chlorophényl)-2-phényléthyl]pipéridine ; et
(±)-1-[1-(2-méthoxyphényl)-2-phényléthyl]pipéridine.

**11.** Composé selon la revendication 10 choisi dans le groupe constitué de :
(±)-1-[1-(3-méthoxyphényl)-1-phényléthyl]pipéridine ;
(±)-1-[1-(4-fluorophényl)-2-phényléthyl]pipéridine ;
(±)-1-[1-(3-méthylphényl)-2-phényléthyl]pipéridine ;
(±)-1-[1-(2-chlorophényl)-2-phényléthyl]pipéridine ; et

(±)-1-[1-(2-méthoxyphényl)-2-phényléthyl]pipéridine.

**12.** Composé selon la revendication 11 qui est la
(±)-1-[1-(2-chlorophényl)-2-phényléthyl]pipéridine.

**13.** Composé selon la revendication 9 choisi dans le groupe constitué de :
(±)-1-(1,2-diphényléthyl)-1,2,3,6-tétrahydropyridine ; et
(±)-1-[2-phényl-1-(2-thiényl)éthyl]-1,2,3,6-tétrahydropyridine.

**14.** Composé selon la revendication 13 qui est la
(±)-1-[2-phényl-1-(2-thiényl)éthyl]-1,2,3,6-tétrahydropyridine.

**15.** Une composition pharmaceutique comprenant une quantité thérapeutique efficace d'un composé actif et d'un support ou diluant pharmaceutiquement acceptable, ledit composé actif étant choisi dans une famille de composés selon la revendication 1.

**16.** Une composition pharmaceutique comprenant une quantité thérapeutique active d'un composé actif et un support ou diluant pharmaceutiquement acceptable, ledit composé actif étant choisi parmi un composé selon la revendication 2.

**17.** Une composition pharmaceutique comprenant une quantité thérapeutique efficace d'un composé actif et d'un support ou diluant pharmaceutiquement acceptable, ledit composé actif étant sélectionné parmi un composé selon la revendication 3.

**18.** Une composition pharmaceutique comprenant une quantité thérapeutique efficace d'un composé actif et d'un support ou diluant pharmaceutiquement acceptable, ledit composé actif étant sélectionné parmi un composé selon la revendication 4.

**19.** Une composition pharmaceutique comprenant une quantité thérapeutique efficace d'un composé actif et d'un support ou diluant pharmaceutiquement acceptable, ledit composé actif étant sélectionné parmi un composé selon la revendication 5.

**20.** Une composition pharmaceutique comprenant une quantité thérapeutique efficace d'un composé actif et d'un support ou diluant pharmaceutiquement acceptable, ledit composé actif étant sélectionné parmi un composé selon la revendication 6.

**21.** Une composition pharmaceutique comprenant une quantité thérapeutique efficace d'un composé actif et d'un support ou diluant pharmaceutiquement acceptable, ledit composé actif étant sélectionné parmi un composé selon la revendication 7.

**22.** Une composition pharmaceutique comprenant une quantité thérapeutique efficace d'un composé actif et d'un support ou diluant pharmaceutiquement acceptable, ledit composé actif étant sélectionné parmi un composé selon la revendication 8.

**23.** Une composition pharmaceutique comprenant une quantité thérapeutique efficace d'un composé actif et d'un support ou diluant pharmaceutiquement acceptable, ledit composé actif étant sélectionné parmi un composé selon la revendication 9.

**24.** Une composition pharmaceutique comprenant une quantité thérapeutique efficace d'un composé actif et d'un support ou diluant pharmaceutiquement acceptable, ledit composé actif étant sélectionné parmi un composé selon la revendication 10.

**25.** Une composition pharmaceutique comprenant une quantité thérapeutique efficace d'un composé actif et d'un support ou diluant pharmaceutiquement acceptable, ledit composé actif étant sélectionné parmi un composé selon la revendication 11.

**26.** Une composition pharmaceutique comprenant une quantité thérapeutique efficace d'un composé actif et d'un support ou diluant pharmaceutiquement acceptable, ledit composé actif étant sélectionné parmi

un composé selon la revendication 12.

**27.** Une composition pharmaceutique comprenant une quantité thérapeutique efficace d'un composé actif et d'un support ou diluant pharmaceutiquement acceptable, ledit composé actif étant sélectionné parmi un composé selon la revendication 13.

**28.** Une composition pharmaceutique comprenant une quantité thérapeutique efficace d'un composé actif et d'un support ou diluant pharmaceutiquement acceptable, ledit composé actif étant sélectionné parmi un composé selon la revendication 14.

**29.** Utilisation d'un composé selon la revendication 1 pour la préparation d'un médicament pour contrôler les processus neuropathologiques et les conséquences neurodégénératives chez les mammifères.

**30.** Utilisation d'un composé selon l'une quelconque des revendications 2 à 14 pour la préparation d'un médicament pour contrôler les processus neuropathologiques et les conséquences neurodégénératives chez les mammifères.

**31.** Utilisation selon la revendication 29 pour la préparation d'un médicament pour le traitement de l'hypoxie, de l'anoxie et de l'ischémie.

**32.** Utilisation selon la revendication 31 pour la préparation d'un médicament pour le traitement de l'ischémie.

**33.** Utilisation d'un composé tel que défini dans les revendications 29-32 choisis dans le groupe constitué de :

(±)-1-(1,2-diphényléthyl)pipéridine ;
(-)-1-(1,2-diphényléthyl)pipéridine ;
(+)-1-(1,2-diphényléthyl)pipéridine ;
(±)-1-[2-phényl-1-(2-thiényl))éthyl]pipéridine hydrosulfate ;
(±)-1-(1,2-diphényléthyl)-1,2,3,6-tétrahydropyridine ;
1-(1,2-diphényléthyl)pipéridine ;
1-[1-phényl-2-(2-pyridinyl)éthényl]pipéridine ;
(±)-1-[1-phényl-2-(2-pyridinyl)éthyl]pipéridine ;
(±)-1-(1,2-diphényléthyl)-1H-azépine ;
(±)-1-(1,2-diphénylbutyl)pipéridine ;
(±)-1-(1,2-diphényl-3-méthylbutyl)pipéridine ;
(±)-1-(1,2-diphényl-1-hydroxyéthyl)pipéridine ;
(±)-1-[2-(1-phényl-1-(2-thiényl)éthyl]-1,2,3,6-tétrahydropyridine ;
(±)-1-[1-(1-phénylcyclopentyl)benzyl]pipéridine ;
(±)-1-[1-(1-phénylcyclohexyl)benzyl]pipéridine ;
(±)-1-(1,2-diphényléthyl)pyrrolidine ;
(±)-1-[1-(4-méthylphényl)-2-phényléthyl]pipéridine ;
(±)-1-[1-(4-méthoxyphényl)-2-phényléthyl]pipéridine ;
(±)-1-[1-(1-phénylcyclopropyl)benzyl]pipéridine ;
(±)-1-[2,2-diméthyl-2-phényl-1-(2-thiényl)éthyl]-pipéridine ;
(±)-1-[1-(3-méthoxyphényl)-2-phényléthyl]pipéridine ;
(±)-1-[1-(4-fluorophényl)-2-phényléthyl]pipéridine ;
(±)-1-(2,2-diméthyl-1,2-diphényléthyl)pipéridine ;
(±)-1-[1-(3-méthylphényl)-2-phényléthyl]pipéridine ;
(±)-1-[1-(2-furyl)-2-phényléthyl]pipéridine ;
(±)-1-[1-(2-chlorophényl)-2-phényléthyl]pipéridine ;
(±)-1-[1-(2-méthoxyphényl)-2-phényléthyl]pipéridine ; et
(±)-1-[1-(1-naphtyl)-2-phényléthyl]pipéridine.

**Revendications pour l'Etat contractant suivant : ES**

1. Un procédé de préparation d'un composé de formule

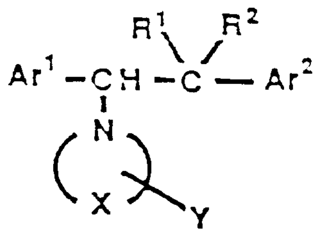

dans laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy ou bien dans laquelle $R^1$ et $R^2$ peuvent être pris ensemble pour former un groupe $C_3$-$C_{10}$ cycloalkyle carbocyclique saturé ou partiellement insaturé ayant 3 à 8 atomes de carbone dans le noyau ;
dans laquelle chacun des $Ar^1$ et $Ar^2$ est un groupe choisi indépendamment parmi les groupes phényle, thiényle, furannyle, pyridinyle, thiazolyle et naphtyle, et dans laquelle chacun des groupes $Ar^1$ et $Ar^2$ précédents peut être substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ haloalkyle, $C_2$-$C_{20}$ alcényle, $C_2$-$C_{20}$ alcynyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle X est choisi parmi CH ou $CH_2$ pour former un noyau ayant 5 à 7 chaînons pourvu que ce noyau soit saturé ou contienne une double liaison ; et dans laquelle Y est un groupe choisi parmi les groupes hydrido, $C_1$-$C_{20}$ alkyle, halo, hydroxyle, $C_1$-$C_{10}$ alcoxy, amino, cyano, nitro et mercapto ;
à la condition que lorsque chacun des $Ar^1$ et $Ar^2$ est un groupe phényle et chacun des $R^1$ et $R^2$ est un groupe hydrido ou bien $R^1$ est H et $R^2$ est hydroxy, alors X ne peut pas être une chaîne alkylène linéaire ayant 4 ou 5 atomes de carbone de façon à former un mélange racémique ; avec en outre la condition que lorsque $A^1$ est un groupe paraméthylphényle ou paraméthoxyphényle et chacun des $R^1$ et $R^2$ est un groupe hydrido et $AR^2$ est un groupe phényle, alors X ne peut pas être une chaîne alkylène linéaire ayant 5 atomes de carbone,
à l'exception des composés dans lesquels :
$Ar^1$ est un groupe phényle, $R^2$ est H et X est $CH_2$ pour former un noyau à 6 chaînons et en même temps

a) $Ar^2$ est un groupe phényle, $R^1$ est F ou OH et
b) $Ar^2$ est un groupe furannyle et $R^1$ est OH et dans laquelle

$Ar^1$, $Ar^2$ est 2,3 ou 4 $NO_2$-phényle, $R^2$ est H, $R^1$ OH et X est $CH_2$ pour former un noyau à 6 chaînons ;
ou bien dans laquelle $R^1$, $R^2$ est H, X est $CH_2$ pour former un noyau à 6 chaînons et $Ar^1$ est un groupe naphtyle, $Ar^2$ est un groupe phényle ou $Ar^1$ est un groupe thiényle et $Ar^2$ est un groupe phényle et dans laquelle
$Ar^1$ est un groupe méthoxyphényle, X est $CH_2$ pour former un noyau à 5 chaînons et en même temps $Ar^2$ est un groupe méthoxyphényle, $R^1$ = H, $R^2$ = OH ou bien $R^1$, $R^2$ = H.
caractérisé en ce que :

A. Une amine de formule (2)

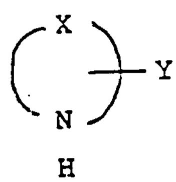

(2)

dans laquelle Y et X sont comme définis précédemment, est mise à réagir en présence d'un acide de Bronsted ou de Lewis dans un solvant approprié avec une cétone de formule (1)

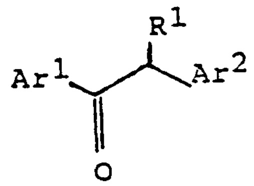

(1)

dans laquelle $Ar^1$, $Ar^2$, $R^1$ sont définis comme précédemment,
ladite réaction (A) générant l'imine (3) de formule

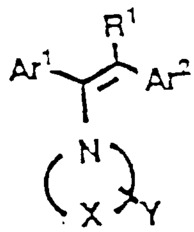

(3)

avec tous les substituants comme défini précédemment, ladite imine (3) étant subséquemment transformée par réduction par un agent réducteur approprié dans un solvant, donnant le composé désiré de formule

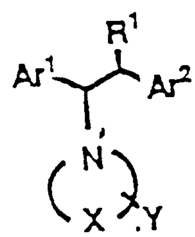

ou bien
B) une amine de formule (2)

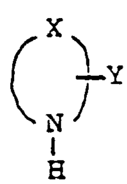

(2)

avec X et Y comme défini précédemment,
soit à l'état pur, soit dans un solvant, est mise à réagir avec un composé de formule (9)

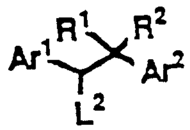

avec $Ar^1$, $Ar^2$, $R^1$, $R^2$ comme défini précédemment et $L^2$ étant un groupe séparable approprié, de sorte que ledit composé de formule (9) soit généré de la cétone correspondante, donnant ainsi les composés désirés, ou bien,
C) un composé de formule (12)

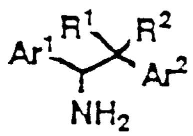

avec tous les substituants comme défini précédemment, et généré à partir de la cétone correspondante, ou de la réaction du nitrile (1) $AR^2CN$ avec un composé de formule

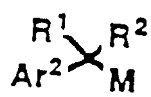

avec les substituants comme défini, est mise à réagir avec un composé de formule (13)

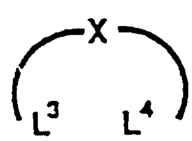

(13)

dans laquelle X est comme défini précédemment et $L^3$ et $L^4$ étant de bons groupes séparables, pour donner les composés désirés.

**2.** Un procédé selon la revendication 1 pour la préparation d'un composé de formule

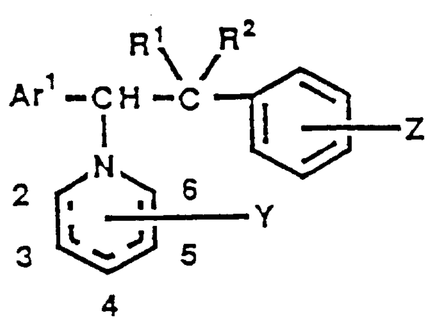

dans laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe carbocyclique saturé ou partiellement

insaturé ayant 3 ou 7 atomes de carbone dans le noyau ;
dans laquelle $Ar^1$ est un groupe choisi parmi les groupes phényle, thiényle, furannyle, pyridinyle, thiazolyle et naphtyle, et dans laquelle chacun des groupes $Ar^1$ précédents ayant une position substituable peut être substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ haloalkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle Y est un groupe choisi parmi les groupes hydrido, $C_1$-$C_{20}$ alkyle, halo, hydroxyle, alcoxy et amino ; et dans laquelle Z est un ou plusieurs groupes choisis parmi les groupes hydrido, $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxyalkyle, amino, cyano, nitro et mercapto.

**3.** Un procédé selon la revendication 2, selon laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo, $C_1$-$C_{10}$ alcoxy, ou dans laquelle $R^1$ et $R^2$ peuvent être pris ensemble pour former un groupe carbocyclique saturé ou partiellement insaturé ayant 3 ou 7 atomes de carbone dans le noyau ;
dans laquelle $Ar^1$ est choisi parmi les groupes phényle, thiényle, pyridinyle, et naphtyle, et dans laquelle chacun des groupes $Ar^1$ précédents ayant une position substituable peut être substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ haloalkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un plusieurs groupes choisis parmi les groupes hydrido, $C_1$-$C_{20}$ alkyle linéaire ou ramifié, $C_3$-$C_{10}$ cycloalkyle, halo, hydroxyle, $C_1$-$C_{10}$ alcoxy et amino.

**4.** Un procédé selon la revendication 3 selon laquelle chacun des $R^1$ et $R^2$ est un groupe choisi indépendamment parmi les groupes hydrido, $C_1$-$C_{20}$ alkyle linéaire ou ramifié et hydroxyle ou dans laquelle $R^1$ et $R^2$ peuvent être pris ensemble pour former un groupe carbocyclique saturé ou partiellement insaturé ayant 3 ou 6 atomes de carbone dans le noyau ;
dans laquelle $Ar^1$ est un groupe choisi parmi les groupes phényle, thiényle, pyridinyle et naphtyle et dans laquelle chacun des groupes $Ar^1$ précédents ayant une position ou plusieurs positions substituables peut être substitué par un ou plusieurs radicaux choisis parmi les groupes hydrido, $C_1$-$C_{20}$ alkyle linéaire ou ramifié, halo, $C_1$-$C_{20}$ halo alkyle, hydroxyle, $C_1$-$C_{10}$ alcoxy, $C_1$-$C_{20}$ alcoxy amino et nitro ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes hydrido, $C_1$-$C_{20}$ alkyle linéaire ou ramifié, halo, hydroxyle et $C_1$-$C_{10}$ alcoxy.

**5.** Un procédé selon la revendication 1 pour la préparation d'un composé de formule

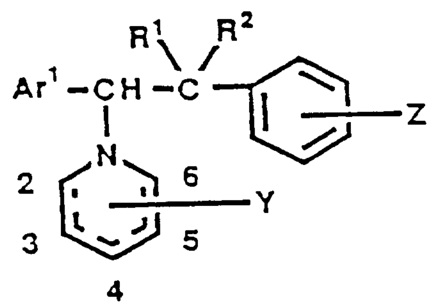

dans laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe carbocyclique saturé ou partiellement insaturé ayant 3 ou 7 atomes de carbone dans le noyau ;
dans laquelle $Ar^1$ est un groupe choisi parmi les groupes phényle, thiényle, furannyle, pyridinyle, thiazolyle et naphtyle ; et dans laquelle chacun des groupes $Ar^1$ précités ayant une position substituable est substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$

alcoxy, alcoxy alkyle, amino, cyano, nitro et mercapto ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; et dans laquelle le trait discontinu dans le noyau contenant N représente une double liaison entre deux atomes de carbone adjacents quelconques mettant en oeuvre les positions 2-, 3-, 4-, 5- et 6- du noyau contenant N.

**6.** Un procédé selon la revendication 5 selon laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe carbocyclique saturé ou partiellement insaturé ayant 3 ou 7 atomes de carbone dans le noyau ;
dans laquelle $Ar^1$ est un groupe choisi parmi les groupes phényle, thiényle, furannyle, pyridinyle, thiazolyle et naphtyle, et dans laquelle chacun des groupes précédents ayant une ou plusieurs positions substituables peut être substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes hydrido, $C_1$-$C_{20}$ alkyle linéaire ou ramifié, $C_3$-$C_{10}$ cycloalkyle, halo, hydroxyle, $C_1$-$C_{10}$ alcoxy et amino ; et dans laquelle le trait discontinu dans le noyau contenant N représente une double liaison entre 2 atomes de carbone adjacents quelconques mettant en oeuvre les positions 3-, 4-, 5- du noyau contenant N.

**7.** Un procédé selon la revendication 6 selon laquelle chacun des $R^1$ et $R^2$ est un groupe choisi indépendamment parmi les groupes hydrido, $C_1$-$C_{20}$ alkyle linéaire ou substitué, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe cycloalkyle saturé ou partiellement insaturé ayant 3 ou 7 atomes de carbone dans le noyau ;
dans laquelle $Ar^1$ est choisi parmi les groupes phényle, thiényle, pyridinyle et naphtyle, et dans laquelle chacun des groupes précédents ayant une position substituable substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes hydrido, $C_1$-$C_{20}$ alkyle linéaire ou ramifié, $C_3$-$C_{10}$cycloalkyle, halo, hydroxyle, $C_1$-$C_{10}$ alcoxy et amino ; et dans laquelle le trait discontinu dans le noyau contenant N représente une double liaison entre 2 atomes de carbone adjacents quelconques mettant en oeuvre les positions 3-, 4-, 5- du noyau contenant N.

**8.** Un procédé selon la revendication 7 selon laquelle chacun des $R^1$ et $R^2$ est un groupe choisi indépendamment parmi les groupes hydrido, $C_1$-$C_{20}$ alkyle linéaire ou ramifié et hydroxyle ; dans laquelle $R^1$ et $R^2$ peuvent être pris ensemble pour former un groupe cycloalkyle saturé ou partiellement insaturé ayant 3 ou 6 atomes de carbone dans le noyau ;
dans laquelle $Ar^1$ est choisi parmi les groupes phényle, thiényle, pyridinyle et naphtyle, et dans laquelle chacun des groupes précédents ayant une ou plusieurs positions substituables est substitué par un ou plusieurs radicaux choisis parmi les groupes hydrido, $C_1$-$C_{20}$ alkyle linéaire ou ramifié, halo, $C_1$-$C_{20}$ halo alkyle, hydroxyle, $C_1$-$C_{10}$ alcoxy, amino et nitro ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes hydrido, $C_1$-$C_{20}$ alkyle linéaire ou ramifié, halo, hydroxyle et $C_1$-$C_{10}$ alcoxy ; et dans laquelle le trait discontinu à l'intérieur des noyaux contenant N représente une double liaison entre 2 atomes de carbone adjacents quelconques mettant en oeuvre les positions 3-, 4-, 5- du noyau contenant N.

**9.** Un procédé selon la revendication 1 pour la préparation d'un composé choisi dans le groupe constitué de :

(-)-1-(1,2-diphényléthyl)pipéridine ;
(+)-1-(1,2-diphényléthyl)pipéridine ;
(±)-1-[1-phényl-2-(2-pyridinyl)éthyl]pipéridine ;

1-[1-phényl-2-(2-pyridinyl)éthényl]pipéridine ;
(±)-1-(1,2-diphényléthyl)-1H-azépine ;
(±)-1-(1,2-diphénylbutyl)pipéridine ;
(±)-1-(1,2-diphényl-3-méthylbutyl)pipéridine ;
(±)-1-[1-(1-phénylcyclopentyl)benzyl]pipéridine ;
(±)-1-[1-(1-phénylcyclohexyl)benzyl]pipéridine ;
(±)-1-[1-(1-phénylcyclopropyl)benzyl]pipéridine ;
(±)-1-[2,2-diméthyl-2-phényl-1-(2-thiényl)éthyl]pipéridine ;
(±)-1-[1-(3-méthoxyphényl)-2-phényléthyl]pipéridine ;
(±)-1-[1-(4-florophényl)-2-phényléthyl]pipéridine ;
(±)-1-(2,2-diméthyl-1,2-diphényléthyl)pipéridine ;
(±)-1-[1-(3-méthylphényl)-2-phényléthyl]pipéridine ;
(±)-1-[1-(2-furyl)-2-phényléthyl]pipéridine ;
(±)-1-[1-(2-chlorophényl)-2-phényléthyl]pipéridine ;
(±)-1-[1-(2-méthoxyphényl)-2-phényléthyl]pipéridine ;
(±)-1-(1,2-diphényléthyl)-1,2,3,6-tétrahydropyridine ; et
(±)-1-[2-phényl-1-(2-thiényl)éthyl]-1,2,3,6-tétrahydropyridine.

**10.** Un procédé selon la revendication 9 pour la préparation d'un composé choisi dans le groupe constitué de :
(-)-1-(1,2-diphényléthyl)pipéridine ;
(+)-1-(1,2-diphényléthyl)pipéridine ;
(±)-1-[1-phényl-2-(2-pyridinyl)éthyl]pipéridine ;
(±)-1-(1,2-diphényléthyl)-1H-azépine ;
(±)-1-(1,2-diphénylbutyl)pipéridine ;
(±)-1-[1-(1-phénylcyclohexyl)benzyl]pipéridine ;
(±)-1-[1-(1-phénylcyclopropyl)benzyl]pipéridine ;
(±)-1-[1-(3-méthoxyphényl)-2-phényléthyl]pipéridine ;
(±)-1-[1-(4-fluorophényl)-2-phényléthyl]pipéridine ;
(±)-1-(2,2-diméthyl-1,2-diphényléthyl)pipéridine ;
(±)-1-(1-(3-méthylphényl)-2-phényléthyl)pipéridine ;
(±)-1-[1-(2-chlorophényl)-2-phényléthyl]pipéridine ; et
(±)-1-[1-(2-méthoxyphényl)-2-phényléthyl]pipéridine.

**11.** Un procédé selon la revendication 10 pour la préparation d'un composé choisi dans le groupe constitué de :
(±)-1-[1-(3-méthoxyphényl)-1-phényléthyl]pipéridine ;
(±)-1-[1-(4-fluorophényl)-2-phényléthyl]pipéridine ;
(±)-1-[1-(3-méthylphényl)-2-phényléthyl]pipéridine ;
(±)-1-[1-(2-chlorophényl)-2-phényléthyl]pipéridine ; et
(±)-1-[1-(2-méthoxyphényl)-2-phényléthyl]pipéridine.

**12.** Un procédé selon la revendication 11 selon laquelle le composé préparé est la
(±)-1-[1-(2-chlorophényl)-2-phényléthyl]pipéridine.

**13.** Un procédé selon la revendication 9 pour la préparation d'un composé choisi dans le groupe constitué de :
(±)-1-(1,2-diphényléthyl)-1,2,3,6-tétrahydropyridine ; et
(±)-1-[2-phényl-1-(2-thiényl)éthyl]-1,2,3,6-tétrahydropyridine.

**14.** Un procédé selon la revendication 13 pour la préparation de la
(±)-1-[2-phényl-1-(2-thiényl)éthyl]-1,2,3,6-tétrahydropyridine.

**15.** Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 1.

16. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 2.

17. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 3.

18. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 4.

19. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 5.

20. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 6.

21. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 7.

22. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 8.

23. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 9.

24. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 10.

25. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 11.

26. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 12.

27. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 13.

28. Un procédé de préparation d'une composition pharmaceutique par mélangeage d'un support ou diluant pharmaceutiquement acceptable avec une quantité thérapeutique efficace d'un composé actif sélectionné parmi une famille de composés préparés selon la revendication 14.

29. Utilisation d'un composé selon la revendication 1 pour la préparation d'un médicament pour contrôler les processus neuropathologiques et les conséquences neurodégénératives chez les mammifères.

30. Utilisation d'un composé selon l'une quelconque des revendications 2 à 14 pour la préparation d'un médicament pour contrôler les processus neuropathologique et les conséquences neurodégénératives chez les mammifères.

**31.** Utilisation selon la revendication 29 pour la préparation d'un médicament pour le traitement de l'hypoxie, de l'anoxie ou de l'ischémie.

**32.** Utilisation selon la revendication 31 pour la préparation d'un médicament pour le traitement de l'ischémie.

**33.** Utilisation d'un composé tel que défini dans les revendications 29-32 choisis dans le groupe constitué de :

(±)-1-(1,2-diphényléthyl)pipéridine;
(-)-1-(1,2-diphényléthyl)pipéridine ;
(+)-1-(1,2-diphényléthyl)pipéridine ;
(±)-1-[2-phényl-1-(2-thiényl))éthyl]pipéridine hydrosulfate ;
(±)-1-(1,2-diphényléthyl)-1,2,3,6-tétrahydropyridine ;
1-(1,2-diphényléthyl)pipéridine ;
1-[1-phényl-2-(2-pyridinyl)éthényl]pipéridine ;
(±)-1-[1-phényl-2-(2-pyridinyl)éthyl]pipéridine ;
(±)-1-(1,2-diphényléthyl)-1H-azépine ;
(±)-1-(1,2-diphénylbutyl)pipéridine ;
(±)-1-(1,2-diphényl-3-méthylbutyl)pipéridine ;
(±)-1-(1,2-diphényl-1-hydroxyéthyl)pipéridine ;
(±)-1-[2-(1-phényl-1-(2-thiényl)éthyl]-1,2,3,6-tétrahydropyridine ;
(±)-1-[1-(1-phénylcyclopentyl)benzyl]pipéridine ;
(±)-1-[1-(1-phénylcyclohexyl)benzyl]pipéridine ;
(±)-1-(1,2-diphényléthyl)pyrrolidine ;
(±)-1-[1-(4-méthylphényl)-2-phényléthyl]pipéridine ;
(±)-1-[1-(4-méthoxyphényl)-2-phényléthyl]pipéridine ;
(±)-1-[1-(1-phénylcyclopropyl)benzyl]pipéridine ;
(±)-1-[2,2-diméthyl-2-phényl-1-(2-thiényl)éthyl]-pipéridine ;
(±)-1-[1-(3-méthoxyphényl)-2-phényléthyl]pipéridine ;
(±)-1-[1-(4-fluorophényl)-2-phényléthyl]pipéridine ;
(±)-1-(2,2-diméthyl-1,2-diphényléthyl)pipéridine ;
(±)-1-[1-(3-méthylphényl)-2-phényléthyl]pipéridine ;
(±)-1-[1-(2-furyl)-2-phényléthyl]pipéridine ;
(±)-1-[1-(2-chlorophényl)-2-phényléthyl]pipéridine ;
(±)-1-[1-(2-méthoxyphényl)-2-phényléthyl]pipéridine ; et
(±)-1-[1-(1-naphtyl)-2-phényléthyl]pipéridine.

**Revendications pour l'Etat contractant suivant : GR**

**1.** Un composé de formule

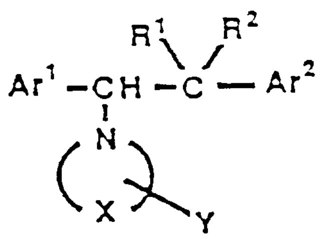

dans laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy ou bien dans laquelle $R^1$ et $R^2$ peuvent être pris ensemble pour former un groupe $C_3$-$C_{10}$ cycloalkyle carbocyclique saturé ou partiellement insaturé ayant 3 à 8 atomes de carbone dans le noyau ;
dans laquelle chacun des $Ar^1$ et $Ar^2$ est un groupe choisi indépendamment parmi les groupes phényle, thiényle, furannyle, pyridinyle, thiazolyle et naphtyle, et dans laquelle chacun des groupes $Ar^1$ et $Ar^2$ précédents peut être substitué par un ou plusieurs radicaux choisis parmi les groupes hydrido, $C_1$-$C_{20}$

alkyle linéaire ou ramifié, $C_3$-$C_{10}$ cycloalkyle, halo, $C_1$-$C_{20}$ haloalkyle, $C_2$-$C_{20}$ alcényle, $C_2$-$C_{20}$ alcynyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle X est choisi parmi CH ou $CH_2$ pour former un noyau ayant 5 à 7 chaînons pourvu que ce noyau soit saturé ou contienne une double liaison ; et dans laquelle Y est un groupe choisi parmi les groupes hydrido, $C_1$-$C_{20}$ alkyle, halo, hydroxyle, $C_1$-$C_{10}$ alcoxy, amino, cyano, nitro et mercapto;

à la condition que lorsque chacun des $Ar^1$ et $Ar^2$ est un groupe phényle et chacun des $R^1$ et $R^2$ est un groupe hydrido ou bien $R^1$ est H et $R^2$ est hydroxy, alors X ne peut pas être une chaîne alkylène linéaire ayant 4 ou 5 atomes de carbone de façon à former un mélange racémique ; avec en outre la condition que lorsque $A^1$ est un groupe paraméthylphényle ou paraméthoxyphényle et chacun des $R^1$ et $R^2$ est un groupe hydrido et $AR^2$ est un groupe phényle, alors X ne peut pas être une chaîne alkylène linéaire ayant 5 atomes de carbone,

à l'exception des composés dans lesquels :

$Ar^1$ est un groupe phényle, $R^2$ est H et X est $CH_2$ pour former un noyau à 6 chaînons et en même temps

a) $Ar^2$ est un groupe phényle, $R^1$ est F ou OH et

b) $Ar^2$ est un groupe furannyle et $R^1$ est OH et dans laquelle

$Ar^1$, $Ar^2$ est 2,3 ou 4 $NO_2$-phényle, $R^2$ est H, $R^1$ OH et X est $CH_2$ pour former un noyau à 6 chaînons; ou bien dans laquelle $R^1$, $R^2$ est H, X est $CH_2$ pour former un noyau à 6 chaînons et $Ar^1$ est un groupe naphtyle, $Ar^2$ est un groupe phényle ou $Ar^1$ est un groupe thiényle et $Ar^2$ est un groupe phényle et dans laquelle

$AR^1$ est un groupe méthoxyphényle, X est $CH_2$ pour former un noyau à 5 chaînons et en même temps $Ar^2$ est un groupe méthoxyphényle, $R^1$ = H, $R^2$ = OH ou bien $R^1$, $R^2$ = H.

2. Composé selon la revendication 1 de formule :

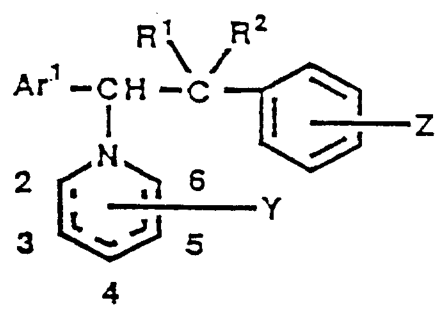

dans laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe carbocyclique saturé ou partiellement insaturé ayant 3 ou 7 atomes de carbone dans le noyau ;

dans laquelle $Ar^1$ est un groupe choisi parmi les groupes phényle, thiényle, furannyle, pyridinyle, thiazolyle et naphtyle, et dans laquelle chacun des groupes $Ar^1$ précédents ayant une position substituable peut être substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ haloalkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle Y est un groupe choisi parmi les groupes hydrido, $C_1$-$C_{20}$ alkyle, halo, hydroxyle, alcoxy et amino ; et dans laquelle Z est un ou plusieurs groupes choisis parmi les groupes hydrido, $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxyalkyle, amino, cyano, nitro et mercapto.

3. Composé selon la revendication 2, selon laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo, $C_1$-$C_{10}$ alcoxy, ou dans laquelle $R^1$ et $R^2$ peuvent être pris ensemble pour former un groupe carbocyclique saturé ou partiellement insaturé ayant 3 ou 7 atomes de carbone dans le noyau ; dans laquelle $Ar^1$ est choisi parmi les groupes phényle, thiényle, pyridinyle, et naphtyle, et dans

laquelle chacun des groupes $Ar^1$ précédents ayant une position substituable peut être substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ haloalkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un plusieurs groupes choisis parmi les groupes hydrido, $C_1$-$C_{20}$ alkyle linéaire ou ramifié, $C_3$-$C_{10}$ cycloalkyle, halo, hydroxyle, $C_1$-$C_{10}$ alcoxy et amino.

**4.** Composé selon la revendication 3, selon laquelle chacun des $R^1$ et $R^2$ est un groupe choisi indépendamment parmi les groupes hydrido, $C_1$-$C_{20}$ alkyle linéaire ou ramifié et hydroxyle ou dans laquelle $R^1$ et $R^2$ peuvent être pris ensemble pour former un groupe carbocyclique saturé ou partiellement insaturé ayant 3 ou 6 atomes de carbone dans le noyau ;
dans laquelle $Ar^1$ est un groupe choisi parmi les groupes phényle, thiényle, pyridinyle et naphtyle et dans laquelle chacun des groupes $Ar^1$ précédents ayant une position ou plusieurs positions substituables peut être substitué par un ou plusieurs radicaux choisis parmi les groupes hydrido, $C_1$-$C_{20}$ alkyle linéaire ou ramifié, halo, $C_1$-$C_{20}$ halo alkyle, hydroxyle, $C_1$-$C_{10}$ alcoxy, $C_1$-$C_{20}$ alcoxy amino et nitro ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes hydrido, $C_1$-$C_{20}$ alkyle linéaire ou ramifié, halo, hydroxyle et $C_1$-$C_{10}$ alcoxy.

**5.** Composé selon la revendication 1 de formule:

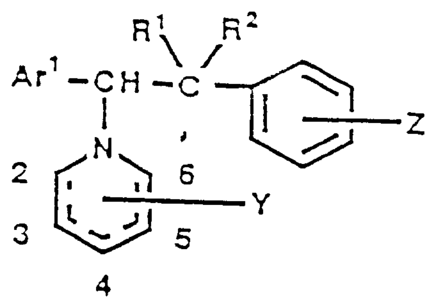

dans laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe carbocyclique saturé ou partiellement insaturé ayant 3 ou 7 atomes de carbone dans le noyau ;
dans laquelle $Ar^1$ est un groupe choisi parmi les groupes phényle, thiényle, furannyle, pyridinyle, thiazolyle et naphtyle ; et dans laquelle chacun des groupes $Ar^1$ précités ayant une position substituable est substitué par un ou plusieurs radicaux choisis parmi les groupes hydrido $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, alcoxy alkyle, amino, cyano, nitro et mercapto ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; et dans laquelle le trait discontinu dans le noyau contenant N représente une double liaison entre deux atomes de carbone adjacents quelconques mettant en oeuvre les positions 2-, 3-, 4-, 5- et 6- du noyau contenant N.

**6.** Composé selon la revendication 5 selon laquelle chacun des $R^1$ et $R^2$ est un groupe choisi indépendamment parmi les groupes hydrido, $C_1$-$C_{20}$ alkyle linéaire ou ramifié, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe carbocyclique saturé ou partiellement insaturé ayant 3 ou 7 atomes de carbone dans le noyau;
dans laquelle $Ar^1$ est un groupe choisi parmi les groupes phényle, thiényle, furannyle, pyridinyle, thiazolyle et naphtyle, et dans laquelle chacun des groupes précédents ayant une ou plusieurs positions substituables peut être substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle

Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes hydrido, $C_1$-$C_{20}$ alkyle linéaire ou ramifié, $C_3$-$C_{10}$ cycloalkyle, halo, hydroxyle, $C_1$-$C_{10}$ alcoxy et amino ; et dans laquelle le trait discontinu dans le noyau contenant N représente une double liaison entre 2 atomes de carbone adjacents quelconques mettant en oeuvre les positions 3-, 4-, 5- du noyau contenant N.

**7.** Composé selon la revendication 6, selon laquelle chacun des $R^1$ et $R^2$ est un groupe choisi indépendamment parmi les groupes hydrido, $C_1$-$C_{20}$ alkyle linéaire ou substitué, $C_3$-$C_{10}$ cycloalkyle, hydroxyle, halo et $C_1$-$C_{10}$ alcoxy, ou dans laquelle $R^1$ et $R^2$ peuvent être pris ensemble pour former un groupe cycloalkyle saturé ou partiellement insaturé ayant 3 ou 7 atomes de carbone dans le noyau ;
dans laquelle $Ar^1$ est choisi parmi les groupes phényle, thiényle, pyridinyle et naphtyle, et dans laquelle chacun des groupes précédents ayant une position substituable substitué par un ou plusieurs radicaux choisis parmi les groupes hydrido, $C_1$-$C_{20}$ alkyle linéaire ou ramifié, $C_3$-$C_{10}$ cycloalkyle, halo, $C_1$-$C_{20}$ halo alkyle, hydroxyle, $C_1$-$C_{10}$ hydroxyalkyle, $C_1$-$C_{10}$ alcoxy, $C_1$-$C_{20}$ alcoxy alkyle, amino, cyano, nitro et mercapto ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino; et dans laquelle Z est un ou plusieurs groupes choisis parmi les groupes hydrido, $C_1$-$C_{20}$ alkyle linéaire ou ramifié, $C_3$-$C_{10}$ cycloalkyle, halo, hydroxyle, $C_1$-$C_{10}$ alcoxy et amino ; et dans laquelle le trait discontinu dans le noyau contenant N représente une double liaison entre 2 atomes de carbone adjacents quelconques mettant en oeuvre les positions 3-, 4-, 5- du noyau contenant N.

**8.** Composé selon la revendication 7, selon laquelle chacun des $R^1$ et $R^2$ est un groupe choisi indépendamment parmi les groupes hydrido, $C_1$-$C_{20}$ alkyle linéaire ou ramifié et hydroxyle ; dans laquelle $R^1$ et $R^2$ peuvent être pris ensemble pour former un groupe cycloalkyle saturé ou partiellement insaturé ayant 3 ou 6 atomes de carbone dans le noyau ;
dans laquelle $Ar^1$ est choisi parmi les groupes phényle, thiényle, pyridinyle et naphtyle, et dans laquelle chacun des groupes précédents ayant une ou plusieurs positions substituables est substitué par un ou plusieurs radicaux choisis parmi les groupes hydrido, $C_1$-$C_{20}$ alkyle linéaire ou ramifié, halo, $C_1$-$C_{20}$ halo alkyle, hydroxyle, $C_1$-$C_{10}$ alcoxy, amino et nitro ; dans laquelle Y est un groupe choisi parmi les groupes hydrido, $C_1$-$C_{20}$ alkyle linéaire ou ramifié, halo, hydroxyle, $C_1$-$C_{10}$ alcoxy et amino ; et dans laquelle Z est un ou plusieurs groupes choisis parmi les groupes hydrido, $C_1$-$C_{20}$ alkyle linéaire ou ramifié, halo, hydroxyle et $C_1$-$C_{10}$ alcoxy ; et dans laquelle le trait discontinu à l'intérieur des noyaux contenant N représente une double liaison entre 2 atomes de carbone adjacents quelconques mettant en oeuvre les positions 3-, 4-, 5- du noyau contenant N.

**9.** Composé selon la revendication 1 choisi dans le groupe constitué de
(-)-1-(1,2-diphényléthyl)pipéridine ;
(+)-1-(1,2-diphényléthyl)pipéridine ;
(±)-1-[1-phényl-2-(2-pyridinyl)éthyl]pipéridine ;
1-[1-phényl-2-(2-pyridinyl)éthényl]pipéridine ;
(±)-1-(1,2-diphényléthyl)-1H-azépine ;
(±)-1-(1,2-diphénylbutyl)pipéridine ;
(±)-1-(1,2-diphényl-3-méthylbutyl)pipéridine ;
(±)-1-[1-(1-phénylcyclopentyl)benzyl]pipéridine ;
(±)-1-[1-(1-phénylcyclohexyl)benzyl]pipéridine ;
(±)-1-[1-(1-phénylcyclopropyl)benzyl]pipéridine ;
(±)-1-[2,2-diméthyl-2-phényl-1-(2-thiényl)éthyl]pipéridine ;
(±)-1-[1-(3-méthoxyphényl)-2-phényléthyl]pipéridine ;
(±)-1-[1-(4-florophényl)-2-phényléthyl]pipéridine ;
(±)-1-(2,2-diméthyl-1,2-diphényléthyl)pipéridine ;
(±)-1-[1-(3-méthylphényl)-2-phényléthyl]pipéridine ;
(±)-1-[1-(2-furyl)-2-phényléthyl]pipéridine ;
(±)-1-[1-(2-chlorophényl)-2-phényléthyl]pipéridine ;
(±)-1-[1-(2-méthoxyphényl)-2-phényléthyl]pipéridine ;
(±)-1-(1,2-diphényléthyl)-1,2,3,6-tétrahydropyridine ; et
(±)-1-[2-phényl-1-(2-thiényl)éthyl]-1,2,3,6-tétrahydropyridine.

**10.** Composé selon la revendication 9, choisi dans le groupe constitué de :
(-)-1-(1,2-diphényléthyl)pipéridine ;
(+)-1-(1,2-diphényléthyl)pipéridine ;
(±)-1-[1-phényl-2-(2-pyridinyl)éthyl]pipéridine ;
(±)-1-(1,2-diphényléthyl)-1H-azépine ;
(±)-1-(1,2-diphénylbutyl)pipéridine ;
(±)-1-[1-(1-phénylcyclohexyl)benzyl]pipéridine ;
(±)-1-[1-(1-phénylcyclopropyl)benzyl]pipéridine ;
(±)-1-[1-(3-méthoxyphényl)-2-phényléthyl]pipéridine ;
(±)-1-[1-(4-fluorophényl)-2-phényléthyl]pipéridine ;
(±)-1-(2,2-diméthyl-1,2-diphényléthyl)pipéridine ;
(±)-1-(1-(3-méthylphényl)-2-phényléthyl)pipéridine ;
(±)-1-[1-(2-chlorophényl)-2-phényléthyl]pipéridine ; et
(±)-1-[1-(2-méthoxyphényl)-2-phényléthyl]pipéridine.

**11.** Composé selon la revendication 10 choisi dans le groupe constitué de :
(±)-1-[1-(3-méthoxyphényl)-1-phényléthyl]pipéridine ;
(±)-1-[1-(4-fluorophényl)-2-phényléthyl]pipéridine ;
(±)-1-[1-(3-méthylphényl)-2-phényléthyl]pipéridine ;
(±)-1-[1-(2-chlorophényl)-2-phényléthyl]pipéridine ; et
(±)-1-[1-(2-méthoxyphényl)-2-phényléthyl]pipéridine.

**12.** Composé selon la revendication 11 qui est la
(±)-1-[1-(2-chlorophényl)-2-phényléthyl]pipéridine.

**13.** Composé selon la revendication 9 choisi dans le groupe constitué de :
(±)-1-(1,2-diphényléthyl)-1,2,3,6-tétrahydropyridine ; et
(±)-1-[2-phényl-1-(2-thiényl)éthyl]-1,2,3,6-tétrahydropyridine.

**14.** Composé selon la revendication 13 qui est la
(±)-1-[2-phényl-1-(2-thiényl)éthyl]-1,2,3,6-tétrahydropyridine.

**15.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 1.

**16.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 2.

**17.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 3.

**18.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 4.

**19.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 5.

**20.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 6.

**21.** Un procédé de préparation d'une composition pharmaceutique par mélangeage d'un support ou diluant phamaceutiquement acceptable avec une quantité thérapeutique efficace d'un composé actif choisi

dans une famille de composés selon la revendication 7.

**22.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 8.

**23.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 9.

**24.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 10.

**25.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 11.

**26.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 12.

**27.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 13.

**28.** Un procédé de préparation d'une composition pharmaceutique par mélangeage d'un support ou diluant phamaceutiquement acceptable avec une quantité thérapeutique efficace d'un composé actif choisi dans une famille de composés selon la revendication 14.

**29.** Utilisation d'un composé selon la revendication 1 pour la préparation d'un médicament pour contrôler les processus neuropathologiques et les conséquences neurodégénératives chez les mammifères.

**30.** Utilisation d'un composé selon l'une quelconque des revendications 2 à 14 pour la préparation d'un médicament pour contrôler des processus neuropathologiques et les conséquences neurodégénératives chez les mammifères.

**31.** Utilisation selon la revendication 29 pour la préparation d'un médicament pour le traitement de l'hypoxie, de l'anoxie ou de l'ischemie.

**32.** Utilisation selon la revendication 31 pour la préparation d'un médicament pour le traitement de l'ischémie.

**33.** Utilisation d'un composé tel que défini dans les revendications 29-32 choisis dans le groupe constitué de :

(±)-1-(1,2-diphényléthyl)pipéridine ;
(-)-1-(1,2-diphényléthyl)pipéridine ;
(+)-1-(1,2-diphényléthyl)pipéridine ;
(±)-1-[2-phényl-1-(2-thiényl))éthyl]pipéridine hydrosulfate ;
(±)-1-(1,2-diphényléthyl)-1,2,3,6-tétrahydropyridine ;
1-(1,2-diphényléthyl)pipéridine ;
1-[1-phényl-2-(2-pyridinyl)éthényl]pipéridine ;
(±)-1-[1-phényl-2-(2-pyridinyl)éthyl]pipéridine ;
(±)-1-(1,2-diphényléthyl)-1H-azépine;
(±)-1-(1,2-diphénylbutyl)pipéridine ;
(±)-1-(1,2-diphényl-3-méthylbutyl)pipéridine ;
(±)-1-(1,2-diphényl-1-hydroxyéthyl)pipéridine ;
(±)-1-[2-(1-phényl-1-(2-thiényl)éthyl]-1,2,3,6-tétrahydropyridine ;

(±)-1-[1-(1-phénylcyclopentyl)benzyl]pipéridine ;
(±)-1-[1-(1-phénylcyclohexyl)benzyl]pipéridine ;
(±)-1-(1,2-diphényléthyl)pyrrolidine ;
(±)-1-[1-(4-méthylphényl)-2-phényléthyl]pipéridine ;
(±)-1-[1-(4-méthoxyphényl)-2-phényléthyl]pipéridine ;
(±)-1-[1-(1-phénylcyclopropyl)benzyl]pipéridine ;
(±)-1-[2,2-diméthyl-2-phényl-1-(2-thiényl)éthyl]-pipéridine ;
(±)-1-[1-(3-méthoxyphényl)-2-phényléthyl]pipéridine ;
(±)-1-[1-(4-fluorophényl)-2-phényléthyl]pipéridine ;
(±)-1-(2,2-diméthyl-1,2-diphényléthyl)pipéridine ;
(±)-1-[1-(3-méthylphényl)-2-phényléthyl]pipéridine ;
(±)-1-[1-(2-furyl)-2-phényléthyl]pipéridine ;
(±)-1-[1-(2-chlorophényl)-2-phényléthyl]pipéridine ;
(±)-1-[1-(2-méthoxyphényl)-2-phényléthyl]pipéridine ; et
(±)-1-[1-(1-naphtyl)-2-phényléthyl]pipéridine.